# EUROPEAN PATENT SPECIFICATION

(11) **EP 3 793 680 B1**
(45) Date of publication and mention of the grant of the patent: **22.11.2023**
(21) Application number: 19728828.5
(22) Date of filing: 17.05.2019
(51) Int. Cl.: A61N 5/10, G16H 20/40, A61K 51/12, G16H 20/17, A61M 5/14, A61M 5/142, A61M 5/19, A61M 5/168, A61M 5/178, A61M 5/172, A61M 25/00, A61M 25/06

(54) **RADIOEMBOLIZATION DELIVERY DEVICE**
ABGABEVORRICHTUNG FÜR RADIOEMBOLISATION
DISPOSITIF D'ADMINISTRATION DE RADIO-EMBOLISATION

(30) Priority: 18.05.2018 US 201862673628 P; 18.05.2018 US 201862673632 P
(43) Date of publication of application: 24.03.2021
(60) Divisional application: 23190815.3
(73) Proprietor: Bard Peripheral Vascular, Inc., Franklin Lakes, NJ 07417 (US)
(72) Inventor: AKERELE-ALE, Oladipo Peter, Franklin Lakes, New Jersey 07417 (US); DESPA, Mircea, Franklin Lakes, New Jersey 07417 (US); DORN, Juergen, Franklin Lakes, New Jersey 07417 (US); DROBNIK, Christopher Dean, Franklin Lakes, New Jersey 07417 (US); DROBNIK, Michael Wesley, Franklin Lakes, New Jersey 07417 (US); HEBERT, Casey Tyler, Franklin Lakes, New Jersey 07417 (US); KINGMAN, Amanda, Franklin Lakes, New Jersey 07417 (US); PALMER, Alex, Franklin Lakes, New Jersey 07417 (US); PARMENTIER, William, Franklin Lakes, New Jersey 07417 (US); PUSATERI, Lee, Franklin Lakes, New Jersey 07417 (US); RICHARDS, Andrew, Franklin Lakes, New Jersey 07417 (US); SIMMONS, Brandon David, Franklin Lakes, New Jersey 07417 (US); SOLOMON, Clint, Franklin Lakes, New Jersey 07417 (US); WRIGHT, Mark Nicholas, Franklin Lakes, New Jersey 07417 (US); YARGER, Michael, Franklin Lakes, New Jersey 07417 (US); MARTIN, Adam, Franklin Lakes, New Jersey 07417 (US)
(74) Representative: Hoffmann Eitle
(86) International application number: PCT/US2019/033001
(87) International publication number: WO 2019/222713

(56) References cited:
- EP-A2- 2 179 758
- WO-A1-2012/118687
- DE-A1- 3 035 290
- FR-A1- 2 917 981
- JP-A- 2006 017 660
- US-A- 5 478 323
- US-A1- 2001 021 826
- US-A1- 2006 091 329
- US-A1- 2006 293 552
- US-A1- 2007 129 591
- US-A1- 2008 058 719
- US-A1- 2008 103 564
- US-A1- 2010 084 585
- US-A1- 2012 201 726
- US-A1- 2013 165 899
- US-A1- 2013 317 277
- US-A1- 2014 046 295
- US-A1- 2014 236 093
- US-A1- 2016 325 047
- US-A1- 2016 331 998

## Description

### CROSS-REFERENCES TO RELATED APPLICATIONS

This application claims the benefit of priority to U.S. Provisional App. No. 62/673632, entitled "Radioembolization Delivery Device," filed on May 18, 2018; and U.S. Provisional App. No. 62/673628, entitled "Dual Stage Syringe," filed on May 18, 2018.

### TECHNICAL FIELD

The present invention generally relates to medical devices for treating cancer, and more particularly to medical devices configured and operable to deliver radioactive compounds to a treatment area within a patient's body in procedures such as transarterial radioembolization.

### BACKGROUND

In cancer treatments involving radiation therapy, inadvertent or excess exposure to radiation from radioactive therapeutic agents can be harmful and potentially lethal to patients or medical personnel. Accordingly, medical instruments for radiation therapies must be configured to localize the delivery of radioactive material to a particular area of the patient's body while shielding others from unnecessarily being exposed to radiation.

Transarterial Radioembolization is a transcatheter intra-arterial procedure performed by interventional radiology and is commonly employed for the treatment of malignant tumors. During this medical procedure, a microcatheter is navigated into a patient's liver where radioembolizing microspheres loaded with a radioactive compound, such as yttrium-90 (⁹⁰Y), are delivered to the targeted tumors. The microspheres embolize blood vessels that supply the tumors while also delivering radiation to kill tumor cells.

Generally, medical devices for performing radioembolization procedures require multiple syringes, external tubing, a vial containing the radioactive compound, and a bulky shield assembly for containing and shielding the radioactive vial. Such devices typically involve time consuming and labor-intensive setup procedures. The complex devices are commonly stationary and thereby limit a physician's mobility in an operating room to within a certain proximity of the device. US 2016/331998 A1 is concerned with a radiopharmaceutical delivery and tube management system.

Routine manipulation of a product container storing radioactive material during radioembolization procedures generally requires a Nuclear Medicine Technician, who handles the material with forceps or tweezers. This process involves further potential of exposing additional medical personnel to radioactivity, and contaminating the operating room. Syringes for manually administering the radioactive compound are prone to inconsistent flow rates and pressures. Insufficient injection rates result in decreased bead dispersion, which may impact efficacy of the treatment.

Accordingly, a need exists for a medical device that is configured and operable to perform radioembolization that incorporates a simplistic design and consistent means for administering constant flow rates and pressure of the radioactive compound to the patient's body. A simplified device provides a physician enhanced maneuverability in the operating room during the medical procedure, including an ability to reposition the device about the patient as desired. Additionally, a device with enhanced shielding of the radioactive material enables greater protection to a physician utilizing the medical device while treating a patient.

### BRIEF DESCRIPTION OF THE DRAWINGS

FIG. 1 is a perspective view of a delivery device including a protective shield and handle assembly according to one or more embodiments shown and described herein;
FIG. 2 is a cross-sectional view of the delivery device of FIG. 1 with the handle assembly coupled to the protective shield by a plunger according to one or more embodiments shown and described herein, the cross-section taken along lines 2-2 of FIG. 1;
FIG. 3 is a perspective view of the delivery device of FIG. 1 connected to a syringe and a microcatheter according to one or more embodiments shown and described herein;
FIG. 4A is a partial cross-sectional view of the handle assembly of FIG. 1 in a default position according to one or more embodiments shown and described herein, the cross-section taken along line 4A-4A of FIG. 2;
FIG. 4B is a partial cross-sectional view of the handle assembly of FIG. 1 in an actuated position according to one or more embodiments shown and described herein, the cross-section taken along line 4B-4B of FIG. 2;
FIG. 5 is a perspective view of a handheld delivery device according to one or more embodiments shown and described herein;
FIG. 6 is a partially-exploded perspective view of the handheld delivery device of FIG. 5 including a syringe assembly according to one or more embodiments shown and described herein;
FIG. 7 is a cross-sectional view of the handheld delivery device of FIG. 5 according to one or more embodiments shown and described herein;
FIG. 8 is a perspective view of flushing syringe to be coupled to the handheld delivery device of FIG. 5 according to one or more embodiments shown and described herein;
FIG. 9 is a perspective view of another handheld delivery device according to one or more embodiments shown and described herein;
FIG. 10 is a cross-sectional view of the handheld delivery device of FIG. 9 with multiple syringes received therein, the multiple syringes being manually and electronically actuated, the cross-section taken along line 10-10 of FIG. 9;
FIG. 11 is a perspective view of another handheld delivery device according to one or more embodiments shown and described herein;
FIG. 12 is a cross-sectional view of the handheld delivery device of FIG. 11 with multiple syringes received therein, the multiple syringes being electronically actuated, the cross-section taken along line 12-12 of FIG. 11;
FIG. 13 is a perspective view of a delivery device according to the invention including a protective shield and a vial sled according to one or more embodiments shown and described herein;
FIG. 14 is a partial perspective view of the delivery device of FIG. 13 including a mechanical assembly according to one or more embodiments shown and described herein;
FIG. 15 is a cross-sectional view of the vial sled of FIG. 13 according to one or more embodiments shown and described herein, the cross-section along line 15-15 of FIG. 13;
FIG. 16 is a perspective view of the vial sled of FIG. 13 with a battery pack removed therefrom according to one or more embodiments shown and described herein;
FIG. 17 is a perspective view of a priming assembly of the vial sled of FIG. 13 according to one or more embodiments shown and described herein;
FIG. 18 is a perspective view of a vial assembly including an engagement head according to one or more embodiments shown and described herein;
FIG. 19A is a perspective view of an alternative engagement head of the vial assembly of FIG. 18 according to one or more embodiments shown and described herein;
FIG. 19B is a perspective view of an alternative engagement head of the vial assembly of FIG. 18 according to one or more embodiments shown and described herein;
FIG. 19C is a perspective view of an alternative engagement head of the vial assembly of FIG. 18 according to one or more embodiments shown and described herein;
FIG. 20 is a partial cross-sectional view of the vial assembly of FIG. 18, the cross-section taken along line 20-20 of FIG. 18;
FIG. 21 is a perspective view of a sterile container assembly according to one or more embodiments shown and described herein;
FIG. 22 is a cross-sectional view of the sterile container assembly of FIG. 21 with the vial assembly of FIG. 18 stored therein according to one or more embodiments shown and described herein, the cross-section taken along line 22-22 of FIG. 21;
FIG. 23 is a perspective view of the delivery device of FIG. 13 with the protective shield removed therefrom and a lever arm of the delivery device actuated according to one or more embodiments shown and described herein;
FIG. 24 is a perspective view of the vial sled of FIG. 13 with the priming assembly of FIG. 17 removed therefrom according to one or more embodiments shown and described herein;
FIG. 25 is a perspective view of the vial sled of FIG. 13 with the vial assembly of FIG. 18 inserted therein according to one or more embodiments shown and described herein;
FIG. 26A is a partial cross-sectional view of the vial assembly of FIG. 18 inserted into the vial sled of FIG. 13 at an initial locking position, with the cross-section taken along line 26-26 of FIG. 25;
FIG. 26B is a partial cross-sectional view of the vial assembly of FIG. 18 inserted into the vial sled of FIG. 13 at a full locking position, with the cross-section taken along line 26-26 of FIG. 25;
FIG. 27 is a partial-perspective view of the vial sled coupled to the delivery device of FIG. 13 with the lever arm coupled to the vial assembly of FIG 18 according to one or more embodiments shown and described herein;
FIG. 28A is a schematic view of a display interface of the delivery device of FIG. 13 according to one or more embodiments shown and described herein;
FIG. 28B is another schematic view of a display interface of the delivery device of FIG. 13 according to one or more embodiments shown and described herein;
FIG. 29 is a perspective view of the vial sled coupled to the delivery device of FIG. 13, with the lever arm coupled to the vial assembly of FIG. 18 and translated to an extended position according to one or more embodiments shown and described herein;
FIG. 30 is a perspective view of the vial sled of FIG. 13 with the vial assembly of FIG. 18 received therein, with a series of delivery lines coupled to the vial sled according to one or more embodiments shown and described herein;
FIG. 31 is a perspective view of the vial sled coupled to the delivery device of FIG. 13, with the lever arm coupled to the vial assembly of FIG. 18 and translated to a lowered position according to one or more embodiments shown and described herein;
FIG. 32 is a perspective view of the delivery device of FIG. 13 with the protective shield and the vial sled removed therefrom according to one or more embodiments shown and described herein;
FIG. 33 is a flow diagram of an exemplary method of delivering a radioative dose with the delivery device of FIG. 13;
FIG. 34 is a perspective view of an alternative plunger for use with the vial assembly of FIG. 18 according to one or more embodiments shown and described herein;
FIG. 35 is a cross-sectional view of an alternative plunger for use with the vial assembly of FIG. 18 according to one or more embodiments shown and described herein;
FIG. 36A is a cross-sectional view of the plunger of FIG. 35 in a partially extended position relative to the vial assembly of FIG. 18 according to one or more embodiments shown and described herein;
FIG. 36B is a cross-sectional view of the plunger of FIG. 35 in a fully extended position relative to the vial assembly of FIG. 18 according to one or more embodiments shown and described herein;
FIG. 37 is a perspective view of an alternative plunger for use with the vial assembly of FIG. 18 according to one or more embodiments shown and described herein;
FIG. 38A is a perspective view of the plunger of FIG. 37 in a first orientation according to one or more embodiments shown and described herein;
FIG. 38B is a perspective view of the plunger of FIG. 37 in a second orientation according to one or more embodiments shown and described herein;
FIG. 39A is a cross-sectional view of an alternative vial assembly in a first configuration according to one or more embodiments shown and described herein; and
FIG. 39B is a cross-sectional view of the vial assembly of FIG. 39A in a second configuration according to one or more embodiments shown and described herein.

### DETAILED DESCRIPTION

Reference will now be made in detail to various embodiments of delivery devices for administering radioactive compounds to a patient, examples of which are illustrated in the accompanying drawings. Whenever possible, the same reference numerals will be used throughout the drawings to refer to the same or like parts. Directional terms as used herein-for example up, down, right, left, front, back, top, bottom, distal, and proximal-are made only with reference to the figures as drawn and are not intended to imply absolute orientation.

Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

Unless otherwise expressly stated, it is in no way intended that any method set forth herein be construed as requiring that its steps be performed in a specific order, nor that with any apparatus specific orientations be required. Accordingly, where a method claim does not actually recite an order to be followed by its steps, or that any apparatus claim does not actually recite an order or orientation to individual components, or it is not otherwise specifically stated in the claims or description that the steps are to be limited to a specific order, or that a specific order or orientation to components of an apparatus is not recited, it is in no way intended that an order or orientation be inferred, in any respect. This holds for any possible non-express basis for interpretation, including: matters of logic with respect to arrangement of steps, operational flow, order of components, or orientation of components; plain meaning derived from grammatical organization or punctuation, and; the number or type of embodiments described in the specification.

Unless otherwise defined, all technical and scientific terms used herein have the same meaning as commonly understood by one of ordinary skill in the art to which the invention belongs. The terminology used in the description herein is for describing particular embodiments only and is not intended to be limiting. As used in the specification and appended claims, the singular forms "a," "an," and "the" are intended to include the plural forms as well, unless the context clearly indicates otherwise.

As used herein, the terms "horizontal," "vertical," "distal" and "proximal" are relative terms only, are indicative of a general relative orientation only, and do not necessarily indicate perpendicularity. These terms also may be used for convenience to refer to orientations used in the figures, which orientations are used as a matter of convention only and are not intended as characteristic of the devices shown. The present invention and the embodiments thereof to be described herein may be used in any desired orientation. Moreover, horizontal and vertical walls need generally only be intersecting walls, and need not be perpendicular. As used herein, the singular forms "a," "an" and "the" include plural referents unless the context clearly dictates otherwise. Thus, for example, reference to "a" component includes aspects having two or more such components, unless the context clearly indicates otherwise.

### I. Mechanical Delivery Device

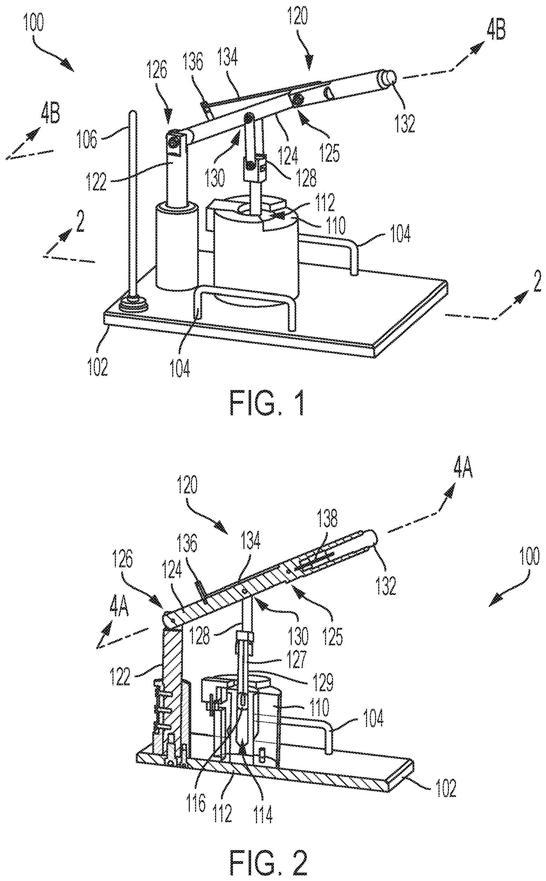

Referring now to FIGS. 1-4, one embodiment of a delivery device **100** is depicted that is configured and operable to deliver a radioactive material (e.g., radioembolizing beads) while reducing radioactive emissions during use of the delivery device **100.** Specifically referring to FIG. 1, the delivery device **100** comprises a base (base plate) **102,** a primary housing **110,** and a handle assembly **120.** Base **102** includes a pair of handles **104** that are configured to facilitate selective positioning of the delivery device **100** during a medical procedure. The base **102** is formed of a radiation shielding material such that any radioactive fluid media stored within the delivery device **100** is effectively shielded from any objects positioned relatively beneath the base **102,** thereby minimizing exposure of the radioactive material contained therein. By way of example only, the radiation shielding material of the base **102** may be formed of any combination of plastics, metals, and/or the like. As merely an illustrative example, the base **102** may be formed of acrylonitrile butadiene styrene (ABS), tungsten, pewter, lead, tin, and various other suitable materials configured to inhibit radioactive emissions. The base **102** further includes an elongated member **106** that is configured to provide a mechanical connection point for holding a refilling agent or mixing fluid. By way of example, the elongated member **106** is configured to hold an encasement device, such as a bag and/or syringe filled with one or more fluid mediums therein (e.g., saline, contrast media, etc).

Although the base **102** of the delivery device **100** is shown and described herein as having a squared and/or rectangular shape and defining a planar surface, in other embodiments the base **102** may include various other shapes, sizes, and/or profiles. Additionally, in some embodiments the base **102** may be omitted from the delivery device **100** entirely without departing from the scope of the present disclosure. The primary housing **110** is integral with the base **102** such that the primary housing **110** is fixedly secured to the base **102.** Similar to the base **102,** the primary housing **110** may be formed of a radiation shielding material that is configured and operable to inhibit radioactive emissions therethrough. As will be described in greater detail below, primary housing **110** is sized and shaped to store radioembolizing beads and/or particles within a central chamber (reservoir) **112** of primary housing **110.** In some examples, primary housing **110** may be formed of a clear material that is operable to provide a magnifying effect for enhanced visualization of the radioembolizing beads contained therein. The material of the primary housing **110** may also be shielding to radiation such as beta particles, x-rays, gamma particles, and/or the like. By way of example only, primary housing **110** may be formed of a polycarbonate. In some embodiments, the primary housing **110** may include a viewing window thereon, where the viewing window is formed of a clear material to facilitate a visualization of the contents disposed within primary housing **110.** The clear material may be further formed of a radioactive shielding material that is configured and operable to inhibit radioactive emissions therethrough.

Handle assembly **120** is configured to provide a mechanical system for delivering radioembolizing beads from delivery device **100** to a patient. In particular, handle assembly **120** provides a greater range of motion, relative to the handle of a syringe that is proportional to an amount of radioactive material to be delivered to a patient, thereby providing an operator with a more accurate sense of a dose of radioactive material being delivered from the delivery device **100.** The configuration and length of handle assembly **120** provides additional distance between the hand of the operator and the radioactive material contained within primary housing **110** to thereby reduce radiation exposure to an operator of the delivery device **100.**

Handle assembly **120** comprises a vertical column **122** integral with base **102.** Vertical column **122** extends vertically from base **102** such that vertical column **122** is oriented perpendicularly relative to base **102.** Handle assembly **120** further comprises an elongated lever **124** having a proximal end **125** and a distal end **126.** Elongated lever **124** is pivotably coupled to vertical column **122** at distal end **126** such that proximal end **125** of elongated lever **124** is configured to pivot relative to base **102** about distal end **126.** Elongated lever **124** includes a plunger **128** extending toward base **102** from an intermediate junction **130** of elongated lever **124** positioned between proximal end **125** and distal end **126.** Plunger **128** is configured to translate relative to base **102** when elongated lever **124** pivots about distal end **126.** As will be described in greater detail below, plunger **128** is slidably received within a central chamber **112** of primary housing **110** such that plunger **128** is configured to access the radioactive material contained therein.

Handle assembly **120** further comprises a handle (actuator) **132** that is pivotably coupled to elongated lever **124** at proximal end **125.** Handle **132** is sized and shaped to be selectively maneuverable about proximal end **125** of elongated lever **124.** In this instance, movement of the handle **132** relative to proximal end **125** is operable to simultaneously pivot handle **132** about elongated lever **124** and pivot the elongated lever **124** about distal end **126.** Accordingly, plunger **128** is configured to translate relative to primary housing **110** and base **102** in response to elongated lever **124** pivoting about distal end **126**,. Thus, actuation of handle **132** is operable to translate plunger **128** into primary housing **110.** Although not shown, it should be understood that plunger **128** may include a plurality of markings along a longitudinal length of plunger **128** to provide visual feedback of a displacement of plunger **128** relative to primary housing **110.** Additionally or alternatively, in some versions, the delivery device **100** may include a sensor (e.g., linear encoder) that detects or measure linear movement of plunger **128** into central chamber **112.** In some versions, delivery device **100** may comprise a locking mechanism configured to engage plunger **128** to thereby releasably fix the plunger **128** at a position relative to primary housing **110.**

In some versions, movement of handle assembly **120** may be automated with a stepper or motor (not shown) to facilitate reproducible flow rates, volumes, or other process parameters. With automated movement of the handle assembly **120,** an operator can operate the delivery device **100** hands-free, thereby further reducing potential radiation exposure and potential for human error.

Referring still to FIG. 1, elongated lever **124** includes a marker **134** attached to handle **132** and an interface display **136** attached to elongated lever **124** adjacent to distal end **126.** Marker **134** and interface display **136** are cooperatively configured to generate a visual feedback to an operator indicating real time information pertaining to a flow rate administered by delivery device **100.** In the embodiment of FIG. 1, interface display **136** includes a series of indicators and is configured to correspond a deflection of handle **132** to either an amount of force or a range of delivery flow rate of the radioactive material. As evident in the view of FIG. 2, with handle **132** oriented parallel relative to elongated lever **124,** interface display **136** extends toward a bottom portion of marker **134** such that interface display **136** is positioned adjacent a portion of interface display **136** that represents an acceptable degree of deflection of handle **132,** and thus an acceptable flow rate. In other embodiments, the interface display **136** may comprise a scale, a ruler, a digital display, a remote smart device, a tablet, and/or the like.

In contrast, referring back to FIG. 1, with handle **132** oriented transversely relative to elongated lever **124,** interface display **136** extends toward a top portion of marker **134** such that interface display **136** is positioned adjacent a portion of interface display **136** that represents an excess degree of deflection of handle **132,** i.e. an unacceptable flow rate. As merely an illustrative example only, the series of indicators on interface display **136** may comprise indicia such as a plurality of colors (e.g., green, yellow, orange, red, etc.), a plurality of numbers (e.g., 1 through 5), or other measurable indicia as will be apparent to those of ordinary skill in the art. Alternatively, in other versions delivery device **100** may include an accelerometer or displacement sensor, in lieu of or in addition to marker **134** and interface display **136,** such that the accelerometer or displacement sensor is configured to correspond a deflection of handle **132** to either an amount of force or a range of delivery flow rate of the radioactive material.

In the delivery device **100** of FIG. 2, the primary housing **110** including central chamber **112** that is sized and shaped to slidably receive plunger **128** therein. As briefly described above, plunger **128** is configured to translate through central chamber **112** of primary housing **110** in response to actuation (i.e. pivot) of handle **132** about distal end **126** of elongated lever **124.** Plunger **128** includes a needle **129** disposed therein. The needle **129** is configured to slidably translate within central chamber **112** as plunger **128** translates relative to primary housing **110.** As further seen in FIG. 2, primary housing **110** includes a vial compartment **114** at a bottom end of central chamber **112.** Vial compartment **114** is sized and shaped to store therapeutic particles (e.g., radioembolizing beads, radioactive particles, microspheres, etc.) therein. Vial compartment **114** is isolated from the remaining portion of central chamber **112** by a protective seal **116** disposed therein between vial compartment **114** and the remainder of central chamber **112.** Thus, the therapeutic particles disposed within vial compartment **114** are not in fluidic communication with the remaining portions of primary housing **110,** because protective seal **116** is configured to generate a protective barrier between central chamber **112** and vial compartment **114.**

Although not shown, it should be understood that base **102** may further comprise a quick release mechanism positioned beneath primary housing **110.** In particular, the quick release mechanism may be sized and shaped to remove vial compartment **114** from within central chamber **112** of primary housing **110.**

Needle **129** is configured to puncture the protective seal **116** in response to translation of plunger **128** through central chamber **112.** In this instance, access to the therapeutic particles within vial compartment **114** is established when handle **132** of elongated lever **124** is pivoted about distal end **126** to an extent corresponding to the displacement between needle **129** and protective seal **116.** Additionally, although not shown, it should be understood that plunger **128** may also include a sterile barrier mechanism proximate to needle **129** that is configured to sterilize the area of contact between needle **129** and protective seal **116.** In this instance, the sterile barrier mechanism is operable to minimize potential contamination of protective seal **116** when needle **129** contacts protective seal **116** to access the therapeutic particles within vial compartment **114.**

By way of example only, the sterile barrier mechanism may comprise a removable Tyvek^{®} disk. With the sterile barrier mechanism positioned proximate to protective seal **116,** the necessity to wipe needle **129** with alcohol prior to advancing needle **129** into vial compartment **114** is removed. Needle **129** includes a plurality of side holes (not shown) along the longitudinal length of needle **129.** The side holes (not shown) are configured to generate turbulence within vial compartment **114** as needle **129** extends therein, thereby mixing the therapeutic particles contained therein. The side holes of needle **129** provide access to a central lumen **127** of needle **129** that extends along a longitudinal length of needle **129.** As described in greater detail herein, the central lumen **127** of needle **129** is configured to receive a fluid medium (e.g., saline) from a fluid reservoir fluidly coupled thereto such that the fluid medium is transferred into the vial compartment **114** via the plurality of side holes as the needle **129** translates downward into the central chamber **112** of the primary housing **110** in response to generating a positive pressure therein.

Although not shown, it should be understood that vial compartment **114,** plunger **128,** and/or needle **129** may include plurality of outwardly protruding flaps, outwardly protruding ribs, or other outwardly protruding features configured to further promote the mixture of the radioembolizing beads and the fluid medium as needle **129** and plunger **128** are advanced into vial compartment **114.** Additionally or alternatively, delivery device **100** may further include a stir bar (not shown) that is operable to enhance mixing of the radioembolizing beads and the fluid medium within vial compartment **114.**

In some versions, delivery device **100** may include a plurality of abutments (not shown) within central chamber **112** of primary housing **110.** The plurality of abutments may extend into central chamber **112** and be configured to releasably engage plunger **128** as plunger **128** is translated through central chamber **112** to thereby generate a plurality of stopping points. In this instance, the plurality of abutments temporarily inhibit advancement of plunger **128** into primary housing **110** to thereby provide a tactile feedback to an operator for managing dose control. The tactile feedback experienced at the plurality of stopping points indicate to an operator of the displacement of plunger **128** relative to primary housing **110,** thereby informing the operator of the sphere concentration, flow rate, and/or a torque or pressure to be delivered by delivery device **100.** In other examples, delivery device **100** may include a stir bar (not shown) within vial compartment **114** and/or central chamber **112** to promote mixing of the radioembolizing beads and the fluid medium received therein.

Referring still to FIG. 2, elongated lever **124** further includes a torque coupling member **138** disposed within elongated lever **124** and handle **132** such that torque coupling member **138** extends between elongated lever **124** and handle **132.** In other words, torque coupling member **138** is configured to couple proximal end **125** of elongated lever **124** to handle **132.** In the present example, torque coupling member **138** is a resiliently biased spring that is configured to bias handle **132** in a substantially parallel orientation relative to a longitudinal length of elongated lever **124,** as is evident in FIG. 2. In this instance, releasing handle **132** returns handle **132** to a default position in parallel orientation with elongated lever **124** such that torque coupling member **138** is operable to suspend plunger **128** in a retracted position relative to central chamber **112** and withdraw needle **129** from contacting the protective seal **116.** As will be described in greater detail below, torque coupling member **138** is configured to resist lateral movement of handle **132** toward base **102** such that a predetermined force is required to actuate handle **132.**

Torque coupling member **138** provides volumetric flow rate, or alternatively volume speed control, during delivery of the radioactive material from delivery device **100** to a patient. In particular, torque coupling member **138** correlates a deflection of the handle **132** to a flow rate generated by the delivery device **100.** In other versions, it should be understood that torque coupling member **138** may be configured to bias the handle **132** in a substantially transverse orientation relative to a longitudinal length of elongated lever **124,** as seen in FIG. 1. In this instance, releasing handle **132** returns handle **132** to a default position in transverse orientation with the elongated lever **124** such that torque coupling member **138** is operable to advance the plunger **128** into an extended position relative to central chamber **112** with needle **129** punctured through protective seal **116.**

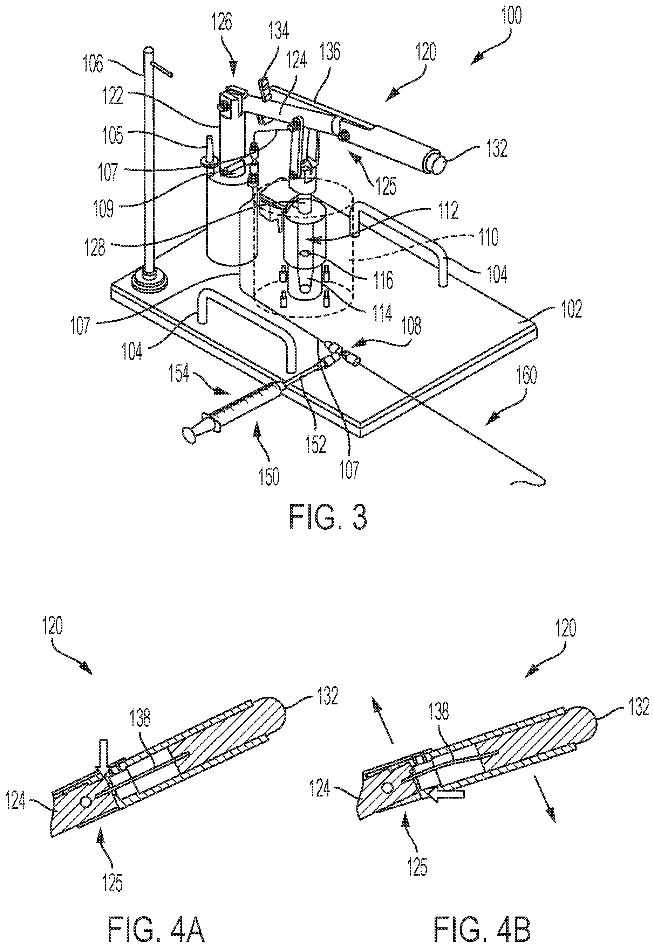

Operation of the delivery device **100** will now be described with reference to FIG. 3. In particular, an operator selectively positions delivery device **100** in an operating room adjacent to a patient by maneuvering the base **102** via handles **104.** With delivery device **100** positioned at a desired location, an operator couples a contrast syringe **150,** optionally, and a catheter **160** to the delivery device **100** via a first connector valve **108.** In particular, first connector valve **108** is a three-way check valve (also known as a T-valve connector) such that a contrast line **152** is connected to contrast syringe **150** at one end and first connector valve **108** at an opposite end. In the present example, contrast syringe **150** includes a contrast medium stored therein, however, it should be understood that contrast syringe **150** may include various other fluid media as will be apparent to those of ordinary skill in the art. Contrast syringe **150** further comprises a plurality of markings **154** along the body of the contrast syringe **150** to thereby indicate to an operator a current volume of contrast medium stored therein. Although not shown, it should be understood that contrast syringe **150** may be coupled to a syringe pump or power injector that is configured to automate the actuation of contrast syringe **150.** In this instance, delivery of the contrast medium stored in the contrast syringe **150** may be administered at reliable and consistent flow rates.

Catheter **160** is similarly coupled to first connector valve **108** such that contrast syringe **150** is in fluidic communication with catheter **160.** In this instance, delivery device **100** is coupled to first connector valve **108** via a delivery line **107** that is connected to first connector valve **108** at one end and to a second connector valve **109** at an opposite end. In the present example, catheter **160** is a microcatheter sized and shaped to intravenously establish fluidic communication between a target treatment site and delivery device **100.** Similar to first connector valve **108,** second connector valve **109** is a three-way check valve (also known as a T-valve connector) such that delivery line **107** from first connector valve **108** is coupled to second connector valve **109** at a first end and a fluid reservoir line **105** is attached thereon at another end.

Fluid reservoir line **105** is coupled to a fluid reservoir (not shown) that may comprise a bag or chamber configured to store a fluid medium therein. In the present example, the fluid reservoir contains saline or a contrast medium therein. By way of example only, the fluid reservoir is configured to store an intravenous sugar solution, such as dextrose solution (D5W). It should also be understood that delivery lines **107** and connector valves **108, 109** are sized and shaped to include smooth diameter transitions or interfaces at their intersection points to thereby minimize dead volumes and the potential for sphere settling in the tubing system.

Needle **129** is similarly coupled to second connector valve **109** (*see* FIG. 3) via another delivery line **107** such that needle **129** of delivery device **100** establishes fluidic communication with contrast syringe **150,** catheter **160,** and fluid reservoir line **105.** The central lumen **127** of needle **129** may be coupled to delivery line **107** such that needle **129** is in fluidic communication with second connector valve **109.** In this instance, fluid reservoir line **105** is in communication with central lumen **127** of needle **129** via the second connector valve **109** such that central lumen **127** is operable to receive a fluid medium from the fluid reservoir (not shown) attached to fluid reservoir line **105.**

As will be described in greater detail below, advancement of needle **129** downward through central chamber **112** of primary housing **110** generates a negative pressure through the central lumen **127** due to a downward translation of the needle **129.** In this instance, the delivery line **107** fluidly coupled to the central lumen **127,** which provides fluid communication between the central lumen **127** and the fluid reservoir line **105** via the second connector valve **109** coupled therebetween, causes a negative pressure to similarly be generated within the delivery line **107** and the second connector valve **109.** A fluid medium stored within a fluid reservoir (not shown) that is coupled to the fluid reservoir line **105** is drawn from the fluid reservoir and through fluid reservoir line **105** as a result of the negative pressure generated by the needle **129** and transferred to the fluid reservoir by the lines **105, 107** and second connector valve **109** fluidly coupled therebetween. Accordingly, the fluid medium is transferred through into central lumen **127** via second connector valve **109** and delivery line **107.** It should be understood that the delivery line **107** extending from the second connector valve **109** extends through a top end of the plunger **128** and into a top end of the central lumen **127** of the needle **129.** With the needle **129** slidably received within the central chamber **112** of the primary housing **110** and the delivery line **105** fluidly coupled to the central lumen **127** of the needle **129,** the delivery line **105** is effectively in fluid communication with central chamber **112** of the primary housing **110.**

In exemplary treatment procedures for expelling the radioembolizing beads, an operator may actuate delivery device **100** by exerting a downward force onto handle **132** relative to base **102** to thereby pivot handle **132** about proximal end **125** and pivot the elongated lever **124** about distal end **126.** As briefly described above, torque coupling member **138** is resiliently biased to inhibit downward movement of handle **132** toward base **102** such that handle **132** is biased toward a parallel configuration with elongated lever **124,** as best seen in FIG. 4A. In this instance, an operator may apply a predetermined force onto handle **132** to overcome the resilient bias of torque coupling member **138** and thereby actuate elongated lever **124,** as seen in FIG. 4B. Application of a consistent force onto handle **132** may overcome the resilient bias of torque coupling member **138** thereby slidably translating the plunger **128** downward through central chamber **112** of primary housing **110.**

Needle **129** is already penetrated through protective seal **116** and in fluidic communication with the radioembolizing beads contained in vial compartment **114** such that the downward translation of plunger **128** advances needle **129** toward a bottom surface of vial compartment **114.** As briefly described above, prior to a fluid medium being suctioned into central lumen **127,** needle **129** is advanced upward relative to vial compartment **114** thereby generating a negative pressure therein by the actuation of handle **132.** In this instance, the fluid medium is effectively dispersed from a fluid reservoir and to central lumen **127** of needle **129** as needle **129** translates upward relative to the bottom surface of vial compartment **114.** Effectively, the bottom surface of vial compartment **114** serves as a refill starting point for delivery device **100.**

The plurality of side holes along needle **129** provide for mixing the fluid medium received within the central lumen **127** into the therapeutic particles (e.g., radioembolizing beads) stored within the vial compartment **114.** By exerting a downward force, with vial compartment **114** now in fluidic communication with central lumen **127** of needle **129,** the fluid medium is effectively expelled from central lumen **127** of needle **129** and into vial compartment **114** in response to a positive pressure being generated therein when the needle **129** translates relatively downward through the central chamber **112.** The radioembolizing bead concentration per delivery cycle can be defined depending on the chosen refill volume. In this instance, plunger **128** is lowered through central chamber **112** together with needle **129** into vial compartment **114.** The mixture of therapeutic particles (e.g., radioembolizing beads) and fluid medium, collectively referred to as a suspension fluid or liquid, is thereby injected through central lumen **127** and toward first connector valve **108** via the interconnected system of second connector valve **109** and delivery lines **107.**

With needle **129** fully advanced into vial compartment **114** (i.e. the refill starting point), handle **132** is ready for refilling delivery device **100.** Handle **132** is lifted up relative to base **102** and remains in a default orientation where handle **132** is substantially parallel with the longitudinal length of elongated lever **124** due to an end stop (not shown) present in the pivot region between handle **132** and elongated lever **124.** In this instance, the function of torque coupling member **138** is bypassed. Alternatively, handle **132** may simply be released such that the downward force applied onto handle **132** is removed. In this instance, the resilient bias of torque coupling member **138** returns handle **132** to the default orientation where handle **132** is substantially parallel with the longitudinal length of elongated lever **124.** In this instance, plunger **128** is retracted through central chamber **112** thereby withdrawing needle **129** from vial compartment **114.** Retraction of plunger **128** and needle **129** generates a negative pressure within vial compartment **114** such that the mixture of radioembolizing beads and fluid medium is extracted through central lumen **127** and toward first connector valve **108** via the interconnected system of second connector valve **109** and delivery lines **107.**

As the mixture medium is being transferred toward first connector valve **108,** an operator may actuate the contrast syringe **150** to thereby transfer a contrast medium through contrast line **152** and toward first connector valve **108** thereby mixing the various media together at first connector valve **108** prior to delivery to catheter **160.** An operator may repeatedly actuate handle **132** to continue filling and flushing a mixture of radioembolizing beads, the fluid medium, and/or a contrast medium into catheter **160** by the pressurization means described above.

### II. Manual Handheld Delivery Device

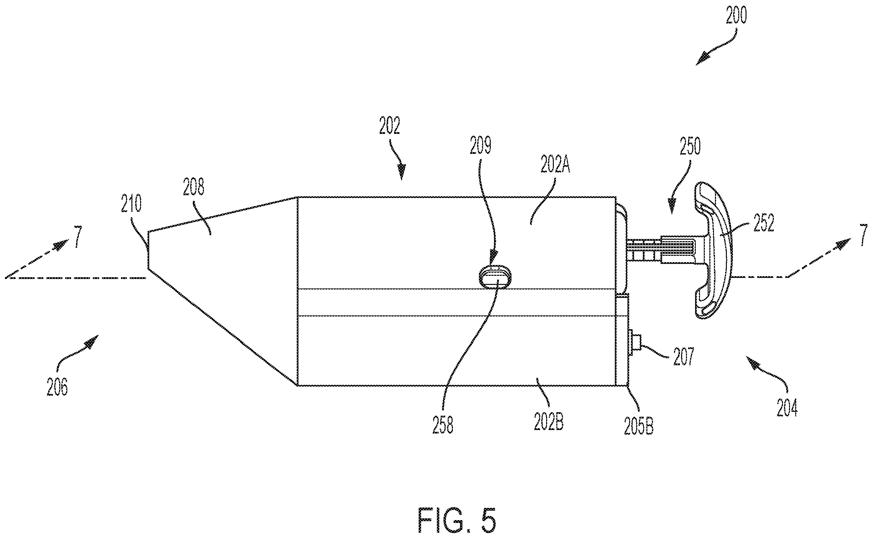

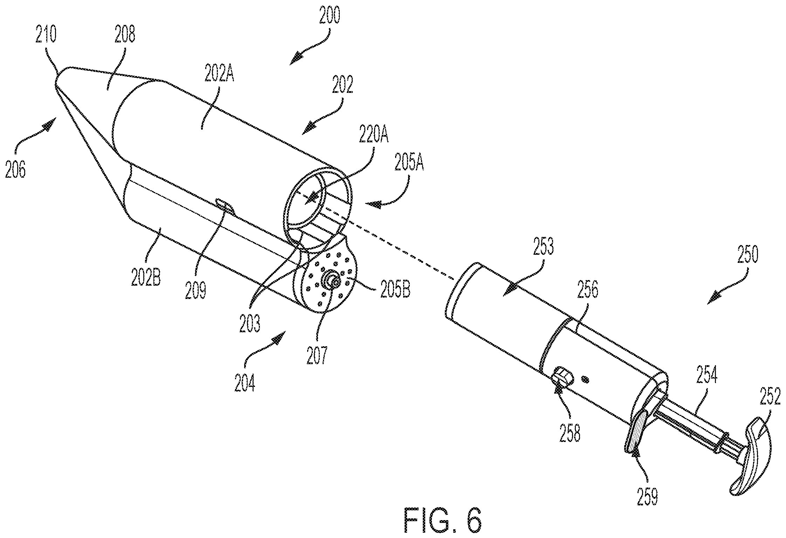

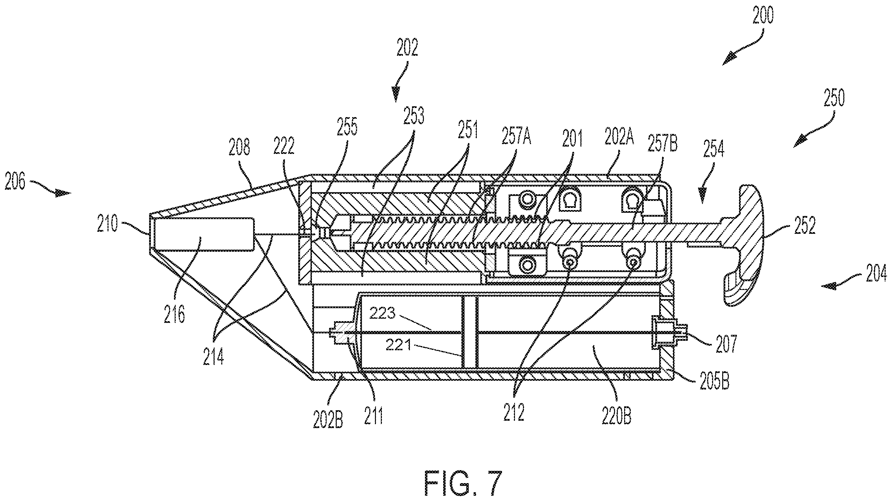

FIGS. 5-7 show another embodiment of a delivery device **200** configured and operable to deliver a radioactive material (e.g., radioembolizing beads) while reducing radioactive emissions during use of the delivery device **200.** Referring specifically to FIG. 5, the delivery device **200** comprises a housing **202** extending between a proximal end **204** and a distal end **206.** The housing **202** includes a pair of chambers **202A, 202B** disposed therein, and in particular at least one chamber **202A** that defines an internal cavity **220A** *(See* FIG. 6) that is sized and shaped to receive a device therein, and at least one chamber **202B** that defines another internal cavity **220B** (*see* FIG. 7) for storing a fluidic substance therein (e.g., saline). In some embodiments, the chamber **202B** may be formed of a translucent material such that the fluidic substance stored within the internal cavity **220B** may be visible from an exterior of the delivery device **200.** In other embodiments the internal cavity **220B** of the second chamber **202B** is sized and shaped to receive a device therein, such as, for example, an external fluid reservoir.

In particular, the internal cavity **220A** of the chamber **202A** is sized and shaped to receive a vial assembly **250** within the delivery device **200.** It should be understood that the internal cavity **220A** of the chamber **202A** may include one or more retention mechanisms that are configured to selectively lock the vial assembly **250** to the delivery device **200** such that vial assembly **250** is securely retained within the internal cavity **220A** during use of the delivery device **200.** Actuation of the retention mechanism may provide for a selective removal of the vial assembly **250** from the internal cavity **220A** of the chamber **202A** such that after use of the delivery device **200** the vial assembly **250** may be disposed of separate from the delivery device **200.**

In the present example, the retention mechanism of the delivery device **200** comprises an aperture **209** positioned along the housing **202,** and in particular, disposed through the chamber **202A.** The aperture **209** of the delivery device **200** is sized and shaped to receive a corresponding retention mechanism of the vial assembly **250.** In particular, the corresponding retention mechanism of the vial assembly **250** comprises a depressible button **258** such that the aperture **209** receives the depressible button **258** when the vial assembly **250** is slidably received through the internal cavity **220A** of the chamber **202A.** As will be described in greater detail herein, the depressible button **258** is configured to resiliently expand outward from the vial assembly **250** in response to an alignment of the depressible button **258** with the aperture **209** as the vial assembly **250** is translated through the chamber **202A.** In other embodiments, the retention mechanisms of the internal cavity **220A** may be configured to permanently secure the vial assembly **250** to the delivery device **200** such that the vial assembly **250** is not subsequently removable from the internal cavity **220A** of the chamber **202A.** In this instance, the delivery device **200** is disposable together with the vial assembly **250.**

Still referring to FIG. 5, with the vial assembly **250** fully positioned within the internal cavity **220A** of the chamber **202A,** a handle **252** of the vial assembly **250** extends proximally from the housing **202** at the proximal end **204,** such that vial assembly **250** is not fully contained within the internal cavity **220A** of the chamber **202A.** In this instance, the handle **252** of the vial assembly **250** is accessible to an operator of the delivery device **200** when the vial assembly **250** is fully assembled in the delivery device **200.** As will be described in greater detail below, with the handle **252** of the vial assembly **250** extending outwardly from and accessible at the proximal end **204** of the delivery device **200,** the vial assembly **250** may be actuated by an operator while the vial assembly **250** is securely received within the internal cavity **220A** of the chamber **202A.** The housing **202** further includes a distal head **208** that is integrally formed with the pair of chambers **202A, 202B** of the housing **202.** The distal head **208** includes a tapered profile relative to an elongated profile of the pair of chambers **202A, 202B.** In particular, the distal head **208** tapers distally toward the distal end **206** of the delivery device **200** to a catheter hub **210** of the delivery device **200.** Alternatively, in other embodiments the catheter hub **210** may include tubing and/or standard connections configured to couple the delivery device **200** to various devices.

The catheter hub **210** is configured to couple the delivery device **200** to a device, such as, for example, a catheter (not shown), to thereby facilitate fluidic communication between the delivery device **200** and the device. For example, the catheter hub **210** may comprise a luer fitting that is selectively engageable with a corresponding luer fitting of a device (e.g., a catheter) to thereby couple the delivery device **200** to the device at the catheter hub **210.** It should be understood that the internal cavities **220A, 220B** of the chambers **202A, 202B** of the delivery device **200** may comprise various other sizes and shapes than those shown and described herein to accommodate additional devices (e.g., the vial assembly **250**) and/or fluid medias therein without departing from the scope of the present disclosure.

Still referring to FIG. 5, the housing **202** is further sized and shaped to accommodate the maneuverability of the delivery device **200** such that the delivery device **200** is configured to be grasped by an operator. Additionally and/or alternatively, the housing **202** of the delivery device **200** may be sized and shaped to accommodate a corresponding dock and/or holding fixture. The housing **202** of the delivery device **200** may be overmolded with various materials, such as, for example, silicone, thermoplastic elastomers, thermoplastic vulcanizates, and the like. In some embodiments, the housing **202** may include, or be constructed of, a radiation shielding material such that any radioactive material contained within the delivery device **200** is sealed therein, such that exposure to radiation emissions from any radioactive material stored therein are limited to the housing **202.** By way of example only, the radiation shielding material of housing **202** may include any combination of plastics and metal. As merely an illustrative example, the housing **202** may be formed of acrylonitrile butadiene styrene (ABS), lead, tungsten, tin, pewter, or other suitable materials configured and operable to inhibit radiation emissions.

Alternatively, it should be understood that the housing **202** may be formed of other material that is not configured to shield against radiation emissions. In this instance, the delivery device **200** may include additional features that are configured to suppress radiation emissions from within the housing **202** of the delivery device **200.** For example, the delivery device **200** may include one or more radiation shield inserts positioned within the internal cavities **220A, 220B** of the pair of chambers **202A, 202B** and/or the distal head **208** to thereby reduce radiation exposure from within the housing **202.** By way of example only, the one or more radiation shield inserts may be formed of acrylonitrile butadiene styrene (ABS), lead, tungsten, tin, pewter, or other suitable materials configured and operable to inhibit radiation emissions. Additionally or alternatively, in some versions the delivery device **200** may be over molded with a radioactive shielding material. As merely an illustrative example, this material may comprise silicone, thermoplastic elastomer, thermoplastic vulcanizates, or other suitable materials configured and operable to inhibit radiation emissions.

The vial assembly **250** may be formed of a material comprising plastic, thermoplastic polymers, polycarbonate, polyethylene, polyethylene terephthalate, and the like. As will be described in greater detail herein, in some embodiments at least a portion of the vial assembly **250** that is removably received within the housing **202** of the delivery device **200** may be formed of a material and/or includes features (e.g., a protective shield **253,** *see* FIG. 7) configured and operable to inhibit radiation exposure from a substance stored therein. In this instance, the housing **202** of the delivery device **200** may be formed of a plastic.

Referring now to FIG. 6, the delivery device **200** is depicted with the vial assembly **250** removed from within the internal cavity **220A** of the chamber **202A.** In particular, the housing **202** of the delivery device **200** includes an opening **205A** at the proximal end **204** of the chamber **202A** for receiving the vial assembly **250** therethrough. In this instance, the opening **205A** is sized and shaped to receive the vial assembly **250** such that the vial assembly **250** encloses the internal cavity **220A** of the chamber **202A** when received therein. The vial assembly **250** includes a handle **252,** a plunger **254,** and an elongated body **256** and a depressible button **258** extending laterally outward from the elongated body **256.** As briefly described above, the depressible button **258** is resiliently biased to an expanded position and is selectively depressible in response to a compression of the depressible button **258** by a predetermined force. In other words, actuation of the depressible button **258** provides for a depression of the depressible button **258** into the elongated body **256** of the vial assembly **250.** Accordingly, the depressible button **258** is configured to resiliently expand outward from the elongated body **256** of the vial assembly **250** upon terminating application of the predetermined force thereon.

The depressible button **258** is sized and shaped to be received through the aperture **209** of the housing **202** such that, in response to an alignment of the depressible button **258** with the aperture **209,** the depressible button **258** expands outwardly from the elongated body **256** and extends through the aperture **209.** In this instance, the vial assembly **250** is effectively coupled to the housing **202** of the delivery device **200** and securely disposed within the internal cavity **220A** of the chamber **202A.** It should be understood that in other embodiments the vial assembly **250** may comprise additional depressible buttons **258** along the elongated body **256** for securing the vial assembly **250** to the housing **202** of the delivery device **200.** Alternatively, in other embodiments the vial assembly **250** may include other suitable retention mechanisms that are configured and operable to attach the vial assembly **250** to the delivery device **200.** As will be described in greater detail herein, in some embodiments actuation of the depressible button **258,** and/or other buttons or mechanisms, may facilitate an actuation of the handle **252** and the plunger **254** for administering a dose from the delivery device **200.** In this instance, the depressible button **258** further serves as a safety feature in addition to a retention mechanism. As will be described in greater detail herein, in some embodiments the delivery device **200** may include one or more sensors disposed thereon, including, for example, a linear encoder. In this instance, the linear encoder may be disposed over and/or coupled to the plunger **254** such that the plunger **254** extends through the linear encoder and the linear encoder translates simultaneously with the plunger **254.**

Still referring to FIG. 6, the chamber **202B** of the housing **202** is sized and shaped to receive a fluid medium therein, and in particular, the chamber **202B** serves as a fluid reservoir for storing a fluid medium (e.g., saline) within the internal cavity **220B.** In particular, the internal cavity **220B** of the chamber **202B** may be sized to receive and store a predetermined volume of a fluid medium therein, such as that transmitted to the chamber **202B** from an external device (e.g., a syringe). By way of example, the predetermined volume of the chamber **202B** may range from about 80 milliliters (mL) to about 120 milliliters (mL), and more particularly 100 milliliters (mL).

The fluid reservoir formed by the chamber **202B** of the housing **202** may store various fluid mediums therein, such as, for example, saline, an intravenous sugar solution, dextore solutions (D5W), and/or a contrast medium. In other embodiments, the chamber **202B** may be configured to receive a fluid reservoir device within the internal cavity **220B,** such as a syringe, a bag, and/or the like. In this instance, the fluid reservoir device may be preassembled into the chamber **202B** of the housing **202,** or alternatively separate from the delivery device **200** such that an operator of the delivery device **200** is required to couple the fluid reservoir device with the housing **202.** The housing **202** further includes a proximal wall **205B** at the proximal end **204** of the chamber **202B** for enclosing the internal cavity **220B.** The proximal wall **205B** includes a port **207** extending proximally therefrom that is configured and operable to couple the internal cavity **220B** of the chamber **202B** to a corresponding device, such as, for example, a syringe (not shown). In the present example, the proximal wall **205B** includes a plurality of vents and/or holes disposed therethrough to facilitate movement of a floating septum disposed within the chamber **202B** (*see* FIG. 7) without generating a vacuum (i.e. negative pressure) therein.

Still referring to FIG. 6, the chamber **202A** of the housing **202** may further include one or more alignment features **203** disposed within the internal cavity **220A.** The alignment features **203** may extend from the chamber **202A** and into the internal cavity **220A** to interface with an exterior surface of the elongated body **256** of the vial assembly **250** to thereby align the vial assembly **250** with the chamber **202A.** In this instance, the alignment features **203** comprise an annular array of grooves extending inwardly from the chamber **202A** and into the internal cavity **220A.** It should be understood that the chamber **202A** may include various other suitable alignment features than those shown and described herein without departing from the scope of the present disclosure.

The handle **252** of the vial assembly **250** is integrally secured to the plunger **254** and the plunger **254** extends into the elongated body **256.** As will be described in greater detail herein, the plunger **254** is configured to move, and in particular rotate and translate, relative to the elongated body **256** of the vial assembly **250** in response to an actuation of the handle **252.** The vial assembly **250** includes a protective shield **253** disposed about at least a portion of the elongated body **256.** In the present example, the protective shield **253** extends about a distal segment of the elongated body **256** of the vial assembly **250,** however, it should be understood that the protective shield **253** may extend along additional and/or fewer segments of the elongated body **256** without departing from the scope of the present disclosure. Additionally, in some embodiments, the protective shield **253** of the vial assembly **250** may include a plurality of markings and/or indicia disposed along an outer surface thereon. As will be described in greater detail herein, the protective shield **253** is formed of a material configured and operable to inhibit radioactive emissions from a material stored within the elongated body **256** of the vial assembly **250.**

Still referring to FIG. 6, the vial assembly further includes a safety tab **259** coupled to the plunger **254,** and in particular along an intermediate portion of a longitudinal length of the plunger **254,** proximate to the elongated body **256.** The safety tab **259** is secured to the plunger **254** and abuts against a proximal end of the elongated body **256.** The safety tab **259** is configured to inhibit movement of the plunger **254,** and in particular a linear translation of the plunger **254** into the elongated body **256,** by engaging the elongated body **256.** The safety tab **259** is selectively removable from the vial assembly **250** in response to applying a force against the safety tab **259** opposite of the plunger **254** to thereby extract the safety tab **259** from engagement with the plunger **254** and the elongated body **256.** Accordingly, removal of the safety tab **259** provides for a translation of the plunger **254** into the elongated body **256.** In other embodiments, a safety lock may comprise a depressible handle interlocked with the handle **252,** or alternatively, an electrical switch that removes a physical impediment inhibiting the handle **252** and the plunger **254** from translating relative to the elongated body **256.**

Referring now to FIG. 7, the chamber **202B** includes a floating septum **221** disposed within the internal cavity **220B** with the floating septum **221** movably coupled to an internal tubing line **223** extending between and coupled to the ports **207, 211.** Accordingly, the floating septum **221** is translatable within the internal cavity **220B** and along the internal tubing line **223.** As will be described in greater detail herein, the floating septum **221** is configured to translate within the internal cavity **220B** of the chamber **202B,** and along the internal tubing line **223,** in response to the port **207** receiving a fluid medium therethrough and into the chamber **202B** and/or the port **211** releasing a fluid medium therethrough and out of the chamber **202B.** The vial assembly **250** is a single-chamber syringe that comprises an internal chamber **251** disposed within the elongated body **256.** The vial assembly **250** is configured to selectively deliver a fluid media contained within the elongated body **256,** and in particular an internal chamber **251,** of the vial assembly **250.** In other words, the elongated body **256** is sized to store a fluid media within the internal chamber **251** for delivery to a patient, when the vial assembly **250** is assembled to the delivery device **200,** in response to an actuation of the handle **252.** In the present example, the fluid media stored within the internal chamber **251** of the elongated body **256** comprises a radioactive material, such as, for example, radioembolizing beads, radioactive microspheres, and the like. As will be described in greater detail herein, the fluid media stored within the internal chamber **251** of the elongated body **256** may be prefilled therein prior to a use of the vial assembly **250** by an operator. The internal chamber **251** may be formed of various materials and/or include various thickness. In the present example, the internal chamber **251** is formed of a plastic and includes a wall thickness of about 9 millimeters (mm).

The vial assembly **250** is formed of a plastic material, such as, for example, polycarbonate, polyethylene, polyethylene terephthalate, or other various plastics. The internal chamber **251** of the vial assembly **250** is encapsulated within a protective shield **253** that is disposed within the elongated body **256** and extends about the internal chamber **251.** The protective shield **253** may be formed of a plastic, such as Acrylonitrile Butadiene Styrene (ABS), a lead, tungsten, tin, pewter, and/or other suitable materials for preventing exposure of the radioactive material from within the internal chamber **251.** It should be understood that the internal chamber **251** of the vial assembly **250** may be prefilled with a radioactive material prior to an assembly of the vial assembly **250** with the delivery device **200.** In this instance, the radioactive material is disposed within the protective shielding 253 of the vial assembly **250** such that radioactive emissions generated by the radioactive material is inhibited by the protective shielding 253 prior to a use of the vial assembly **250** and insertion of the vial assembly **250** into the delivery device **200.**

In other embodiments the vial assembly **250** is a dual-chamber syringe and includes at least two internal chambers **251.** In this instance, the vial assembly **250** is configured to separately maintain a fluid media within each of the chambers **251** such that the fluid media within the chambers **251** are not exposed to each other and capable of being delivered separately from the vial assembly **250** relative to one another. By way of example only, the vial assembly **250** may be configured and operable in accordance with at least some of the teachings of U.S. App. No. 62/673628, entitled "Dual Stage Syringe," filed on even date herewith.

Still referring to FIG. 7, the plunger **254** of the vial assembly **250** extends through the elongated body **256,** and in particular, is coupled to the internal chamber **251** of the vial assembly **250** opposite of the handle **252.** In particular, the plunger **254** is coupled to the internal chamber **251** such that movement of the plunger **254** generates a pressure within the internal chamber **251** for delivering a material stored therein out of the internal chamber **251.** The plunger **254** is a screw-type plunger and includes a threaded portion **257A** extending along a longitudinal length of the plunger **254.** The threaded portion **257A** of the plunger **254** is configured to mesh with a corresponding threaded portion **201** of the vial assembly **250** disposed within the elongated body **256** to facilitate a rotation of the plunger **254** therein. In this instance, a rotation of the handle **252** provides for a simultaneous rotation and linear translation of the plunger **254** through the elongated body **256** and against the internal chamber **251.**

In particular, the handle **252** is configured such that an application of a rotatable force thereon (i.e., twisting the handle **252** relative to the elongated body **256**) provides a rotation and linear translation of the plunger **254** into the elongated body **256.** In this instance, rotating the handle **252** screws the plunger **254** further along the corresponding threaded portion **201** thereby dispensing a material stored within the internal chamber **251** from the delivery device **200** as the plunger **254** applies a continued pressure onto the internal chamber **251.** Rotation of the handle **252** provides a slow and controlled rate of fluid disposition from the internal chamber **251** relative to a translation of the handle **252.**

Still referring to FIG. 7, the plunger **254** includes a non-threaded portion **257B** extending along a longitudinal length of the plunger **254** that is separate from the threaded portion **257A.** The non-threaded portion **257B** of the plunger **254** is configured to slidably engage one or more mechanisms **212** (e.g., ball bearings) disposed within the elongated body **256** that are configured and operable to facilitate a slidable translation of the plunger **254** therein. In this instance, a linear movement of the handle **252** provides for a simultaneous linear translation of the plunger **254** through the elongated body **256** and against the internal chamber **251.** In particular, the handle **252** is configured such that an application of a linear force onto the handle **252** (i.e., pushing the handle **252** relative to the elongated body **256**) provides a linear translation of the plunger **254** into the elongated body **256.** In this instance, pushing the handle **252** toward the elongated body **256** translates the plunger **254** further into the elongated body **256** and toward the internal chamber **251,** thereby dispensing a material stored therein from the delivery device **200** as the plunger **254** applies a continued pressure onto the internal chamber **251.** Translation of the handle **252** provides a fast and controlled rate of fluid disposition from the internal chamber **251** relative to a rotation of the handle **252.** It should be understood that a translation of the plunger **254** provides for a simultaneous translation of the threaded portion **257A** relative to the chamber **202A.** With the threaded portion **257A** meshed with and coupled to the corresponding threaded portion **201** of the vial assembly **250,** the plunger **254** is further configured to translate the threaded portion **201** within the chamber **202A** and relative to the internal chamber **251** of the vial assembly **250.**

The delivery device **200** further includes a fluid reservoir **216** disposed within the housing **202,** and in particular the distal head **208.** In the present example, the fluid reservoir **216** may comprise a manifold (e.g. Y-manifold), a connector valve (e.g., a three-way connector and/or T-valve connector), or various other connector mechanisms. In some embodiments, the fluid reservoir **216** may include one or more check valves to prevent a fluid medium flow in certain directions. As will be described in greater detail herein, the fluid reservoir **216** is configured to provide fluidic communication between the vial assembly **250** and the internal cavity **220B** of the chamber **202B.** Additionally, the fluid reservoir **216** is coupled to the catheter hub **210** such that vial assembly **250** and the internal cavity **220B** of the chamber **202B** are in fluidic communication with the catheter hub **210.**

Still referring to FIG. 7, the fluid reservoir **216** includes a series of delivery lines **214** (i.e., internal tubing) that extends between and fluidly couples the fluid reservoir **216** to the vial assembly **250** and the internal cavity **220B** of the chamber **202B,** respectively. In particular, at least one of the series of delivery lines **214** is coupled to a port **211** of the internal cavity **220B** of the chamber **202B,** opposite of the port **207,** such that the fluid reservoir **216** is in fluid communication with a fluid medium (e.g., saline) stored within the internal cavity **220B.** Further, at least one of the series of delivery lines **214** is coupled to a needle **222** positioned in-line and at a terminal end of the delivery line **214** opposite of the fluid reservoir **216.** In this instance, the needle **222** is positioned within the distal head **208** of the housing **202** such that the needle **222** extends into the internal cavity **220A** of the chamber **202A.**

With the needle **222** extending into the internal cavity **220A** of the chamber **202A,** it should be understood that the needle **222** is operable to couple with and engage the elongated body **256** when the vial assembly **250** is slidably received therein through the opening **205A.** In the present example, the vial assembly **250** includes a septum **255** disposed about a distal end of the elongated body **256,** with the septum **255** configured to receive the needle **222** therethrough when the elongated body **256** is received in the internal cavity **220A** of the chamber **202A.** The septum **255** is formed of an elastomer and is operable to be punctured by the needle **222,** thereby facilitating a fluid communication between the internal chamber **251** of the vial assembly **250** and the fluid reservoir **216** via the delivery line **214** coupled to the needle **222.** It should be understood that the septum **255** may be formed of various other suitable materials that are configured to securely seal the internal chamber **251** of the vial assembly **250** within the elongated body **256** while being further operable to receive the needle **222** therethrough. Although not shown, it should be understood that the fluid reservoir **216** may be fluidly coupled to the catheter hub **210** via a delivery line **214** coupled thereto and extending therebetween. It should further be understood that in other embodiments the vial assembly **250** may include various other needle connection ports other than the septum **255** shown and described above.

Although not shown, it should be understood that in some embodiments the housing **202** may include an interface surface that has one or more displays (e.g., dosimeter display, sensor output display, viewing window, etc.) to provide an operator of delivery device **200** with real-time feedback of the contents, quantities, and operability of the delivery device **200.** Additionally or alternatively, the delivery device **200** may be communicatively coupled to one or more remote displays (e.g., smart device, tablet, etc.). In the embodiments, the delivery device **200** may further include one or more sensors operable to measure a rate of delivery of a fluid media from the delivery device **200,** such as, for example, a mixture of a fluid medium contained within the chamber **202B** and a radioactive material stored within the internal chamber **251** of the vial assembly **250** (e.g., radioembolizing beads). By way of example only, the one or more sensors (e.g., a dosimeter, a linear encoder, an optical sensor, a linear displacement sensor, a flow sensor, an ultrasonic sensor, a magnetic encoder, a laser distance sensor, an inductance sensor, a radial encoder, a volumetric sensor, mechanical transducers, etc.) may be configured to measure a velocity, pressure, force, displacement, flow, capacitance, radiation, and/or the like of the fluid media delivered from the delivery device **200.**

A sensor output display may provide real time monitoring of such measurements calculated by the one or more sensors for an operator's observation during a medical procedure. In particular, such sensors may assist an operator in regulating a delivery after reviewing the measurement outputs from, for example, a display of a device. By way of example, a sensor output display may comprise an LCD screen, a mechanical output, smart device, remote tablet, or other various display outputs positioned along the housing **202** of the delivery device **200** and/or in wireless communication with the delivery device **200.**

As briefly described above, the delivery device may include one or more sensors for monitoring radiation levels of the contents of the delivery device **200.** By way of example only, such sensors may be highly sensitive radiation sensors (e.g., microcircuit, Geiger counter, etc.) that are configured to detect radiation and measure a total ionizing dose (TID) of radiation. Such sensors may be positioned at various locations within the delivery device **200,** and in particular along a travel path of the radioactive materials stored within the delivery device **200** to determine a percent of radioactivity of said materials. A sensor output display may provide real time monitoring of these measurements and comprise various devices, such as, for example, an LCD screen, a mechanical output, smart device, remote tablet, or other various display outputs. It should be understood that in other embodiments the data and information described above may be transmitted (e.g., wirelessly or wired) to a remote device such that a display of the remote device provides said outputs to an operator thereon.

In some embodiments, a viewing window may be positioned along the housing **202,** and in particular the chamber **202A** where the vial assembly **250** is received therein to provide a visual access to the vial assembly **250.** It should be understood that a viewing window may be formed of a radiation shielding material, similar to the protective shield **253** of the vial assembly **250,** such that any radioactive material contained within delivery device **200** is sealed therein, thereby minimizing exposure of the radioactive material through a viewing window. By way of example only, the radiation shielding material of a viewing window may be formed of a plastic, such as Acrylonitrile Butadiene Styrene (ABS), a lead glass, or other suitable materials for preventing exposure to radioactive material. Alternatively, a viewing window may comprise a video monitor that is operable to display a visualization within the chamber **202A.**

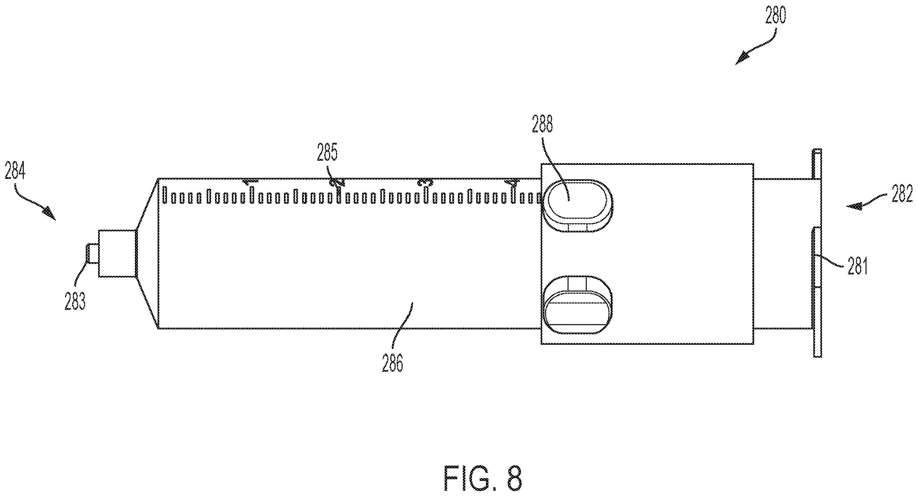

Referring now to FIG. 8, the chamber **202A** of the housing **202** may further include a purging syringe **280** stored therein for purposes of flushing the delivery lines **214** and the fluid reservoir **216** of the delivery device **200** prior to loading the vial assembly **250** for use with the delivery device **200.** It should be understood that in some embodiments the purging syringe **280** may be preassembled within the internal cavity **220A** of the chamber **202A.** The purging syringe **280** includes a proximal end **282** and a distal end **284** with an elongated body **286** extending therebetween. The elongated body **286** of the purging syringe **280** is sized and shaped to be received within the chamber **202A** of the housing **202,** and in particular, to form a press-fit against the internal cavity **220A** of the chamber **202A.** The elongated body **286** may further include a plurality of indicia and/or markings **285** thereon for purposes of measuring and/or identifying a volume of fluid medium stored therein.

The proximal end **282** includes a collar **281** that is sized and shaped to securely fasten the purging syringe **280** to the chamber **202A.** Additionally, the purging syringe **280** includes at least one depressible button **288** extending laterally outward from the elongated body **286,** where the depressible button **288** is sized and shaped to be received within the aperture **209** of the housing **202.** It should be understood that the depressible button **288** of the purging syringe **280** is configured and operable similar to the depressible button **258** of the vial assembly **250** described above. The distal end **284** of the purging syringe **280** includes a port **283** that is sized and shaped to receive the needle **222** therethrough, thereby establishing a fluid communication between the purging syringe **280** and the fluid reservoir **216** of the delivery device **200** via the needle **222** and the delivery line **214** positioned therebetween.

An exemplary mode of operation of the delivery device **200** is described below. The depiction and accompanying description below is not meant to limit the subject matter described herein or represent an exact description of how a fluid media may be delivered using the delivery device **200,** but instead is meant to provide a simple schematic overview to illustrate a general administration of a radioactive media from the delivery device **200** described herein.

Referring to FIGS. 7-8, the delivery lines **214** and the fluid reservoir **216** are initially purged of air using the purging syringe **280.** In particular, an external syringe containing a fluid medium (e.g., saline) is coupled to the chamber **202B** via a connection at the port **207.** The internal cavity **220B** of the chamber **202B** is filled with the fluid medium from the syringe to a desired volume and, once the chamber **202B** is filled, the syringe is decoupled from the port **207.** In this instance, the floating septum **221** is translated within the internal cavity **220B** as the chamber **202B** is filled with the fluid medium via the port **207,** thereby causing the septum **221** to translate along the internal tubing line **223** coupled to and extending between the ports **207, 211.** In particular, the floating septum **221** is translated distally from the port **207** and proximate to the port **211** as the chamber **202B** is filled with the fluid medium. The delivery device **200** is oriented vertically and the purging syringe **280** is pulled back to thereby draw in an amount of fluid medium from the internal cavity **220B** of the chamber **202B** via the delivery lines **214.** In this instance, the purging syringe **280** is pushed forward toward the catheter hub **210** to prime the delivery lines **214** and the fluid reservoir **216** with saline. A delivery line may be coupled to the catheter hub **210** of the delivery device **200,** with an opposing end of the delivery line positioned within a collection bowl to receive the flushed medium therein. These steps may be repeated, as necessary, to remove the air from the delivery lines **214** and the fluid reservoir **216** and effectively prime the delivery device **200** for use during a procedure.

With the delivery lines **214** of the delivery device **200** purged of air, the purging syringe **280** is removed from the internal cavity **220A** of the chamber **202A** via the opening **205A** and the vial assembly **250** is inserted therethrough. In particular, the purging syringe **280** is removed in response to depressing the depressible button **288** at the aperture **209** and extracting the elongated body **286** by pulling the collar **281** at the proximal end **282** proximally from the opening **205A.** Additionally, the vial assembly **250** is received through the opening **205A** and inserted into the housing **202** in response to depressing the depressible button **258** and slidably translating the elongated body **256** into the internal cavity **220A.**

Referring back to FIG. 6, the vial assembly **250** is advanced through the chamber **202A,** with the chamber **202A** continuously applying a predetermined force against the depressible button **258** to thereby maintain the depressible button **258** in a contracted state, until the depressible button **258** is aligned with the aperture **209** of the housing **202.** In this instance, a resilient bias of the depressible button **258** extends the depressible button **258** outward from the elongated body **256** due to a termination of the predetermined force thereon. Simultaneous with the receipt of the depressible button **258** in the aperture **209,** the septum **255** of the vial assembly **250** contacts the needle **222** within the internal cavity **220A** of the chamber **202A.** Accordingly, the septum **255** is punctured and the needle **222** is in fluid communication with the internal chamber **251** of the vial assembly **250.**

In other words, advancing the vial assembly **250** distally into the chamber **202A** provides a series of feedbacks (e.g., visual, audible, tactile, and/or mechanical) to confirm a coupling of the vial assembly **250** with the delivery device **200.** In particular, a receipt of the depressible button **258** in the aperture **209** may provide a visual, audible, tactile and mechanical feedback to an operator that the vial assembly **250** is coupled to the delivery device **200.** Additionally, a puncture of the septum **255** by the needle **222** may provide an audible, tactile and mechanical feedback to an operator that the vial assembly **250** is in fluid communication with the delivery device **200.** In this instance, with the internal chamber **251** now in fluidic communication with the delivery device **200,** any advancement of the handle **252** provides for the delivery of the radioembolizing beads stored within the internal chamber **251** of the vial assembly **250.**

Referring to FIG. 7, the catheter hub **210** of the delivery device **200** may be coupled to a catheter (e.g., microcatheter) via a delivery line extending therebetween. It should be understood that in other embodiments the catheter hub **210** may be coupled to a catheter prior to assembling the vial assembly **250** into the chamber **202A** of the delivery device **200.** With the vial assembly **250** storing radioembolizing beads within the internal chamber **251** with the protective shield **253** disposed thereover, an operator is not required to manipulate any vials containing radioactive material during the medical procedure. Rather, once the vial assembly **250** is assembled into the housing **202** of the delivery device **200** an operator is not required to directly handle the radioembolizing beads any further, thereby reducing the risk of radiological or biological contamination by human error during the procedure.

With the internal cavity **220B** of the chamber **202B** loaded with a fluid media and the vial assembly **250** fully assembled into the delivery device **200,** an operator may selectively actuate the delivery device **200** to deliver a controlled mixture of the therapeutic particles (e.g., radioembolizing beads) from the vial assembly **250** and fluid media from the chamber **202B** during a procedure. As briefly noted above, the delivery device **200** may be communicatively coupled to a remote device, such as, for example, a tablet, a computer, a mobile device, and/or the like. The remote device may receive and display delivery information along an interface display of the remote device for an operator of the delivery device **200** to monitor as the delivery device **200** is in use during a procedure. For example, the delivery information displayed along the remote device may include, but is not limited to, a rate of flow (ml/min), a current volume of media in the chambers **202A, 202B,** an infused volume of media from the chambers **202A, 202B,** a remaining percentage of radioactive activity stored within the delivery device **200,** and/or the like.

Referring back to FIG. 5, to administer a dose of radioactive material with the delivery device **200,** the handle **252** of the vial assembly **250** is actuated to translate the plunger **254** proximally away from the elongated body **256.** In this instance, a negative pressure is generated between the internal chamber **251** of the vial assembly **250** and the internal cavity **220B** of the chamber **202B,** which are in communication with one another through the air-purged delivery lines **214.** Accordingly, pulling the plunger **254** proximally extracts a fluid media stored within the internal cavity **220B** (e.g., saline) through the delivery lines **214** and into the internal chamber **251** of the vial assembly **250** via the needle **222.** In particular, the floating septum **221** is translated proximately toward the port **207** and distally away from the port **211** as the chamber **202B** is emptied of the fluid medium. This suction of fluid media into the internal chamber **251,** where the therapeutic particles are stored, causes a mixture of the two substances therein to form a suspension fluid.

Once the handle **252** and the plunger **254** are pulled proximally to a fullest extent, the handle **252** may be actuated to translate the plunger **254** distally toward the elongated body **256** to generate a positive pressure. The handle **252** may be actuated by either rotating the handle **252** to deliver a slow, controlled dose of the radioactive mixture or by translating the handle **252** to deliver a fast, controlled dose. In some embodiments, depression of the depressible button **258** toward the elongated body **256** may be required to translate the handle **252** and the plunger **254** to deliver a fast, controlled dose of the mixture. In this instance, the depressible button **258** may serve as a secondary safety mechanism for the delivery device **200** when administering a fast dose of the mixture.

Referring back to FIG. 7, a dose of the mixture formed in the internal chamber **251** of the vial assembly **250** is effectively transferred through the needle **222** and into the fluid reservoir **216,** where the dose is thereby delivered out from the delivery device **200** through the catheter hub **210.** With the delivery device **200** coupled to a catheter via the catheter hub **210,** the mixture may be delivered to a patient intravenously by positioning the catheter at a target treatment site within a patient. Additional doses may be delivered by the delivery device **200** by repeating the actuation of the handle **252** described above to refill and purge the mixture of fluid mediums until either a sufficient dose has been administered to a patient (e.g., a sensor output reading from a dosimeter sensor drops to a predetermined level), the internal chamber **251** is depleted, and/or or stasis is achieved. Sensor output displays may provide an operator with real-time informational feedback of the force, pressure, and/or flow of the mixture delivered from the delivery device **200** to the catheter via one or more sensors contained within the delivery device **200.** By monitoring sensor output display an operator is able to regulate the delivery of the radioembolizing beads to the patient and cease delivery when desired.

In instances where a fluid media stored within the internal cavity **220B** of the chamber **202B** is depleted prior to a completion of the procedure, additional fluid media (e.g., saline) may be refilled into the chamber **202B** during the procedure via the port **207.** At a conclusion of the procedure, the delivery device **200** may be discarded. In some embodiments the delivery device **200** may include a transducer therein such that an operator may be capable of actuating the delivery device **200** from a remote location such that an operator is located distally from the radioactive material contained within the delivery device **200.**

Although not shown, it should be understood that the delivery device **200** may further include a device stand that is sized and shaped to removably receive the delivery device **200** thereon. The delivery device may be configured and operable to temporarily maintain the delivery device **200** during a medical procedure. Accordingly, the device stand may facilitate and preserve a sterilization of the delivery device **200** prior to, during, and after use of the delivery device **200** for a procedure.

### III. Semi-Automatic Handheld Delivery Device

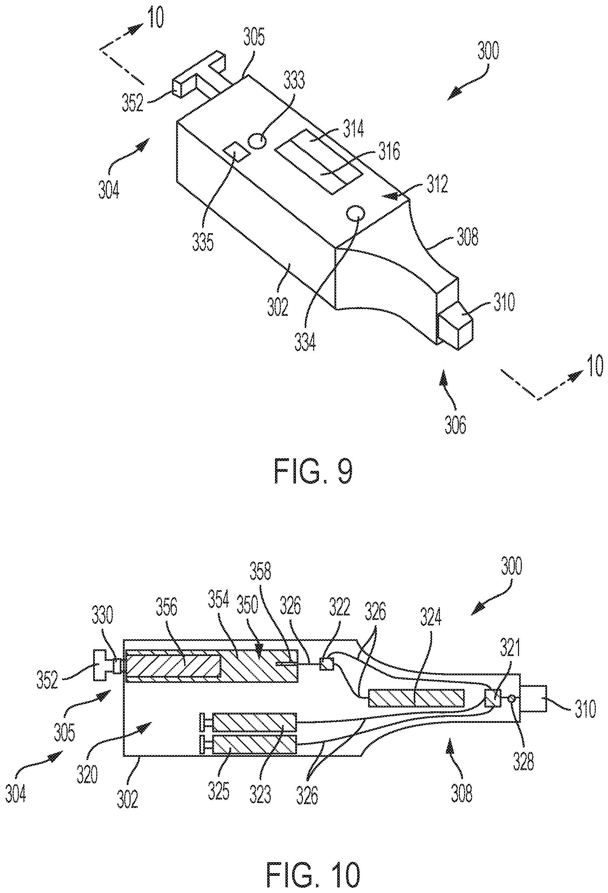

FIGS. 9-10 show another embodiment of a delivery device **300** that is configured and operable to deliver a radioactive material (e.g., radioembolizing beads) while reducing radioactive emissions during use of the delivery device **300.** It should be understood that the delivery device **300** of the present example may be configured and operable similar to the delivery device **200** described above, as the delivery device **300** is substantially similar to the delivery device **200** except for the differences explicitly noted herein.

Referring specifically to FIG. 9, the delivery device **300** includes a housing **302** extending between a proximal end **304** and a distal end **306,** with the distal end **306** of the housing **302** including an elongated housing **308** extending distally therefrom. The elongated housing **308** of the delivery device **300** includes a distal tip **310** that comprises a catheter hub for coupling the delivery device **300** to an external device, such as, for example, a catheter. The housing **302** of the delivery device **300** defines an internal cavity **320** disposed therein *(See* FIG. 10). As will be described in greater detail herein, the internal cavity **320** defined by the housing **302** stores one or more devices (e.g., syringes, fluid reservoirs, valves, and the like) within the delivery device **300.** The housing **302** further includes an interface surface **312** positioned between the proximal end **304** and the distal end **306** of the delivery device **300,** with the interface surface **312** including one or more switches for actuating the one or more devices stored within and coupled to the delivery device **300.** The interface surface **312** further includes one or more displays to providing feedback (e.g., visual) of an output and/or operability of the one or more devices stored within the delivery device **300.**

In the present example, the interface surface **312** of the delivery device **300** includes at least a dosimeter display **314,** a sensor output display **316,** a contrast switch **333,** a flush switch **334,** and a saline switch **335.** It should be understood that a position of the displays 314, 316 and switches **333, 334, 335** shown and described herein are merely for illustrative purposes only such that a location of the displays 314, 316 and the switches **333, 334, 335** may vary without departing from the scope of the present disclosure. As will be described in greater detail below, each switch **333, 335** is communicatively coupled to and configured to actuate a respective device (e.g., a contrast syringe **323,** a saline syringe **325,** respectively) contained within the internal cavity **320** of the housing **302.** Accordingly, manipulating the switches **333, 335** along the interface surface **312** of the housing **302** may provide for an automatic delivery of a fluid medium contained within the syringes **323, 325,** respectively.

Referring now to FIG. 10, the internal cavity **320** of the housing **302** includes at least a pair of connector valves **321, 322,** a contrast syringe **323,** a fluid reservoir **324,** a saline syringe **325,** and a syringe **350.** The various devices disposed within the internal cavity **320** of the housing **302** are fluidly coupled to one another via a series of delivery lines **326** disposed within the internal cavity **320** of the housing **302** and extending therebetween. In particular, the syringe **350** is fluidly coupled to the first connector valve **322** via a delivery line **326** extending therebetween. The syringe **350** includes an external chamber **354,** an internal chamber **356** disposed within the external chamber **354,** and an internal needle **358** disposed within the external chamber **354.** The internal chamber **356** is sized and shaped to be received within the external chamber **354.** In other words, the syringe **350** is a dual-chamber syringe that is capable of storing multiple fluid mediums therein, such that a fluid medium stored within each of the respective chambers **354, 356** are separated from one another. In the present example, a fluid medium stored in the external chamber **354** of the syringe **350** comprises a saline media and a fluid medium stored in the internal chamber **356** of the syringe comprises a radioactive media, such as, for example, radioembolizing beads. It should be understood that in other embodiments the pair of connector valves **321, 322** may be comprise various other devices, such as, for example, a manifold.

The syringe **350** further includes a handle **352** coupled to the internal chamber **356** such that the internal chamber **356** is movable within the internal cavity **320,** and in particular within the external chamber **354,** in response to an actuation (e.g., linear translation) of the handle **352** relative to the housing **302** of the delivery device **300.** It should be understood that the external chamber **354** of the syringe **350** is fixedly secured within the internal cavity **320** of the housing **302** such that the external chamber **354** is immovable in response to an actuation of the handle **352.** The handle **352** extends proximally outward from the housing **302** at the proximal end **304** such that the handle **352** of the syringe **350** is accessible by an operator of the delivery device **300** despite the syringe **350** being disposed within the internal cavity **320** of the housing **302.** The handle **352** of the syringe **350** extends distally from the internal cavity **320** via a syringe opening **305** located at the proximal end **304** of the housing **302.**

It should be understood that with the internal needle **358** of the syringe **350** is fixedly secured within the external chamber **354** along an end opposite of the internal chamber **356** of the syringe **350.** With the internal chamber **356** movably coupled to the handle **352** within the external chamber **354** and the internal needle **358** fixedly disposed within the external chamber **354,** a translation of the handle **352** may provide an interaction of the internal chamber **356** and the internal needle **358.** More specifically, and as will be described in greater detail herein, an actuation of the handle **352** (e.g., translating the handle **352** distally toward the distal end **306** of the delivery device **300**) generates a positive pressure in the external chamber **354** of the syringe **350** as the internal chamber **356** moves within the external chamber **354.**

Still referring to FIG. 10, upon an initial actuation of the handle **352,** a fluid media stored within the external chamber **354** (e.g., saline) is effectively transmitted from the external chamber **354** to the first connector valve **322** via the delivery line **326** coupled to and disposed therebetween. In this instance, a continued translation of the handle **352** in toward the distal end **306** of the delivery device **300** causes the internal chamber **356** to encounter the internal needle **358** within the external chamber **354.** In this instance, the handle **352** is operable to establish fluid communication between the internal chamber **356** and the external chamber **354** in response to the internal needle **358** puncturing the internal chamber **356.** Accordingly, a fluid media stored within the internal chamber **356** (e.g., radioembolizing beads) is thereby transferrable to the external chamber **354,** and with the external chamber **354** depleted of a fluid medium in response to the initial actuation of the handle **352,** the radioembolizing beads stored in the internal chamber **356** may be effectively delivered to the first connector valve **322** via the delivery line **326** coupled therebetween.

The first connector valve **322** disposed within the internal cavity **320** of the housing 302 is similar to the fluid reservoir **216** of the delivery device **200** described above, such that the first connector valve **322** may comprise a Y-manifold, a three-way check valve assembly, and/or the like. The first connector valve **322** provides fluidic communication between the syringe **350** and the fluid reservoir **324** via the series of delivery line **326.** Further, the first connector valve **322** is in fluidic communication with a second connector valve **321,** which is positioned adjacent to the distal end **306** of the housing **302** an disposed within the elongated housing **308.** The contrast syringe **323** and the saline syringe **325** are fluidly coupled to the second connector valve **321.**

Still referring to FIG. 10, the contrast syringe **323** is configured to store a fluid medium therein, and in the present example the contrast syringe **323** includes a contrast medium stored therein. The saline syringe **325** is similarly configured to store a fluid medium therein, and in the present example the saline syringe **325** includes a saline medium stored therein. It should be understood that syringes **323, 325** may include various other suitable fluid media, and in some instances may include identical substances stored therein. Although not shown, it should also be understood that additional or fewer syringes **323, 325, 350** may be included within the internal cavity **320** of the delivery device **300.** Further, although the syringes **323, 325** are shown as having a size and shape that are different than the syringe **350,** it should be understood that the syringes **323, 325, 350** may comprise various suitable shapes and sizes that may be stored within the internal cavity **320** of the housing **302** without departing from the scope of the present disclosure. Additionally, it should further be understood that a position of the syringes **323, 325, 350** shown and described herein are merely for illustrative purposes such that a location of the syringes **323, 325, 350** may vary without departing from the scope of the present disclosure.

The contrast syringe **323** is in fluidic communication with the second connector valve **321** via the delivery line **326** and the saline syringe **325** is in fluidic communication with the second connector valve **321** via a separate delivery line **326.** In this instance, the fluid media contained within the syringes **323, 325, 350,** respectively, are separated and isolated from one another within the internal cavity **320** of the housing **302** until arriving at the second connector valve **321.** In other words, the second connector valve **321** serves as an integration site for the fluid media contained within the syringes **323, 325, 350.** It should be understood that in some embodiments the syringes **323, 325, 350** may be removably received within the internal cavity **320,** and in particular, may not be preassembled within the delivery device **300.** Accordingly, an operator is able to determine which syringes **323, 325, 350** to load into the delivery device **300** based on a particular medical procedure to be performed with the delivery device **300.** Alternatively, in some instances the syringes **323, 325, 350** may be preloaded into the delivery device **300** such that an operator is not required to insert one or more of the syringes **323, 325, 350** into the internal cavity **320** during a medical procedure.

Still referring to FIG. 10, in the present example the switches **333, 334, 335** are configured to be electrically actuated to thereby actuate the syringes **323, 325, 350,** respectively. In other versions, one or more of the switches may be configured to be hydraulically, mechanically, and/or pneumatically actuated to actuate the syringes **323, 325, 350.** In particular, the contrast switch **333** is configured and operable to actuate the contrast syringe **323,** such that actuation of the contrast switch **333** administers a transmission of a fluid medium stored within the contrast syringe **323** (e.g., contrast medium) through the series of delivery lines **326** and into the second connector valve **321.** The saline switch **335** is configured and operable to actuate the saline syringe **325,** such that actuation of the saline switch **335** administers a transmission of a fluid medium stored within the saline syringe **325** (e.g., saline medium) through the series of delivery lines **326** and into the second connector valve **321.** The flush switch **334** serves as a safety lock and is configured to permit delivery of a first fluid medium (e.g., saline) from the external chamber **354** of the syringe **350.** Accordingly, actuation of the handle **352** of the syringe **350** is inoperable to deliver a first fluid medium stored within the external chamber **354** until the flush switch **334** is actuated.

Referring back to FIG. 9, it should be understood that an actuation of the switches **333, 334, 335** may comprise interacting with the interface surface **312** along the housing **302** of the delivery device **300** by contacting the switches **333, 334, 335** (i.e., one click actuation), by continuously engaging the switches **333, 334, 335,** and the like. It should further be understood that in other versions the switches **333, 334, 335** may be remotely located relative to the housing **302** such that delivery device **300** may be actuated wirelessly via a remote device. In either instance, actuation of the switches **333, 335** of the delivery device **300** provides an automatic transmission of the respective fluid media contained therein. It should be understood that a pressure, flow, and/or fill rate employed by the syringes **323, 325** in response to an actuation of the switches **333, 335** may be preprogrammed such that actuation of the switches **333, 335** provides autonomous transmission of the fluid medium at the predetermined rate.

For example, a desired pressure, flow, and/or fill rate of the delivery device **300** may be selectively inputted at the interface surface **312** and/or via a remote device communicatively coupled to the delivery device **300** prior to commencing a procedure with the delivery device **300.** However, it should be understood that a delivery of a fluid media stored within the syringe **350,** and in particular an internal chamber **356** of the syringe **350** (e.g., radioembolizing beads) remains fully manual via the handle **352.** Accordingly, an effective flow and pressure rate for delivering the one or more mediums of the syringe **350** stored within the chambers **354, 356** is mechanically determined based on an application of force onto the handle **352.**

Referring back to FIG. 10, the delivery device **300** may include one or more sensors disposed within the internal cavity **320** of the housing **302.** As described in greater detail above with respect to the delivery device **200,** the one or more sensors (e.g., a dosimeter, a linear encoder, an optical sensor, a linear displacement sensor, a flow sensor, an ultrasonic sensor, a magnetic encoder, a laser distance sensor, an inductance sensor, a radial encoder, a volumetric sensor, mechanical transducers, etc.) may be configured and operable to measure a flow, capacitance, radiation, volume, and/or the like of the various fluid mediums stored within and administered by the delivery device **300.** In the present example, the delivery device **300** includes a dosimeter sensor **328** and a displacement sensor **330.** In particular, the dosimeter sensor **328** is disposed within the housing **302,** and in particular within the elongated housing **308** of the delivery device **300.** The dosimeter sensor **328** is fluidly coupled to the second connector valve **321** via the delivery line **326** extending therebetween and is operable to measure a radiation level of the fluid media administered through the dosimeter sensor **328** from the second connector valve **321** to the catheter hub at the distal tip **310** of the delivery device **300.** The dosimeter sensor **328** is communicatively coupled to the dosimeter display **314** positioned along the interface surface **312** such that an operator of the delivery device **300** may monitor data detected by the dosimeter sensor **328** thereon.

The displacement sensor **330** is positioned along the handle **352** of the syringe **350** such that the displacement sensor **330** is positioned external from the internal cavity **320** of the housing **302.** The displacement sensor **330** is operable to measure a linear displacement of the handle **352** relative to the housing **302** to determine a force, pressure, and/or flow of the fluid medium administered from the syringe **350** to the catheter hub at the distal tip **310.** The displacement sensor **330** is communicatively coupled to the sensor output display **316** positioned along the interface surface **312** such that an operator of the delivery device **300** may monitor data detected by the displacement sensor **330** thereon. It should be understood that additional and/or fewer sensors, displays, switches, and/or syringes, may be provided in the delivery device **300** without departing from the scope of the present disclosure.

Still referring to FIG. 10, in an exemplary mode of operation of the delivery device **300,** an operator may use the delivery device **300** in a manner substantially similar to that of the delivery device **200** described above. For instance, with syringes **323, 325, 350** assembled in the internal cavity **320** of the housing **302,** the delivery device **300** is coupled to an external catheter via the catheter hub positioned at the distal tip **310.** In this instance, the flush switch **334** is actuated by depressing the flush switch **334** downward, thereby unlocking a movement of the handle **352** of the syringe **350.** Accordingly, an operator may apply a predetermined force onto the handle **352,** and more particularly push the handle **352** distally toward the housing **302** to commence a flush of the syringe **350,** the connector valves **321, 322,** and the catheter hub at the distal tip **310** via the series of delivery lines **326** disposed therebetween. The delivery device **300** is flushed with the fluid medium stored within the external chamber **354** of the syringe **350** in response to actuating the handle **352,** where the fluid medium stored within the external chamber **354** may comprise, for example, a saline medium. Accordingly, the saline is transferred through the catheter hub at the distal tip **310** and into an external catheter coupled to the delivery device **300** thereto, thereby purging the corresponding catheter system of any air contained therein.

Continuing a distal translation of the handle **352** distally into the housing **302,** while the flush switch **334** remains continuously depressed along the interface surface **312,** provides a first feedback to an operator of the delivery device **300.** By way of example only, the delivery device **300** may be configured to generate a feedback (e.g., visual, audio, tactile, mechanical, etc.) in response to a depletion of a fluid medium (e.g., saline) stored within the external chamber **354** of the syringe **350.** Upon a depletion of the external chamber **354,** an operator actuates one or more of the switches **333, 335** to transmit a fluid medium stored therein, respectively. Continued translation of the handle **352** distally into the housing **302** of the delivery device **300** generates a second feedback in response to the internal needle **358** puncturing the internal chamber **356** of the syringe **350.** In this instance, fluidic communication between the internal chamber **356** and the external chamber **354** is formed such that a fluid media stored within the internal chamber **356** (e.g., radioembolizing beads) may be effectively transferred therefrom.

Still referring to FIG. 10, in this instance the handle **352** is manually retracted in a proximal direction relative to the housing **302,** which requires continued actuation of the flush switch **334** along the interface surface **312.** A negative pressure is generated within the syringe **350** in response such that the negative pressure causes the fluid media stored within the fluid reservoir **324** to transfer through the first connector valve **322** and into the internal chamber **356** via the external chamber **354.** Accordingly, a fluid medium stored within the fluid reservoir **324** is intermixed with the fluid media contained within the internal chamber **356.** Subsequent distal translation of the handle **352** toward the housing **302** transfers the mixture of fluid mediums from the syringe **350,** through the first connector valve **322,** and into the second connector valve **321.** In this instance, an operator may further actuate either switch **333, 335** to thereby transfer a contrast medium and/or saline medium from the contrast syringe **323** and/or saline syringe **325,** respectively, to the second connector valve **321.**

Accordingly, a further mixture of mediums is formed at the second connector valve **321** from the one or more fluid media contained within the syringes **323, 325, 350.** Thus, prior to the mixture of fluid media being delivered through a catheter hub at the distal tip **310** of the delivery device **300** and into an external catheter coupled thereto, the delivery device **300** is operable to mix multiple fluid mediums therein for delivery to a patient. The sensor output display **316** along the interface surface **312** provides real time informational feedback of the force, pressure, and/or flow of the mixture delivered from delivery device **300** to the catheter via the displacement sensor **330.** The displacement sensor **330** allows an operator to regulate a delivery of the radioembolizing beads to the patient and cease delivery when desired. An operator may continue delivering the radioembolizing beads from the delivery device **300** until the dosimeter display **314** indicates that a radiation exposure level measured by the dosimeter sensor **328** has dropped to an acceptable level (e.g., approximately zero radioactive material remaining in delivery device **300**).

### IV. Automatic Handheld Delivery Device

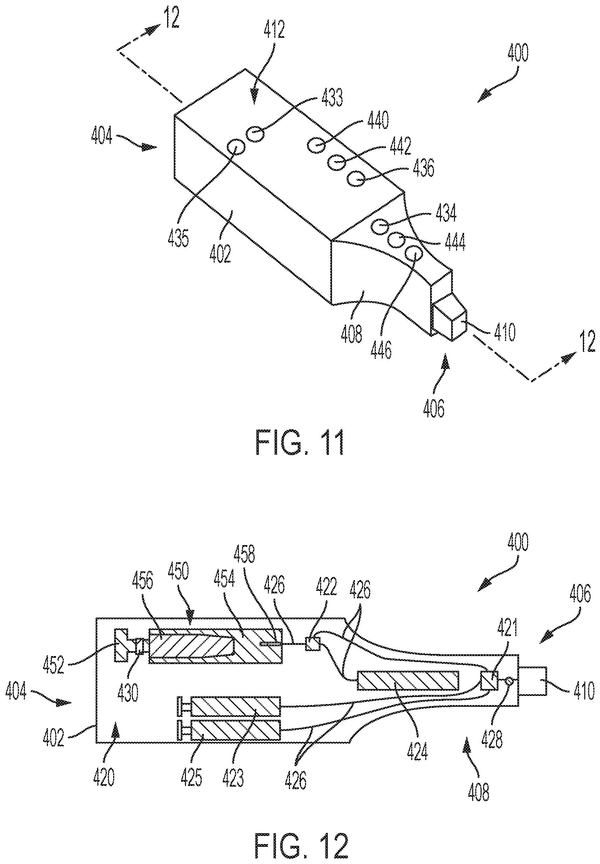

FIGS. 11-12 shows another embodiment of a delivery device **400** that is configured and operable to deliver a radioactive material (e.g., radioembolizing beads) while reducing radioactive emissions during use of the delivery device **400.** It should be understood that the delivery device **400** of the present example may be configured and operable similar to the delivery device **200, 300** described above as the delivery device **400** is substantially similar to the delivery device **200, 300** except for the differences explicitly noted herein.

Referring specifically to FIG. 11, the delivery device **400** includes a housing **402** extending between a proximal end **404** and a distal end **406,** with the distal end **406** of the housing **402** including an elongated housing **408** extending distally therefrom. The elongated housing **408** of the delivery device **400** includes a catheter hub **410** for coupling the delivery device **400** to an external device, such as, for example, a catheter. The housing **402** of the delivery device **400** defines an internal cavity **420** disposed therein *(See* FIG. 12). Similar to the delivery device **300** described above, the internal cavity **420** defined by the housing **402** of the delivery device **400** stores one or more devices (e.g., syringes, fluid reservoirs, valves, manifolds, and the like) within the delivery device **400,** such as the pair of connector valves **421, 422,** the contrast syringe **423,** the manifold and/or fluid reservoir **424,** the saline syringe **425,** and the syringe **450.** It should be understood that the connector valves **421, 422,** the syringes **423, 425, 450** and the fluid reservoir **424** of the delivery device **400** are configured and operable substantially similar to those described above with respect to the delivery device **300.** In some embodiments, the pair of connector valves **421, 422** may comprise various other devices, such as, for example, a manifold.

Still referring to FIG. 11, the housing **402** further includes an interface surface **412** positioned between the proximal end **404** and the distal end **406** of the delivery device **400,** with the interface surface **412** including one or more switches for actuating the one or more devices stored within and coupled to the delivery device **400.** The interface surface **412** further includes one or more displays for providing feedback (e.g., visual) of an output and/or operability of the one or more devices stored within the delivery device **400.** The delivery device **400** includes a contrast switch **433,** a flush switch **434,** and a saline switch **435** positioned along the interface surface **412** that are substantially similar to the switches **333, 334, 335** of the delivery device **300** described above. Unlike the delivery device **300,** however, the delivery device **400** does not include a dosimeter display or sensor output display along the interface surface **412.**

Rather, the delivery device **400** includes a first engagement switch **440** and a first dispense switch **442** positioned along the interface surface **412.** Further, the elongated housing **408** includes a second engagement switch **444** and a second dispense switch **446** positioned proximate to the switches **440, 442.** Although not shown, it should be understood that the switches **444, 446** may alternatively be located along the interface surface **412.** It should further be understood that a location of the switches along the interface surface **412** of the delivery device **400** are merely for illustrative purposes such that the switches may be positioned along various other surfaces of the delivery device **400** without departing from the scope of the present disclosure.

Still referring to FIG. 11, the delivery device **400** is configured to deliver a fluid medium stored within the respective syringes **423, 425, 450** in response to an actuation (e.g., depression) of a corresponding switch **433, 434, 435.** In other words, as will be described in greater detail below, dissimilar to the flush switch **334** of the delivery device **300** described above, actuation of the flush switch **434** of the delivery device **400** provides for an automated translation of the handle **452** of the syringe **450.** In the present example, the switches **433, 434, 435** are configured to be electrically actuated to flush the syringes **423, 425, 450,** respectively. In other versions, the switches **433, 434, 435** may be configured to be hydraulically, mechanically, and/or pneumatically actuated to flush the syringes **423, 425, 450.**

Referring to FIG. 12, the handle **452** of the syringe **450** is disposed within the housing **402** of the delivery device **400** such that the delivery device **400** does not include a syringe opening at the proximal end **404** of the housing **402.** Accordingly, actuation of the handle **452** is controlled at least in part by the flush switch **434** along the interface surface **412,** rather than by a manual actuation as the handle **352** of the delivery device **300** requires as described in greater detail above.

In an exemplary mode of operation, the delivery device **400** is employed in a substantially similar manner as the delivery device **300** described above. For instance, with the syringes **423, 425, 450** assembled in the internal cavity **420** of the housing **402,** the delivery device **400** is coupled to a catheter via the catheter hub **410** of the housing **402.** With the catheter positioned within a target treatment site of a patient's body, the flush switch **434** is actuated to automatically translate the handle **452** distally to thereby flush a fluid medium stored within the external chamber **454** of the syringe (e.g., saline) therefrom and into the connector valves **421, 422** and the catheter hub **410,** respectively, via the series of delivery lines **426.** Accordingly, the saline is transferred through the catheter hub **410** and into the catheter coupled thereto thereby purging the catheter system of any air contained therein.

Referring back to FIG. 11, continued actuation of the flush switch **434** provides a continued translation of the handle **452** in a distal direction until a first feedback (e.g., visual, audio, tactile, mechanical, etc.) is generated. The first feedback may be indicative of a depletion of the fluid medium stored within the external chamber **454** (e.g., saline) of the syringe **450.** In this instance, an operator ceases actuating (e.g., pressing) the flush switch **434** and may actuate either the contrast switch **433** and/or the saline switch **435** to thereby transmit a contrast medium or saline medium to the second connector valve **421,** respectively, from either of the syringes **423, 425.**

Actuating the first and second engagement switches **440, 444** concurrently provides for a translation of the internal needle **458** within the external chamber **454** of the syringe **450** and toward the internal chamber **456.** Accordingly, dissimilar to the delivery device **300** described above, the internal needle **458** of the delivery device **400** is movable within the external chamber **454** in response to an actuation of the engagement switches **440, 444.** The internal needle **458** is translated within the external chamber **454** until the internal needle **458** encounters the internal chamber **456** within the external chamber **454.** In this instance, the internal chamber **456** is punctured by the internal needle **458** thereby establishing access to a fluid media stored within the internal chamber **456** (e.g., radioembolizing beads). A second feedback (e.g., visual, audio, tactile, mechanical, etc.) is generated indicating fluidic communication to the internal chamber **456** being established.

Referring back to FIG. 12, manual retraction of the handle **452** in a proximal direction toward the proximal end **404** of the delivery device **400** is provided in response to actuating the fill switch **436.** Proximal retraction of the handle **452** generates a negative pressure within the syringe **450** thereby causing a fluid medium stored within the fluid reservoir **424** to be drawn into the internal chamber **456** via the series of delivery lines **426** and the first connector valve **422** coupled therebetween. With the internal chamber **456** having received a fluid medium of the fluid reservoir **424** therein, a mixture of the mediums is formed within the internal chamber **456.** An operator may cease actuating (e.g., pushing) the fill switch **436** to thereby terminate a proximal translation of the handle **452.**

In this instance, with a mixture of fluid mediums from the fluid reservoir **424** and the internal chamber **456** formed within the internal chamber **456,** actuating both the dispense switches **442, 446** provides for a translation of the handle **452** in a distal direction toward the distal end **406** of the delivery device **400,** thereby generating a positive pressure to deliver the mixture from the syringe **450,** through the first connector valve **422,** and into the second connector valve **421.** In this instance, either of the switches **433, 435** may be actuated to thereby transfer a contrast agent and/or saline from the contrast syringe **423** and/or saline syringe **425,** respectively. Accordingly, an additional mixture may be formed at the second connector valve **421** with the fluid media transferred from the syringes **423, 425, 450** prior to the mixture being delivered through the catheter hub **410** and into a connecting catheter thereon. As described in greater detail above with respect the delivery devices **100, 200, 300,** the delivery device **400** of the present example may include one or more sensors (e.g., a dosimeter, a linear encoder, an optical sensor, a linear displacement sensor, a flow sensor, an ultrasonic sensor, a magnetic encoder, a laser distance sensor, an inductance sensor, a radial encoder, a volumetric sensor, mechanical transducers, etc.) therein for detecting, measuring, and outputting data relating to the therapeutic particles administered by the delivery device **400** to a patient.

### V. Mechanical Delivery Device with Removable Sled Assembly

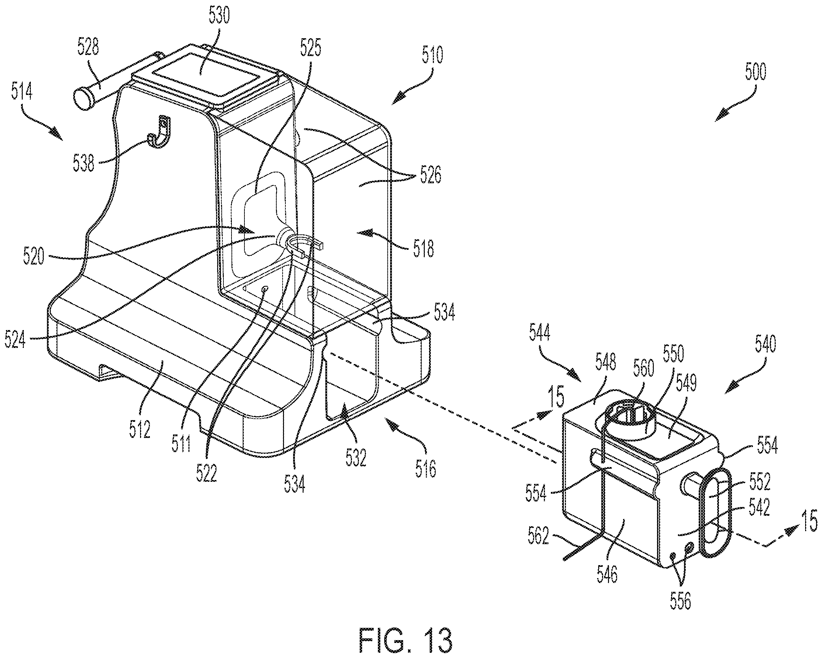

FIGS. 13-29 show another embodiment of a delivery device **500** that is configured and operable to deliver a radioactive material (e.g., radioembolizing beads) while reducing radioactive emissions during use of the delivery device **500.** It should be understood that the delivery device **500** of the present example may be configured and operable similar to the delivery device **100** described above as the delivery device **500** is substantially similar to the delivery device **100** except for the differences explicitly noted herein.

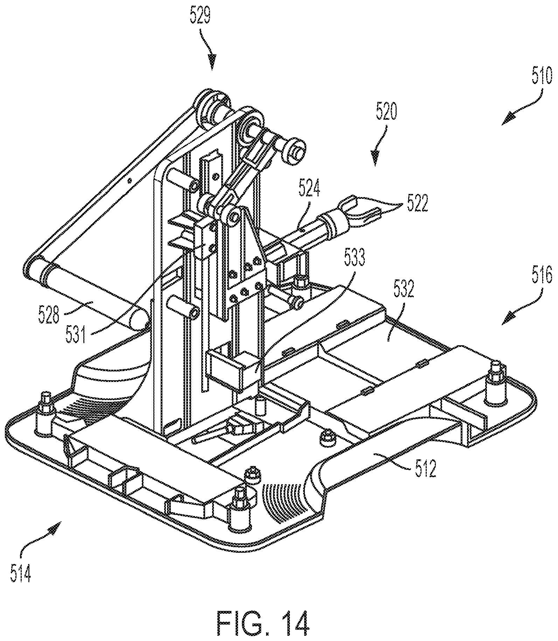

Referring initially to FIG. 13, the delivery device **500** comprises a console assembly **510** and a sled assembly **540** that are operable to transition between a coupled state and decoupled state relative to one another. The console assembly **510** of the delivery device **500** comprises a base **512** defined by and extending between a proximal end **514** and a distal end **516.** The proximal end **514** of the base **512** includes a handle (delivery handle) **528** movably coupled to the console assembly **510** and an interface display **530** positioned on the console assembly **510.** As will be described in greater detail herein, the interface display **530** is operable to transmit information and/or data to an operator of the delivery device **500,** and in particular data detected by an electrical system of the delivery device **500** which may comprise one or more sensors disposed within the delivery device **500** *(See* FIG. 14). It should be understood that the delivery device **500** may include an electrical microprocessor that operates the interface display **530.** In other embodiments, the interface display **530** may comprise a remote smart device, a tablet, and/or the like.

The proximal end **514** of the base **512** further includes an attachment device **538** that is configured to securely retain an external device to the base **512** of the console assembly **510.** The attachment device **538** is operable to facilitate an attachment of a complimentary device to the console assembly **510** for use with the delivery device **500** during a procedure. In the present example, the attachment device **538** is a hook assembly extending outwardly from a side of the base **512** that is sized and shaped to attach a saline bag (i.e., the complimentary device) to the console assembly **510.** In other embodiments, the engagement mechanism may comprise various other forms or configurations for securing a complimentary device to the console assembly **510.**

Still referring to FIG. 13, the distal end **516** of the console assembly **510** defines a vial containment region **518** that is sized and shaped to receive the console assembly **510** therein, as will be described in greater detail herein. The console assembly **510** further includes a vial engagement mechanism **520** extending from the base **512** adjacent to the distal end **516.** In particular, the vial engagement mechanism **520** extends laterally outward from the base **512** of the console assembly **510** toward the distal end **516.** The vial engagement mechanism **520** is positioned within the vial containment region **518** of the console assembly **510** and is movably coupled to the handle **528.** In particular, the handle **528** of the console assembly **510** is operable to move, and in particular translate, the vial engagement mechanism **520** within the vial containment region **518** in response to an actuation of the handle **528.** It should be understood that an ergonomic design of the handle **528** serves to facilitate delivery of a dose from the delivery device **500** through a range of various operator angles relative to the base **512** to thereby enhance a mobility of performing a procedure with the delivery device **500.**

Referring now to FIG. 14, the console assembly **510** includes a mechanical assembly **529** disposed within the base **512** that is configured and operable to convert a manual motion of the handle **528** to a corresponding linear displacement of the vial engagement mechanism **520.** In the present example, the mechanical assembly **529** is coupled to the handle **528** and the vial engagement mechanism **520** such that selective actuation of the handle **528** at the proximal end **514** causes a simultaneous actuation of the vial engagement mechanism **520** at the distal end **516.** As will be described in greater detail herein, the mechanical assembly **529** of the present example allows for fluid volume control and fluid flow volume control during a dose delivery with the delivery device **500.** It should be understood that a mechanical configuration of the mechanical assembly **529** of the present example may comprise various linkages, gears, pullies, springs and/or the like that are specifically configured to amplify a force applied to the handle **528** with a corresponding displacement of the vial engagement mechanism **520.** In some embodiments, the mechanical assembly **529** may comprise and/or be substituted by one or more electrically-driven systems, motors, and/or other devices operable to provide for a movement of the vial engagement mechanism **520** relative to the vial containment region **518** and/or provide a feedback to an operator as the handle **528** is actuated.

In other embodiments the mechanical assembly **529** may be configured such that the handle **528** may be actuated (i.e., moved) in various other arrangements or orientations than that shown and described herein to generate a corresponding linear displacement of the vial engagement mechanism **520.** For example, the mechanical assembly **529** of the console assembly **510** may be configured to convert a linear, rotational, lateral and/or other various motions of the handle **528** to generate a disproportionate displacement of the vial engagement mechanism **520,** with the displacement exceeding a force applied at the handle **528.**

Still referring to FIG. 14, and as briefly described above, the console assembly **510** includes one or more sensors for monitoring and detecting certain conditions and/or materials stored in the console assembly **510** during use of the delivery device **500.** In the present example, the console assembly **510** includes a linear displacement sensor **531** and a radiation sensor **533.** The linear displacement sensor **531** is securely attached to the mechanical assembly **529** of the console assembly **510** such that the linear displacement sensor **531** is operable to move within the console assembly **510** in response to an actuation of the handle **528** and a corresponding movement of the vial engagement mechanism **520.** The linear displacement sensor **531** is configured to detect and monitor a displacement distance, a velocity of displacement, and/or the like of the handle **528** and the vial engagement mechanism **520.**

As will be described in greater detail herein, by measuring a displacement distance or velocity of the handle **528** and/or the vial engagement mechanism **520,** computer readable and executable instructions of the delivery device **500,** when executed by a processor of the delivery device **500,** may determine a flow rate of a fluid media being delivered by the delivery device **500.** Additionally or alternatively, the computer readable and executable instructions of the delivery device **500,** when executed by a processor of the delivery device **500,** may further determine a remaining volume of a fluid media stored within the delivery device **500.** As briefly noted above, the data detected by the linear displacement sensor **531** and the information determined by the processor of the delivery device **500** may be displayed at the interface display **530** for operator review.

Still referring to FIG. 14, the radiation sensor **533** is securely attached to the base **512** of the console assembly **510** at a location adjacent to the vial containment region **518.** In particular, the radiation sensor **533** is positioned proximate to the sled cavity **532** that is sized and shaped to receive the sled assembly **540** therein. As will be described in greater detail herein, the sled assembly **540** is configured to store and administer therapeutic particles (e.g., radioactive beads, microspheres, medium) therethrough such that the radiation sensor **533** is operable to detect and monitor a radiation level of the therapeutic particles due to a proximate location of the radiation sensor **533** with the sled assembly **540.** In particular, the sled assembly **540** is configured to partially receive a vial assembly **580** therein for administering the therapeutic particles from the delivery device **500** and to a patient.

As will further be described herein, by detecting a radiation level of the radioactive medium stored and transferred through the sled assembly **540,** computer readable and executable instructions of the delivery device **500,** when executed by a processor of the delivery device **500,** may determine a radiation dosage delivered from the delivery device **500.** Additionally or alternatively, the computer readable and executable instructions executed by a processor of the delivery device **500** may further determine a remaining radiation dosage contained within the delivery device **500** during a procedure. As briefly noted above, the data detected by the radiation sensor **533** and the information determined by the processor of the delivery device **500** may be displayed at the interface display **530** for operator review. It should be understood that in other embodiments the delivery device **500** may include additional or fewer sensors than those shown and described herein (e.g., a dosimeter, a linear encoder, an optical sensor, a linear displacement sensor, a flow sensor, an ultrasonic sensor, a magnetic encoder, a laser distance sensor, an inductance sensor, a radial encoder, a volumetric sensor, mechanical transducers, etc.). A dosimeter and/or radiation sensor of the delivery device **500** may be configured to measure a remaining exposure to ionizing radiation stored within the delivery device **500,** and in particularly the sled assembly **540** and/or the vial assembly **580.**

As merely illustrative examples only, a linear encoder may be paired with a scale that is configured to encode a position of a remaining dosage of therapeutic particles within the vial assembly **580** such that the linear encoder converts the encoded position into an analog or digital signal that may be decoded into a quantity. An optical sensor/encoder of the delivery device **500** may be configured to convert light rays from within the sled assembly **540** and/or the vial assembly **580** into an electrical signal to measure a physical quantity of light that is thereby translated into a readable form for measuring a remaining radiation dosage contained within the delivery device **500.** A magnetic encoder of the delivery device **500** may be configured and operable similar to the optical encoder to determine a remaining radiation dosage but utilizes magnetic fields in lieu of light. An inductive sensor encoder of the delivery device **500** may be configured to utilize electromagnetic induction to detect and measure a remaining dosage stored within the vial assembly **580** by developing a magnetic field therein in response to a current flowing therethrough. A laser distance sensor of the delivery device **500** may be configured to measure a remaining dosage within the vial assembly **580** through transmitting a laser to measure a distance within the vial body **589** to a top liquid surface of the therapeutic particles remaining therein.

By way of further examples, a flow sensor of the delivery device **500** may be positioned in-line with the tubing set of the delivery device **500,** and in particular the needle **559,** the manifolds **555A, 555B,** and/or one or more of the ports **556,** and may be configured to measure an amount of fluid (e.g., suspension liquid after the therapeutic particles have effectively mixed with the fluid medium) that passes thereby. An ultrasonic sensor of the delivery device **500** may comprise a transmitter, receiver, and/or transceiver configured to measure a distance to an object (e.g., remaining volume of dosage within the vial assembly **580**) based on transmitting ultrasonic signals (i.e. sound waves) therein and measuring an elapsed time before receiving back the bounced sound waves. A radial encoder of the delivery device **500** may comprise an absolute encoder and/or an incremental encoder configured to convert an angular position or motion of the handle **528,** the plunger **584,** the mechanical assembly **529,** and/or other components of the delivery device **500** to analog or digital ouput signals corresponding to a remaining dosage within the vial assembly **580.**

Referring back to FIG. 13, the vial engagement mechanism **520** comprises a pair of lever arms **522** extending outwardly from a neck **524** of the vial engagement mechanism **520,** with the neck **524** extending laterally outward from the base **512** of the console assembly **510.** The neck **524** of the vial engagement mechanism **520** is disposed within a protective cover **525** such that only the pair of lever arms **522** of the vial engagement mechanism **520** extends through the protective cover **525.** The protective cover **525** is operable to shield one or more internal components of the console assembly **510** from an exterior of the console assembly **510,** and in particular from the vial containment region **518.** As will be described in greater detail herein, the vial containment region **518** of the console assembly **510** is sized and configured to receive one or more radioactive materials therein. In some embodiments, the protective cover **525** of the console assembly **510** may be formed of various materials, including, for example, silicon.

The pair of lever arms **522** is simultaneously movable with the neck **524** of the vial engagement mechanism **520** in response to an actuation of the handle **528** of the console assembly **510.** Further, the pair of lever arms **522** are fixed relative to one another such that a spacing formed between the pair of lever arms **522** is relatively fixed. The pair of lever arms **522** of the vial engagement mechanism **520** is configured to securely engage the vial assembly **580** therebetween, and in particular within the spacing formed by the pair of lever arms **522.** Accordingly, the vial engagement mechanism **520** is operable to securely attach the vial assembly **580** to the console assembly **510** at the vial containment region **518.** Although the vial engagement mechanism **520** is shown and described herein as including a pair of lever arms **522,** it should be understood that the vial engagement mechanism **520** may include various other structural configurations suitable for engaging the vial assembly **580.**

Still referring to FIG. 13, the console assembly **510** further includes a safety shield **526** secured to the distal end **516** of the base **512** along the vial containment region **518.** In particular, the safety shield **526** is a protective covering that is sized and shaped to enclose the vial containment region **518** of the console assembly **510** when secured thereon. The safety shield **526** is selectively attachable to the distal end **516** of the base **512** and is formed of a material that is configured to inhibit radioactive emissions from one or more radioactive doses stored within the vial containment region **518.** By way of example only, the safety shield **526** may be formed of acrylonitrile butadiene styrene (ABS), lead, tungsten, tin, pewter, or other suitable materials configured and operable to inhibit radiation emissions. In the present example, the safety shield **526** include a wall thickness of about 0.95 cm. In addition to inhibiting radiation exposure, the safety shield **526** serves as a prevent and contains any spills and/or leaks of radioactive mediums that may occur at the one or more luer connections contained within the vial containment region **518** and between the console assembly **510,** the sled assembly **540,** and the vial assembly **580.** As described in greater detail herein, with the safety shield **526** being selectively attachable to the console assembly **510,** the safety shield **526** may be separately cleaned after a use of the delivery device **500** during a procedure.

In other embodiments, the delivery device **500** may include a splash guard in addition to and/or in lieu of the safety shield **526.** The splash guard may be formed of a non-opaque housing that encloses the vial containment region **518,** similar to the safety shield **526,** and may be selectively opened and closed through various mechanisms. For example, in some embodiments the splash guard may include a sliding window, a hinge coupling to the console assembly **510** such that the splash guard is pivotable, and/or the like. The splash guard may be formed of various polymers, including, but not limited to, polycarbonate. It should be understood that the splash guard may serve to provide a protective shielding against spills and/or leaks during a loading of the sled assembly **540** and/or the vial assembly **580** to the console assembly **510** during a preparation of the delivery device **500** for use in a procedure.

The distal end **516** of the console assembly **510** further includes a sled cavity **532** that is sized and shaped to receive the sled assembly **540** therein. The sled cavity **532** includes a pair of alignment features **534** extending therein, with the alignment features **534** sized and shaped to correspond with complimentary alignment features of the sled assembly **540** (e.g., alignment ribs **554**) to thereby facilitate a coupling of the sled assembly **540** with the base **512** of the console assembly **510** within the sled cavity **532.** In the present example, the pair of alignment features **534** comprise longitudinal recesses extending laterally along the sled cavity **532,** however, it should be understood that the pair of alignment features **534** may take various other forms and configurations than those shown and described herein without departing from the scope of the present disclosure. For example, the alignment features of the console assembly **510** may include one or more magnets that are configured to mate with corresponding magnets of the sled assembly **540.**

Still referring to FIG. 13, the sled assembly **540** is configured to partially receive a vial assembly **580** therein for administering therapeutic particles (e.g., radioactive fluid medium) from the delivery device **500** and to a patient. In particular, the sled assembly **540** comprises a proximal end **542** and a distal end **544** with a pair of sidewalls **546** extending therebetween. The proximal end **542** of the sled assembly **540** includes a handle **552** extending proximally therefrom. The handle **552** is configured to facilitate movement of the sled assembly **540,** and in particular, an insertion of the sled assembly **540** into the sled cavity **532** of the console assembly **510.** The proximal end **542** further includes one or more ports **556** for coupling one or more delivery lines (i.e., tubing) to the sled assembly **540.** With the one or more delivery lines further be coupled to one or more external devices at an end of the line opposite of the ports **556,** the ports **556** effectively serve to fluidly couple the sled assembly **540** to the one or more external devices via the delivery lines connected thereto. The pair of sidewalls **546** of the sled assembly **540** includes at least one alignment rib **554** extending laterally outward therefrom, where the alignment ribs **554** are sized and shaped to correspond with and mate to the pair of alignment features **534** of the console assembly **510.** Accordingly, the pair of alignment ribs **554** are configured to facilitate an alignment and engagement of the sled assembly **540** with the console assembly **510** when the distal end **544** is slidably received within the sled cavity **532** of the base **512.** As will be described in greater detail herein, the pair of alignment features **534** and the pair of alignment ribs **554** are operable to inhibit a vertical deflection and/or movement of the sled assembly **540** during use of the delivery device **500,** and more specifically, during a vertical translation of the vial engagement mechanism **520** and a corresponding vertical retraction of the vial assembly **580** that is received within the sled assembly **540.**

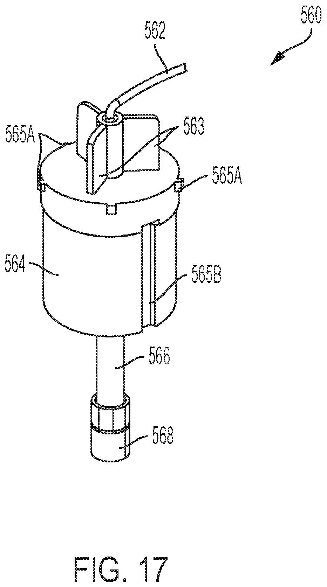

The sled assembly **540** further includes a top surface **548** extending from the proximal end **542** and the distal end **544** and positioned between the pair of sidewalls **546.** The top surface **548** of the sled assembly includes a recessed region **549** and a locking system **550.** The recessed region **549** is sized and shaped to form a recess and/or cavity along the top surface **548,** where the recessed region **549** is capable of receiving and/or collecting various materials therein, including, for example, leaks of various fluid media during use of the delivery device **500.** The locking system **550** of the sled assembly **540** forms an opening along the top surface **548** that is sized and shaped to receive one or more devices therein, such as a priming assembly **560** and a vial assembly **580** (*See* FIG. 17). In some embodiments, the sled assembly **540** comes preloaded with the priming assembly **560** disposed within the locking system **550.** The priming assembly **560** includes a priming line **562** extending outwardly from the locking system **550** of the sled assembly **540.** As will be described in greater detail herein, the priming assembly **560** serves to purge the delivery device **500** of air prior to utilizing the delivery device **500** in a procedure.

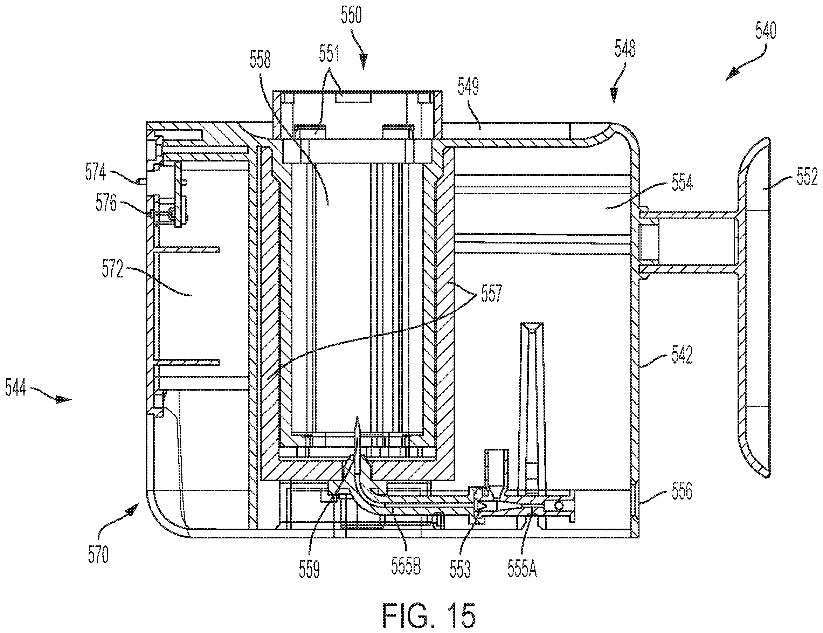

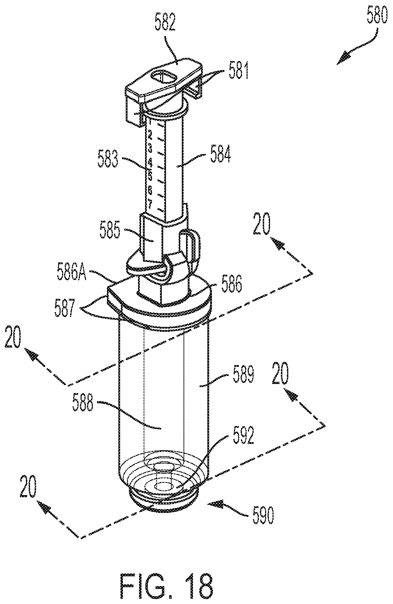

Referring now to FIG. 15, the locking system **550** includes an annular array of projections **551** extending outwardly therefrom, and in particular, extending laterally into the aperture formed by the locking system **550** along the top surface **548.** The annular array of projections **551** are formed within an inner perimeter of the locking system **550** and extend along at least two sequentially-arranged rows. As will be described in greater detail herein, the annular array of projections **551** included in the locking system **550** are configured to engage a corresponding locking feature **586** of the vial assembly **580** (*See* FIG. 18) to thereby securely fasten the vial assembly **580** to the sled assembly **540.** It should be understood that the multiple rows of projections **551** of the locking system **550** serve to provide a double-locking system to ensure the sled assembly **540,** and in particular a needle **559** of the sled assembly **540,** is securely maintained through a septum **592** of the vial assembly **580** (*See* FIG. 18) during use of the delivery device **500** in a procedure. Accordingly, the double-locking system formed by the locking system **550** reduces occurrences of unintended delivery of a dose during preparation of the delivery device **500** for a procedure. It should further be understood that additional and/or fewer projections **551** may be included along the locking system **550** than those shown and described herein without departing from the scope of the present disclosure. Alternatively, in other embodiments the locking system **550** may include various other suitable engagement features, other than the annular array of projections **551** shown and described herein, that are configured and operable to a snap-fit engagement with the vial assembly **580.** For example, in other embodiments the locking system **550** may comprise a threaded portion, one or more magnets, one or more crush ribs, and/or the like.

The sled assembly **540** further includes a vial chamber **558** that is sized and shaped to receive the priming assembly **560** and the vial assembly **580** therein, respectively. In other words, the vial chamber **558** is sized to individually receive both the priming assembly **560** and the vial assembly **580** separate from one another. The vial chamber **558** is encapsulated around a protective chamber or shield **557** disposed about the vial chamber **558.** The protective shield **557** is formed of a material configured to inhibit radioactive emissions from extending outwardly from the vial chamber **558,** such as, for example, a metal. Additionally, the sled assembly **540** includes a needle extending through the protective shield **557** and into the vial chamber **558** along a bottom end of the vial chamber **558.** The needle **559** is fixedly secured relative to the vial chamber **558** such that any devices received through the aperture of the locking system **550** and into the vial chamber **558** are to encounter and interact with the needle **559** (e.g., the priming assembly **560,** the vial assembly **580,** and the like).

Still referring to FIG. 15, the needle **559** is coupled to a distal manifold **555A** and a proximal manifold **555B** disposed within the sled assembly **540,** and in particular the manifold **555A, 555B** is positioned beneath the vial chamber **558** and the protective shield **557.** The proximal manifold **555B** is fluidly coupled to the needle **559** and the distal manifold **555A** is fluidly coupled to the one or more ports **556** of the sled assembly **540.** The proximal manifold **555B** is in fluid communication with the distal manifold **555A** through a one-way check valve **553** disposed therebetween. It should be understood that the one-way check valve **553** is configured to facilitate fluid communication from the proximal manifold **555B** to the distal manifold **555A** and prevent fluid communication from the distal manifold **555A** to the proximal manifold **555B.** In other words, the one-way check valve **553** prevents a backflow of fluid into the sled assembly **540** and/or the vial assembly **580** coupled thereto.

Accordingly, the proximal manifold **555B** is in fluid communication with the one or more ports **556** via the distal manifold **555A,** however, the one or more ports **556** are not in fluid communication with the proximal manifold **555B** due to a position of the one-way check valve **553** disposed between the manifolds **555A, 555B.** Thus, the needle **559** is in fluid communication with the one or more delivery lines and/or devices coupled to the sled assembly **540** at the one or more ports **556** via the manifolds **555A, 555B** secured therebetween. As will be described in greater detail herein, the one or more ports **556** of the sled assembly **540** may be coupled to a bag (e.g., saline bag), a syringe, a catheter, and/or the like via one or more delivery lines coupled thereto. In other embodiments, the needle **559** may be omitted entirely for an alternative device, such as, for example, a valve system, a needleless injection port, and/or the like.

Still referring to FIG. 15, the sled assembly **540** includes a removable battery pack **570** coupled to the sled assembly **540** along the distal end **544.** The removable battery pack **570** comprises a battery **572,** electrical contacts **574,** and a removable tab **576.** It should be understood that in some embodiments the removable battery pack **570** may be preloaded onto the sled assembly **540** while in other embodiments the removable battery pack **570** is separate from the sled assembly **540** such that an operator is required to couple the removable battery pack **570** to the sled assembly **540** along the distal end **544.** In either instance, the battery **572** of the delivery device **500** is isolated from one or more fluid paths and radiation sources due to a location of the battery **572** in the removable battery pack **570.**

The battery **572** may comprise various quantities and types of batteries for powering the delivery device **500,** such as, for example, four (4) disposable double-A (AA) batteries, alkaline batteries, Li-ion batteries, mignon batteries, single cell dry batteries, and/or the like. In some embodiments, the battery **572** may be encapsulated in a polymer or wax material. As will be described in greater detail herein, the electrical contacts **574** of the removable battery pack **570** extend outwardly from the removable battery pack **570** and are operable to contact against and interact with corresponding electrical contacts **511** of the console assembly **510** (*See* FIG. 13) when the sled assembly **540** is coupled to the base **512** at the sled cavity **532.** Accordingly, the removable battery pack **570** is operable to provide electrical power to the delivery device **500,** and in particular the console assembly **510,** when the sled assembly **540** is coupled to the console assembly **510.**

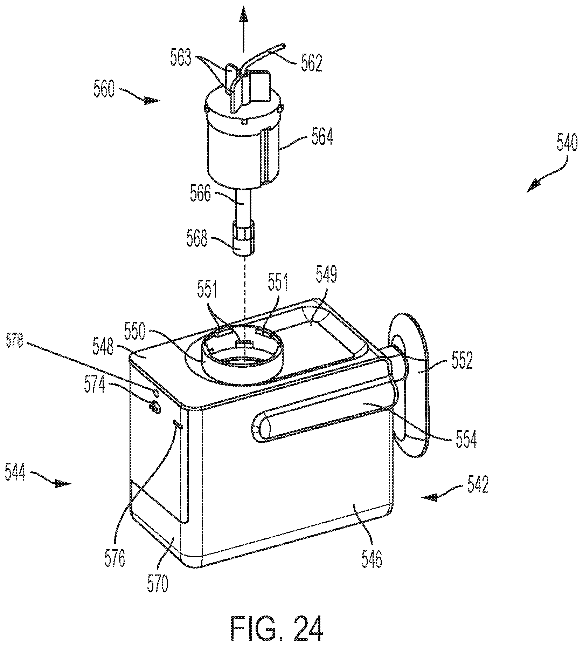

Still referring to FIG. 15, the removable tab **576** of the removable battery pack **570** is selectively removable from the removable battery pack **570.** The removable tab **576** is operable to check a battery status of the removable battery pack **570** upon removal of the removable tab **576.** As will be described in greater detail herein, removal of the removable tab **576** prior to a commencement of a procedure with the delivery device **500** provides an operator of the delivery device **500** an indication of whether the removable battery pack **570** contains sufficient power stored therein to perform a procedure. The removable battery pack **570** generates a feedback indicating a sufficiency of the battery **572** in response to a removal of the removable tab **576.** For example, in some embodiments the removable battery pack **570** includes a LED status indicator **578** (*see* FIG. 24) that visually displays a color indicative of a battery power of the battery **572** (e.g., green, yellow, red). In other embodiments, the removable battery pack **570** may include a speaker that generates an audible alert indicative of a battery power of the battery **572.** It should be understood that in other embodiments the sled assembly **540** and/or the console assembly **510** may be electrically powered by various other suitable power sources without departing from the scope of the present disclosure. For example, one or more of the sled assembly **540** and/or the console assembly **510** may be directly coupled to an external power supply, the console assembly **510** may include one or more batteries stored therein, and/or the like.

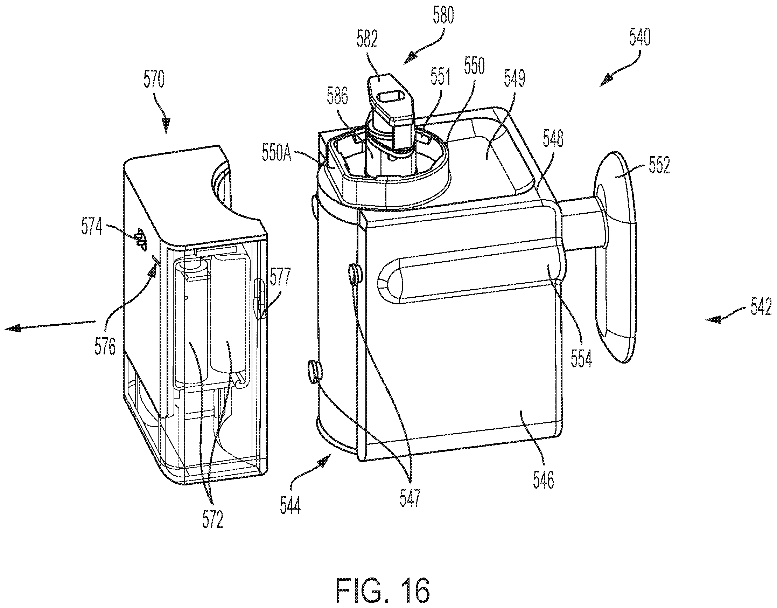

Referring now to FIG. 16, the sled assembly **540** includes one or more retention features **547** disposed along the distal end **544** of the sled assembly **540** for securing the removable battery pack **570** thereto. In particular, the retention features **547** of the sled assembly **540** comprise protrusions extending outwardly from the distal end **544.** The removable battery pack **570** includes one or more corresponding retention features **577** disposed along a surface of the removable battery pack **570** opposite of the electrical contacts **574**, where the corresponding retention features **577** of the removable battery pack **570** are configured to engage the retention features **547** of the sled assembly **540.** In particular, the retention features **577** of the removable battery pack **570** comprise recesses extending inwardly into the removable battery pack **570** to receive the retention features **547** of the sled assembly **540** therein, to thereby securely couple the removable battery pack **570** to the sled assembly **540** at the distal end **544.** It should be understood that various other retention features **547**, **577** may be included in the sled assembly **540** and the removable battery pack **570** than those shown and described herein without departing from the scope of the present disclosure. For example, corresponding retention features may comprise magnets, snaps, threads, and/or the like.

Additionally, as will be described in greater detail herein, in some embodiments the locking system **550** may include at least one planar wall **550A** relative to a remaining circular orientation of the locking system **550.** In this instance, an aperture formed by the locking system **550** through the top surface **548** of the sled assembly **540** is irregularly-shaped, rather than circularly-shaped as shown and described above. In this instance, the vial assembly **580** includes an locking feature **586** that has a shape and size that corresponds to the locking system **550**, and in particular the at least one planar wall **550A** such that the vial assembly **580** is received within the sled assembly **540** only when an orientation of the vial assembly **580** corresponds with an alignment of the locking feature **586** and the locking system **550.** In other words, a corresponding planar wall **586A** of the locking feature **586** (*See* FIG. 18) must be aligned with the planar wall **550A** of the locking system **550** for the vial assembly **580** to be receivable within an aperture formed by the locking system **550** of the sled assembly **540.**

Referring now to FIG. 17, the priming assembly **560** of the delivery device **500** is depicted. The priming assembly **560** comprises the priming line **562**, a handle **563**, a central body **564**, an elongated shaft **566**, and a needle end **568.** The central body **564** is sized and shaped to be slidably received within the vial chamber **558** of the sled assembly **540**, and in particular, includes a diameter that is substantially similar to a diameter of the vial chamber **558** such that a press-fit is formed between the central body **564** and the vial chamber **558** when the priming assembly **560** is received within the sled assembly **540.** The handle **563** and the elongated shaft **566** are integrally formed with the central body **564**, with the handle **563** extending vertically outward from the central body **564** at an end opposite of the elongated shaft **566.**

In other words, the handle **563** extends relatively upward from the central body **564** and the elongated shaft **566** extends relatively downward from the central body **564**, in a direction opposite of the handle **563.** Accordingly, when the priming assembly **560** is slidably received within the vial chamber **558** of the sled assembly **540**, the handle **563** is positioned adjacent to the top surface **548** of the sled assembly **540** and the elongated shaft **566** is disposed within the sled assembly **540.** The handle **563** is configured to facilitate grasping and maneuvering the priming assembly **560** for insertion into and extraction out of the sled assembly **540**, respectively. It should be understood that in other embodiments the handle **563**, the central body **564**, and/or the elongated shaft **566** may be separate components assembled together to form the priming assembly **560.**

Still referring to FIG. 17, the elongated shaft **566** is sized at a predetermined length to thereby position the central body **564** and the handle **563** of the priming assembly **560** proximate to the aperture formed by the locking system **550** of the sled assembly **540.** In this instance, the handle **563** may be readily accessible to an operator of the delivery device **500** via the aperture formed by the locking system **550.** It should be understood that a collective longitudinal length of the handle **563**, the central body **564**, and the elongated shaft **566** is substantially similar to a longitudinal length of the vial chamber **558** of the sled assembly **540** such that the handle **563** is partially disposed within the vial chamber **558** and/or partially exposed from the vial chamber **558** (*See* FIG. 13).

The elongated shaft **566** of the priming assembly **560** further includes the needle end **568** positioned along a terminal end of the elongated shaft **566** opposite of the central body **564.** The needle end **568** is formed of a material that is operable to receive the needle **559** of the sled assembly **540** therethrough in response to the priming assembly **560** being received within the vial chamber **558** of the sled assembly **540.** For example, the needle end **568** of the priming assembly **560** may be formed of an elastomer material that is configured to be punctured by the needle **559** when the needle end **568** is slidably inserted through the vial chamber **558** and positioned against the needle **559.** In the present example, the priming assembly **560** further includes one or more alignment features **565A, 565B** positioned along the central body **564** that are configured to maintain the priming assembly **560** in the vial chamber **558** of the sled assembly **540.**

Referring now to FIG. 18, the vial assembly **580** of the delivery device **500** is depicted. The vial assembly **580** comprises an engagement head **582**, a plunger **584**, an locking feature **586**, and a vial body **589.** In particular, the engagement head **582** of the vial assembly **580** is positioned at a terminal end of the plunger **584** opposite of the locking feature **586** and the vial body **589.** The engagement head **582** includes a pair of arms **581** extending laterally outward relative to a longitudinal length of the plunger **584** extending downwardly therefrom. In the present example, the engagement head **582** is integrally formed with the plunger **584**, however, it should be understood that in other embodiments the engagement head **582** and the plunger **584** may be separate features fastened thereto. In either instance, the engagement head **582** and the plunger **584** is movable relative to the locking feature **586** and the vial body **589** such that the engagement head **582** and the plunger **584** are slidably translatable through the locking feature **586** and the vial body **589.** In particular, as will be described in greater detail herein, the plunger **584** may translate into and out of an internal chamber **588** of the vial body **589** in response to a linear translation of the vial engagement mechanism **520** when the engagement head **582** is secured to the pair of lever arms **522.**

The plunger **584** includes a plurality of indicia and/or markings **583** positioned along a longitudinal length of the plunger **584.** The plurality of markings **583** is indicative of a relative extension of the engagement head **582** and the plunger **584** from the locking feature **586** and the vial body **589.** As briefly noted above, the engagement head **582** is configured to attach the vial assembly **580** to the vial engagement mechanism **520.** In particular, the pair of arms **581** of the engagement head **582** are sized and shaped to couple with the pair of lever arms **522** of the vial engagement mechanism **520** when the vial assembly **580** is received within the sled assembly **540** and the sled assembly is inserted into the sled cavity **532** of the console assembly **510.** As will be described in greater detail herein, the pair of lever arms **522** are received between the pair of arms **581** of the engagement head **582** and the plunger **584** in response to a predetermined translation force applied to the vial engagement mechanism **520.** The engagement head **582** and the plunger **584** may be formed of various materials, including, but not limited to, a metal, plastic, and/or the like.

Still referring to FIG. 18, the vial assembly **580** further includes a safety tab **585** coupled to the plunger **584** relatively above the locking feature **586** and below the engagement head **582** such that the safety tab **585** is positioned along the longitudinal length of the plunger **584.** The safety tab **585** may be formed of various materials, such as, for example, a plastic, and is preassembled onto the vial assembly **580** prior to a use of the delivery device **500.** The safety tab **585** is removably fastened to the plunger **584** and inhibits the plunger **584** from translating relative to the vial body **589.** In particular, the safety tab **585** abuts against the locking feature **586** in response to an application of linear force onto the plunger **584** to translate the plunger **584** relatively downward into the vial body **589.** In this instance, the safety tab **585** is configured to inhibit an inadvertent movement of the plunger **584**, and in response, an inadvertent delivery of a fluid media stored within the internal chamber **588** of the vial body **589** (e.g., therapeutic particles, radioembolizing beads). As will be described in greater detail herein, the safety tab **585** is selectively disengaged from the plunger **584** in response to a coupling of the vial assembly **580** with the vial engagement mechanism **520**, and in particular an engagement of the pair of lever arms **522** with the engagement head **582.**

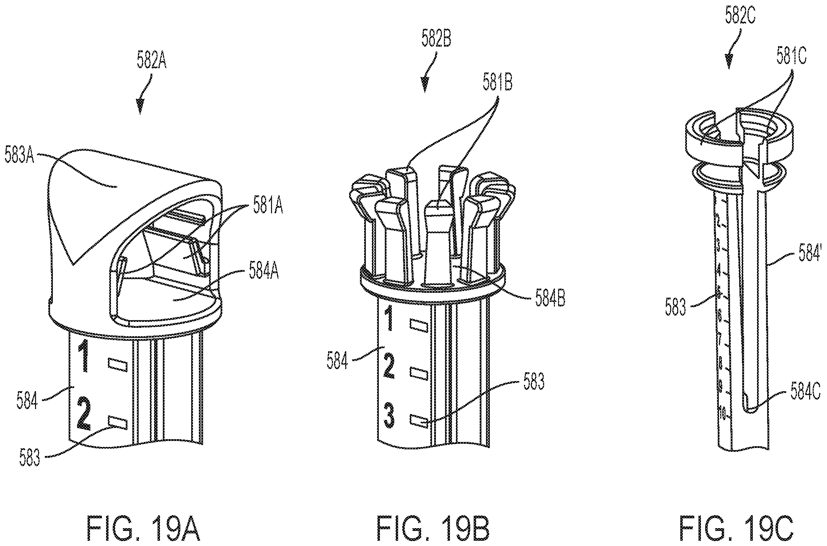

Although the engagement head **582** of the vial assembly **580** is shown and described herein as including a pair of arms **581** extending laterally outward from the plunger **584**, it should be understood that the engagement head **582** may include various other structural configurations suitable for engaging the pair of lever arms **522** of the vial engagement mechanism **520.** For example, referring now to FIGS. 19A-19C, alternative embodiments of an engagement head of the vial assembly **580** are depicted. It should be understood that the engagement heads shown and described herein may be similarly incorporated onto the vial assembly **580** as the engagement head **582** described above.

Referring now to FIG. 19A, an alternative embodiment of an engagement head **582A** is depicted. The engagement head **852A** comprises a ring **583A** that defines an aperture with a top planar surface **584A** of the plunger **584.** The ring **583A** includes at least a pair of flexible tabs (resilient arms) **581A** extending laterally inwardly from the ring **583A** and into the aperture formed therein. In particular, the pair of flexible tabs **581A** is separated from one another at opposing sides of the ring **583A** and are angled relative inward toward one another. In this instance, the pair of flexible tabs **581A** is transverse relative to a longitudinal length of the plunger **584.** In the present example, the pair of flexible tabs **581A** is manually flexible such that the pair of flexible tabs **581A** may be selectively compressed outwardly away from one another when an inward force is applied thereto (e.g., from the pair of lever arms **522** received through the ring **583A**). The pair of flexible tabs **581A** is resiliently biased to expand outward relative to one another and into the aperture defined by the ring **583A** in a default state.

In the present example, the pair of lever arms **522** of the vial engagement mechanism **520** may be received through the aperture formed by the ring **583A** such that the pair of lever arms **522** are positioned between, and engaged against, the pair of flexible tabs **581A.** In this instance, the engagement head **582A** is securely fastened to the vial engagement mechanism **520.** It should be understood that the engagement head **582A** of the present example may be configured and operable to correspond to alternative embodiments of a vial engagement mechanism that includes features distinct from the pair of lever arms **522** of the vial engagement mechanism **520** shown and described above.

Referring now to FIG. 19B, an alternative engagement head **582B** is depicted. The engagement head **582B** comprises a plurality of flexible fingers **581B** extending verticallyupward from the plunger **584.** In particular, the plurality of flexible fingers **581B** extends parallel to, and in coaxial alignment with, a longitudinal length of the plunger **584.** A terminal end of each of the plurality of flexible fingers **581B** is positioned relatively above a top planar surface **584B** of the plunger **584.** In the present example, the plurality of flexible fingers **581B** is manually flexible such that the plurality of flexible fingers **581B** may be selectively compressed inward toward one another when an outward force is applied thereto. The plurality of flexible fingers **581B** is resiliently biased to expand outward away from one another in a default state.

Accordingly, inserting the engagement head **582B** into the vial engagement mechanism **520**, and in particular between the pair of lever arms **522** of the vial engagement mechanism **520**, causes the plurality of flexible fingers **581B** to be compressed inwardly and thereby engage against the pair of lever arms **522** that are disposed about the plurality of flexible fingers **581B.** In this instance, the plurality of flexible fingers **581B** is securely fastened to the vial engagement mechanism **520** through an outward expansion of the flexible fingers **851B** against the pair of lever arms **522.** It should be understood that the engagement head **582B** of the present example may be configured and operable to correspond to alternative embodiments of a vial engagement mechanism that includes features distinct from the pair of lever arms **522** of the vial engagement mechanism **520** shown and described above.

Referring now to FIG. 19C, another alternative engagement head **582C** is depicted. The engagement head **582C** comprises a pair of flexible clamps **581C** positioned above a plunger **584'.** The plunger **584'** of the present embodiment is different than the plunger **584** of the prior embodiments in that the plunger **584'** is bifurcated along a longitudinal length of the plunger **584'** with the bifurcation extending from the pair of flexible clamps **581C** of the engagement head **582C** to a terminal end **584C.** The pair of flexible claims 581C extends parallel to, and in coaxial alignment with, a longitudinal length of the plunger **584.** In the present example, the pair of flexible clamps **581C** is manually flexible such that the pair of flexible clamps **581C** may be selectively compressed inward toward one another when an outward force is applied thereto. The pair of flexible clamps **581C** is resiliently biased to expand outward away from one another in a default state.

Accordingly, inserting the engagement head **582C** into the vial engagement mechanism **520**, and in particular between the pair of lever arms **522** of the vial engagement mechanism **520**, causes the pair of flexible clamps **581C** to be compressed inwardly and thereby engage against the pair of lever arms **522** that are disposed about the pair of flexible clamps **581C.** In this instance, the pair of flexible clamps **581C** is securely fastened to the vial engagement mechanism **520** through an outward expansion of the flexible clamps **581C** against the pair of lever arms **522.** It should be understood that the engagement head **582C** of the present example may be configured and operable to correspond to alternative embodiments of a vial engagement mechanism that includes features distinct from the pair of lever arms **522** of the vial engagement mechanism **520** shown and described above. It should further be understood that various other configurations and geometries of an engagement head may be incorporated with the vial assembly **580** without departing from the scope of the present disclosure. For example, in others embodiments the engagement head of the vial assembly **580** may comprise one or more magnets, threads, cams, and/or the like.

Referring back to FIG. 18, the locking feature **586** extends about a top end of the vial body **589.** In the present example, the locking feature **586** of the vial assembly **580** comprises a bushing that defines a lateral edge **587** extending laterally outward along an outer perimeter of the locking feature **586.** The lateral edge **587** of the locking feature **586** is sized and shaped to engage the annular array of projections **551** of the locking system **550** when the vial assembly **580** is received within the vial chamber **558** of the sled assembly **540.** As will be described in greater detail herein, the locking feature **586**, and in particular the lateral edge **587** of the locking feature **586**, is configured to securely fasten the vial assembly **580** to the locking system **550** to inhibit removal of the vial body **589** from the vial chamber **558** of the sled assembly **540** during use of the delivery device **500** in a procedure. In some embodiments, as briefly described above, the locking feature **586** includes at least one planar wall **586A** such that the locking feature **586** comprises an irregular-profile. The at least one planar wall **586A** is configured to correspond to the planar wall **550A** of the locking system **550** such that an alignment of the planar walls **550A**, **586A** is required for the vial assembly **580** to be received through an aperture formed by the locking system **550.**

It should be understood the planar walls **550A**, **550B** serve to ensure that the safety tab **585** of the vial assembly **580** is coupled to the plunger **584** in a manner that allows for a removal of the safety tab **585** by the vial engagement mechanism **520.** In particular, the pair of lever arms **522** of the vial engagement mechanism **520** is configured to exert a lateral force against the safety tab **585** in response to the sled assembly **540** being slidably received within the sled cavity **532.** Accordingly, an orientation of the safety tab **585** relative to the pair of lever arms **522** may be facilitated to ensure ease of removal of the safety tab **585**, when the sled assembly **540** is coupled to the console assembly **510**, by requiring a proper alignment of the vial assembly **580** with the locking system **550** when the vial assembly is coupled to the sled assembly **540.**

Still referring to FIG. 18, the vial body **589** extends downwardly relative from the locking feature **586** and has a longitudinal length that is sized to receive at least a portion of a longitudinal length of the plunger **584** therein. By way of example only, a longitudinal length of the vial body **589** may be about 8 millimeters to about 10 millimeters, and in the present example comprises 9 millimeters, while a longitudinal length of the plunger **584** may be about 9 millimeters to about 11 millimeters, and in the present example comprises 10 millimeters. Accordingly, in some embodiments a longitudinal length of the plunger **584** exceed a longitudinal length of the vial body **589** such that a translation of the plunger **584** into the internal chamber **588** of the vial body **589** causes a fluid media stored therein to be transferred outward from the vial body **589.** As will be described in greater detail herein, a translation of the plunger **584** through the internal chamber **588** of the vial body **589** provides for an administration of a fluid media stored within the vial body **589** outward from the vial assembly **580.** The vial body **589** may be formed of various materials, including, for example, a thermoplastic polymer, copolyester, polycarbonate, a biocompatible plastic, polysulfone, ceramics, metals, and/or the like.

The vial body **589** is of the present example is formed of a material that is configured to inhibit radioactive emissions from a fluid media stored within the internal chamber **588** of the vial body **589.** For example, the vial body **589** may be formed of a plastic, such as polycarbonate, and have a width of approximately 9 millimeters (mm). A density and material composition of the vial body **589** may collectively inhibit gamma radiation emission from electron particles stored within the internal chamber **588.** In the present example, a chemical composition of the plastic of the vial body **589**, along with the 9 mm wall thickness, provides a plurality of atoms disposed within the vial body **589** that are capable of encountering the electron particles generating beta radiation and reducing an emission of said radiation from the vial assembly **580.** Accordingly, the vial assembly **580** allows an operator to handle the radioactive material stored within the vial body **589** without being exposed to beta radiation. It should be understood that various other materials and/or wall sections may be incorporated in the vial body **589** of the vial assembly **580** in other embodiments without departing from the scope of the present disclosure.

Still referring to FIG. 18, the vial body **589** of the vial assembly **580** is sealed at a first terminal end by the locking feature **586.** The vial assembly **580** further includes a cap **590** positioned at an opposing, terminal end of the vial body **589** opposite of the locking feature **586,** such that the cap **590** seals a second terminal end of the vial body **589** of the vial assembly **580.** Additionally, the vial assembly **580** includes a septum **592** positioned adjacent to the cap **590** and in fluid communication with a terminal end of the vial body **589** opposite of the locking feature **586.** The septum **592** forms a seal against a terminal end of the vial body **589** and the cap **590** retains the septum **592** therein. The septum **592** may be formed of various materials, including, for example, an elastomer, silicon, bromobutyl elastomer, rubber, urethanes, and/or the like. The septum **592** is configured to provide an air-tight seal for the vial body **589** to thereby inhibit a release of a fluid media stored therein (e.g., radioembolizing beads). As will be described in greater detail herein, the septum **592** of the vial assembly **580** is configured to be punctured by the needle **559** of the sled assembly **540** when the vial assembly **580** is received within the vial chamber **558**, thereby establishing fluid communication between the vial body **589** and the sled assembly **540.** In other embodiments, the septum **592** may be omitted entirely for an alternative device, such as, for example, a valve system, needle injection port, and/or the like.

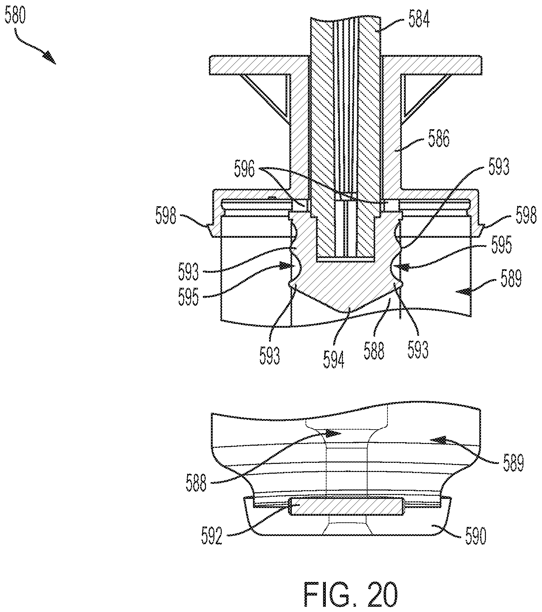

Referring to FIG. 20, the vial assembly **580** further includes a stopper **594** fixedly coupled to a terminal end of the plunger **584** opposite of the engagement head **582.** In this instance, with the plunger **584** coupled to, and slidably translatable through, the internal chamber **588** of the vial body **589**, the stopper **594** is effectively disposed within the vial body **589.** Accordingly, it should be understood that the stopper **594** is sized and shaped in accordance with a size (e.g., a diameter) of the internal chamber **588** of the vial body **589.** The stopper **594** is secured to the plunger **584** such that the stopper **594** is slidably translatable through the vial body 589 in response to a translation of the plunger 584 through the vial body 589. The stopper 594 is defined by two or more ribs 593 extending laterally outward and one or more troughs 595 defined between at least two ribs 593. In the present example, the stopper 594 includes six ribs 593 and two cavities formed therebetween, however, it should be understood that additional and/or fewer ribs 593 and troughs 595 may be included in the stopper 594 in other embodiments.

The stopper 594 is configured to form a liquid-seal against the internal chamber 588 of the vial body 589, and is formed of a various polymers with a predetermined viscoelasticity. For example, in some embodiments the stopper **594** is formed of an elastomer, silicone, rubber, urethane, plastic, polyethylene, polypropylene, and/or the like. In this instance, the stopper **594** is operable to inhibit a fluid media stored within the vial body **589** from extending (i.e., leaking) past the stopper **594** and out of the vial body **589.** In particular, the two or more ribs **593** of the stopper **594** abut against, and form a seal along, the internal chamber **588** of the vial body **589** to thereby inhibit a fluid media from passing beyond the ribs **593.** The one or more troughs **595** formed between the two or more ribs **593** of the stopper **594** are configured to receive, and more specifically capture, any fluid media that may inadvertently extend (i.e., leak) beyond the ribs **593** of the stopper **594.** Accordingly, the one or more troughs **595** serve as a safety mechanism of the vial assembly **580** to ensure a fluid media is maintained within the vial body **589** and not exposed beyond the vial assembly **580.**

Still referring to FIG. 20, the two or more ribs **593** of the stopper **594** are additionally configured to push a fluid media stored within the vial body **589** in one or more directions therein (e.g., toward the cap **590**) in response to a translation of the plunger **584.** With the ribs **593** of the stopper **594** pressed against the internal chamber **588** of the vial body **589**, translation of the plunger **584** provides for a translation of the ribs **593** against and along the internal chamber **588** of the vial body **589** such that any fluid media located in front (i.e., beneath) of the stopper **594** is effectively redirected within the vial body **589** in a direction of travel of the plunger **584** and the stopper **594.** The vial assembly **580** further includes an annular washer **596** disposed within the vial body **589.** In particular, the annular washer **596** is securely fixed to the plunger **584** adjacent to the stopper **594**, which is secured to the plunger **584** at a terminal end opposite of the engagement head **582.** Accordingly, the annular washer **596** is secured to the plunger **584** and disposed within the vial body **589** adjacent to the stopper **594.** With the annular washer **596** secured to the plunger **584** adjacent to the stopper **594**, the annular washer **596** is effectively disposed within the vial body **589.**

The annular washer **596** may be formed of various materials, including, for example, a plastic, metal, and/or the like. The annular washer **596** may be fixedly secured to the plunger **584** via various suitable means, including, for example, by an adhesive. It should be understood that the annular washer **596** is sized and shaped in accordance with a size (e.g., a diameter) of the internal chamber **588** of the vial body **589** such that the annular washer **596** slidably translates within the internal chamber **588** of the vial body **589** simultaneous with the plunger **584** and the stopper **594.** The annular washer **596** is configured to inhibit a removal of the plunger **584** from the vial body **589** by abutting against a bottom end of the locking feature **586** when the plunger **584** is translated relatively outward (i.e., upward) to a fullest extent. In other words, with the annular washer **596** securely fixed to a terminal end of the plunger **584** that is disposed within the vial body **589**, and with the plunger **584** having a size that is smaller than the vial body **589** to allow for a translation of the plunger **584** therethrough, the annular washer **596** serves to form an impediment for the plunger **584** to be translated outward of the vial body **589.** The annular washer **596** is configured to engage a bottom end of the locking feature **586** in response to a retraction of the plunger **584** from the vial body **589** at a predetermined distance (i.e., predetermined length of the plunger **584**).

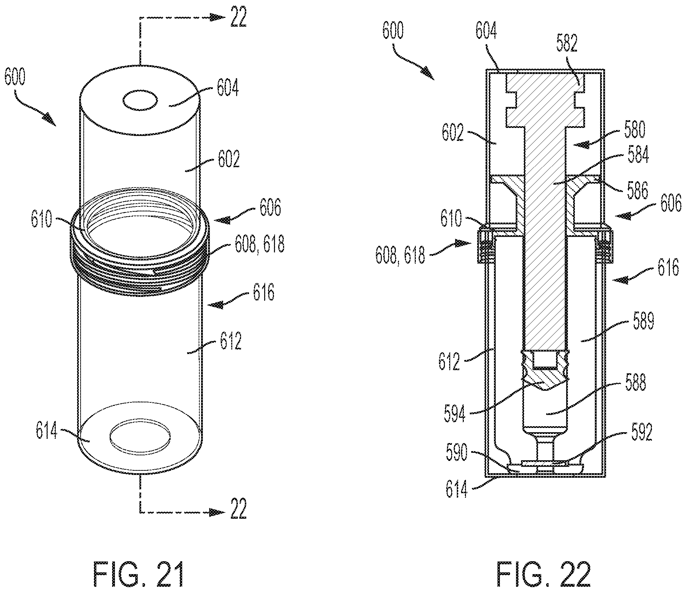

Referring now to FIG. 21, a sterile container assembly **600** is depicted. The sterile container assembly **600** is sized and shaped to receive the vial assembly **580** therein for storing and transporting the vial assembly **580** prior to use of the vial assembly **580** while maintaining a sterility of the vial assembly **580.** The sterile container assembly **600** comprises a top housing **602** including a closed end **604** and an open end **606**, and a bottom housing **612** including a closed end **614** and an open end **616.** The closed ends **604**, **614** of both housings **602**, **612** of the sterile container assembly **600** include a material that is operable to form a liquid seal, such as, for example, a synthetic material, polyethylene fiber, and/or the like. The seal formed at the closed ends **604**, **614** of both housings **602**, **612** are configured to permit steam penetration therethrough for sterilization of the contents of the housings **602**, **612**.

The open ends **606**, **616** of both housings **602**, **612** include corresponding mating system **608**, **618** that are configured to couple the housings **602**, **612** to one another. In the present example, the mating systems **608**, **618** of the sterile container assembly **600** are corresponding threaded portions positioned along the open ends **606**, **616** of each of the housings **602**, **612** such that the threaded portions are configured to mesh with one another to secure the top housing **602** to the bottom housing **612.** It should be understood that various other mating systems **608**, **618** may be incorporated with the sterile container assembly **600** without departing from the scope of the present disclosure, such as, for example, magnets, elastics, snaps, and/or the like. The sterile container assembly **600** may be formed of various materials, including, but not limited to, a metal, plastic, and/or the like. The sterile container assembly **600** is configured and operable to inhibit leaks of therapeutic particles externally therefrom when the top housing **602** is coupled to the bottom housing **612** due to the liquid seals formed along the closed ends **604**, **614** and the gasket seal **610** formed between the open ends **606**, **616.**

Referring now to FIG. 22, the vial assembly **580** is depicted as being received within the sterile container assembly **600.** In particular, the cap **590** of the vial assembly **580** is received at and positioned proximate to the closed end **614** of the bottom housing **612** of the sterile container assembly **600.** Further, the engagement head **582** of the vial assembly **580** is received at and positioned proximate to the closed end **604** of the top housing **602** of the sterile container assembly **600.** In this instance, the open ends **606**, **616** of the housings **602**, **612** of the sterile container assembly **600** are secured to one another via the corresponding mating systems **608**, **618** of each of the housings **602**, **612.** In some embodiments, at least one of the top housing **602** and/or the bottom housing **612** includes a gasket seal adjacent to the open end **606**, **616** such that a seal is formed proximate to the mating systems **608**, **618** when the top housing **602** is coupled to the bottom housing **612.** In the present example, the top housing **602** includes an annular gasket seal **610** extending within the top housing **602** adjacent to the open end **606**, and in particular, along the mating system **608** of the top housing **602.** The gasket seal **610** is configured to form an airtight seal between the housings **602**, **612** of the sterile container assembly **600** when the mating systems **608**, **618** are coupled thereto.

In other embodiments, the vial assembly **580** may be stored and transferred to the delivery device **500** via a loading system (not shown). The loading system may include a radiation shielding and is configured to hold the vial assembly **580** therein. The loading system may include a removable sled that may be aligned with the vial engagement mechanism **520** of the console assembly **510**, where the sled includes one or more plates for providing radiation shielding that are formed of various materials, including lead, tungsten, and/or various other polymers. The lead plates may be formed of varying wall thicknesses, including, for example, 0.95 cm. In some embodiments, the loading system may be an extendable tray that selectively retracts and/or pivots back into place for use with the delivery device **500.** The sled of the loading system may include a trough along a portion of the loading system where the vial assembly **580** is stored such that the trough receives and maintains any spills and/or leaks of fluid media from the vial body **589.**

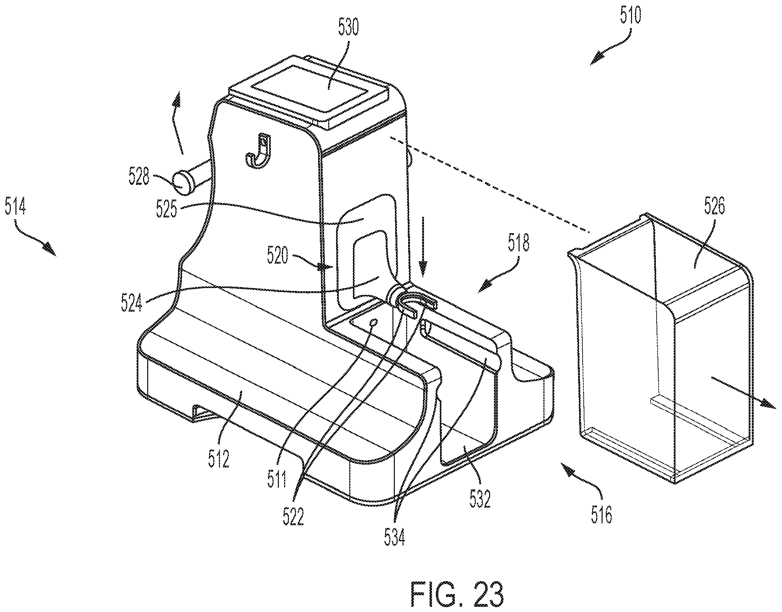

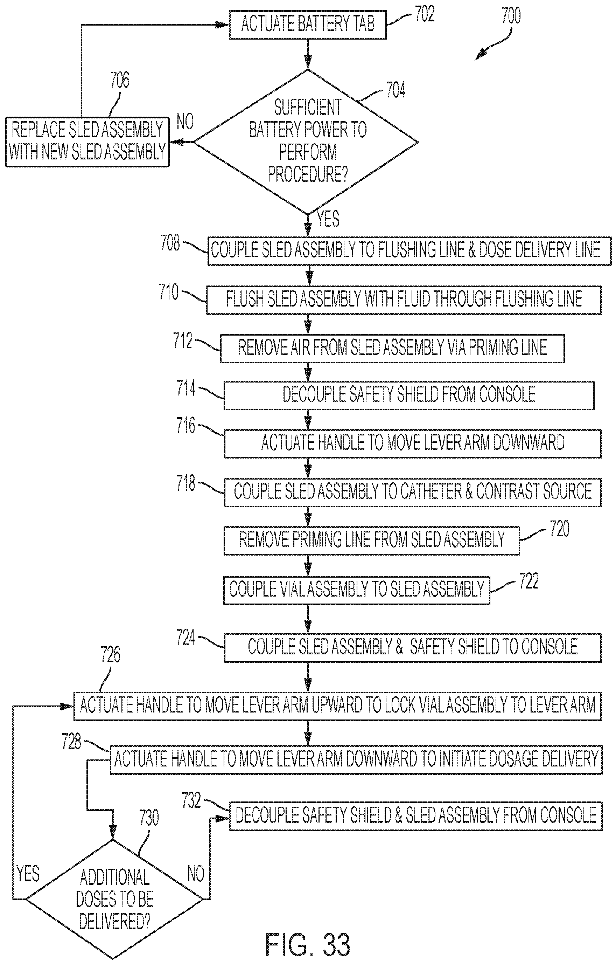

Referring now to FIGS. 23-32 in conjunction with the flow diagram of FIG. 33, an exemplary method **700** of operating the delivery device **500** is schematically depicted. The depiction of FIGS. 23-33 and accompanying description below is not meant to limit the subject matter described herein or represent an exact description of how a fluid media may be delivered using the delivery device **500**, but instead is meant to provide a simple schematic overview to illustrate a general administration of a radioactive media from the delivery device **500** described herein.

At step **702** of FIG. 33, the removable tab **576** of the removable battery pack **570** is actuated to determine a quantity of power contained within the battery **572** of the removable battery pack **570.** In particular, the removable tab **576** is removed from the removable battery pack **570** and a feedback output is generated identifying a status of the battery **572** of the removable battery pack **570.** At step **706**, an operator of the delivery device **500** determines whether the battery **572** of the removable battery pack **570** contains a sufficient amount of power to perform the procedure by observing the feedback output generated by the removable battery pack **570.** In the present example, the removable battery pack **570** includes an LED status indicator **578** (*see* FIG. 24) that displays a green light when the battery **572** contains sufficient amount of power to perform a procedure and a red light when the battery **572** contains an insufficient amount of power to perform a procedure.

In response to determining that the battery **572** contains an insufficient amount of power at step **704**, an operator replaces the sled assembly **540** with a new sled assembly **540** for use with the console assembly **510** to perform the procedure with at step **706.** Alternatively, in other embodiments an operator may decouple the removable battery pack **570** from the sled assembly **540** and attach a new removable battery pack **570** to the original sled assembly **540**, rather than replacing the sled assembly **540** entirely. In either instance, the exemplary method **700** returns to step **702** where the removable tab **576** of the new removable battery pack **570** is actuated to determine whether a sufficient amount of power exists in the battery **572** to perform the procedure.

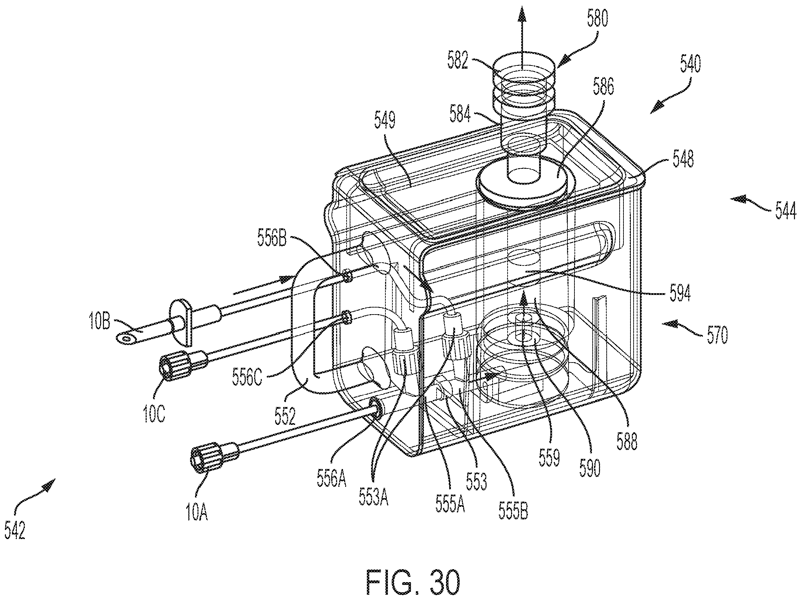

Referring now to FIG. 30, in response to determining that the battery **572** contains a sufficient amount of power at step **702**, one or more delivery lines are coupled to the sled assembly **540** via the one or more ports **556** at step **708.** In particular, a dose delivery line **10A** is coupled to the sled assembly **540** at a delivery port **556A**, a contrast line **10B** is coupled to the sled assembly **540** at a contrast port **556B**, and a flushing line **10C** is coupled to the sled assembly **540** at a flushing port **556C.** An opposing end of the dose delivery line **10A** is initially coupled to a fluid reservoir, such as, for example, a collection bowl. As will be described in greater detail herein, the dose delivery line **10A** may be subsequently coupled to an external device, such as a catheter, once the sled assembly **540** has been effectively primed by a fluid medium via the contrast line **10B.** An opposing end of the flushing line **10C** is coupled to an external device, such as, for example, a syringe. With both the dose delivery line **10A** and the flushing line **10C** coupled to the sled assembly **540**, the sled assembly **540** is flushed with a fluid medium (e.g., saline) from the syringe coupled to the flushing line **10C** at step **710.** In this instance, the fluid medium is injected through the flushing line **10C**, into the distal manifold **555A** of the sled assembly **540**, and out of the sled assembly **540** through the dose delivery line **10A.** Accordingly, the fluid medium is ultimately received at the collection bowl and disposed thereat by the dose delivery line **10A.** It should be understood that in other embodiments where the console assembly **510** and/or the sled assembly **540** are electrically coupled to an external power source in lieu of the removable battery pack **570** described above, the corresponding steps **702**, **704**, **706** of the exemplary method **700** described above may be substituted and/or omitted entirely without departing from the scope of the present disclosure.

With the distal manifold **555A** of the sled assembly **540** separated from the proximal manifold **555B** by the one-way valve **553** disposed therebetween, the fluid medium flushed through the distal manifold **555A** from the syringe (via the flushing port **556C**) is prevented from passing through the proximal manifold **555B** and the needle **559** coupled thereto. Rather, the fluid medium injected from the syringe and through the flushing line **10C** is received at the flushing port **556C**, passed through the distal manifold **555A** in fluid communication with the flushing port **556C**, and redirected by the one-way valve **553** towards the dose delivery port **556A** that is coupled to the dose delivery line **10A.** In this instance, the dose delivery line **10A** receives and transfers the fluid medium to the collection bowl coupled thereto, such that the fluid medium is not directed beyond the one-way valve **553** and into the proximal manifold **555B** that is in fluid communication with the needle **559.** It should be understood that step **710** may be repeated as necessary to effectively flush the sled assembly **540** and the dose delivery line **10A** coupled thereto.

Referring back to FIG. 24 at step **712**, the contrast line **10B** is coupled to the sled assembly **540** at a contrast port **556B.** An opposing end of the contrast line **10B** is coupled to a fluid medium supply, such as, for example, a bag secured to the console assembly **510** via the attachment device **538.** In the present example, the bag is a saline bag such that the fluid medium stored therein is saline. In this instance, with the sled assembly **540** including the priming assembly **560** positioned within the vial chamber **558** and the needle end **568** in fluid communication with the needle **559**, a syringe is fluidly coupled to the priming line **562** of the priming assembly **560** and a plunger of the syringe is drawn back to pull saline through the contrast line **10B**, the contrast port **556B**, the sled assembly **540**, the priming line **562** and into the syringe from the saline bag. The plunger of the syringe is thereafter pushed inwards to transfer the extracted saline back through the priming line **562**, the central body **564**, the elongated shaft **566**, and the needle end **568** of the priming assembly **560** such that the saline is received into the needle **559** of the sled assembly **540.** Accordingly, the manifolds **555A**, **555B** of the sled assembly **540** are effectively primed with the saline from the syringe as the needle **559** that received the saline from the priming assembly **560** is in fluid communication with the manifolds **555A**, **555B**. With the manifolds **555A**, **555B** in further fluid communication with the dose delivery line **10A** via the delivery port **556A**, the saline is effectively distributed to the collection bowl coupled thereto. It should be understood that step **712** may be repeated as necessary to remove all air from the sled assembly **540** and the collection line coupled thereto.

Referring now to FIG. 23 and at step **714**, the safety shield **526** of the console assembly **510** is decoupled from the base **512** such that the vial containment region **518** is exposed. With the vial engagement mechanism **520** positioned within the vial containment region **518**, and the safety shield **526** removed from the base **512** of the console assembly **510**, the vial engagement mechanism **520** is readily accessible to an operator of the delivery device **500.** At step **716**, the handle **528** of the console assembly **510** is actuated to thereby move the vial engagement mechanism **520** with the vial containment region **518.** More specifically, the handle **528** is translated and/or pivoted upward relative to the base **512** to thereby translate the pair of lever arms **522** and the neck **524** of the vial engagement mechanism **520** downward relative to the base **512**, such that the vial engagement mechanism **520** is positioned proximate to the sled cavity **532.**

Referring now to FIG. 30 and at step **718**, the sled assembly **540** is coupled to one or more external devices via the one or more ports **556.** In particular, the sled assembly **540** is fluidly coupled to a catheter (e.g., microcatheter) via the dose delivery line **10A** that is coupled to the delivery port **556A** of the sled assembly **540.** In this instance, the catheter is in fluid communication with the sled assembly **540** via the dose delivery line **10A.** Further at step **718**, the sled assembly **540** is fluidly coupled to a contrast source, such as, for example, a saline bag secured to the console assembly **510** via the attachment device **538** (*See* FIG. 13). The sled assembly **540** is in fluid communication with the saline bag via a contrast line **10B** coupled to the contrast port **556B** of the sled assembly **540.** In this instance, the saline bag is in fluid communication with the sled assembly **540** via the contrast line **10B** secured to the contrast port **556B.**

The contrast port **556B** is in fluid communication with the proximal manifold **555B** while the delivery port **556A** is in fluid communication with the distal manifold **555A.** As will be described in greater detail herein, saline from the saline bag may be withdrawn through the needle **559** of the sled assembly **540** and into the vial body **589** of the vial assembly **580** as the contrast port **556B** is coupled to the proximal manifold **555B**, rather than the distal manifold **555A** which is separated from the proximal manifold **555B** by the one-way check valve **553** disposed therebetween.

Referring now to FIG. 24 and step **720**, the priming assembly **560** is removed from the sled assembly **540** by grasping the handle **563** and withdrawing the priming assembly **560** outwardly from the vial chamber **558.** As the handle **563** is pulled from the sled assembly **540** through the aperture formed by the locking system **550**, the needle end **568** of the priming assembly **560** is decoupled from the needle **559** of the sled assembly **540.** In some embodiments, a feedback is generated (e.g., mechanical, tactile, etc.) indicating a detachment of the needle end **568** from the needle **559** such that an operator receives an indication of the disconnection.

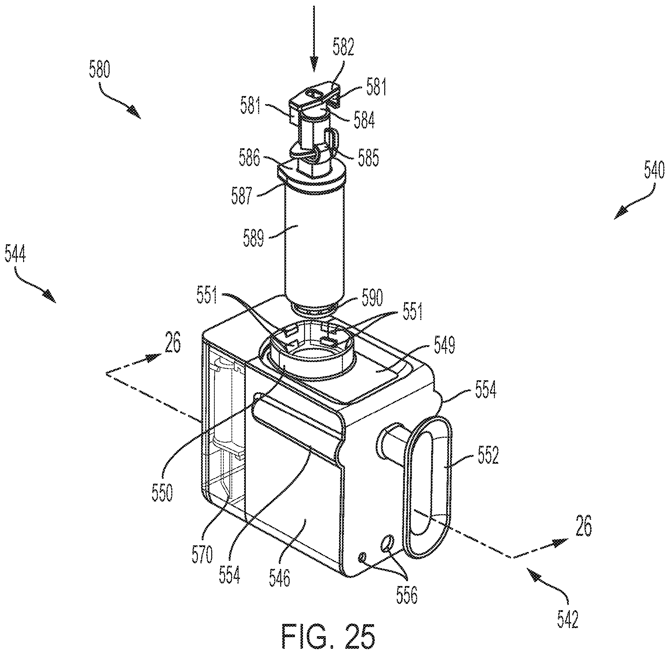

Referring now to FIG. 25 at step **722**, the vial assembly **580** is slidably inserted into the sled assembly **540.** In particular, the vial assembly **580** is removed from the sterile container assembly **600** in which the vial assembly **580** is initially stored in. The vial assembly **580** is removed from the sterile container assembly **600** by separating the housings **602**, **612** of the sterile container assembly **600** in response to a decoupling of the corresponding mating systems **608**, **618** of the housings **602**, **612.** With the sterile container assembly **600** containing the gasket seal **610** and the liquid seals along the closed ends **604**, **614** of both housings **602**, **612**, the sterile container assembly **600** effectively maintains the radioactive media stored within the vial assembly **580** during a storage and transport of the vial assembly **580** for use in the procedure. It should be understood that in some embodiments the sterile container assembly **600** housing the vial assembly **580** therein may be stored within a lead pot until use of the vial assembly **580** is required. The cap **590** of the vial assembly **580** is inserted through the aperture formed by the locking system **550** at the top surface **548** of the sled assembly **540** and the vial assembly **580** is gradually inserted therethrough until the locking feature **586** contacts the locking system **550.**

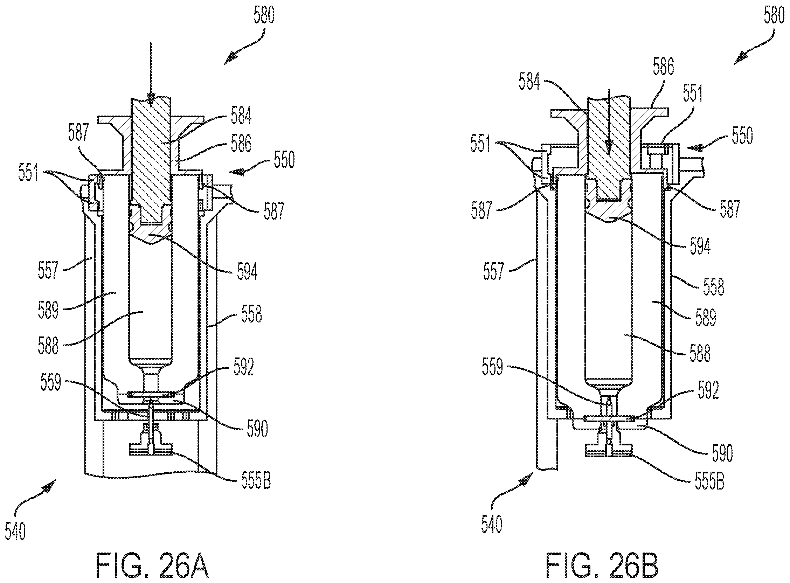

Referring now to FIG. 26A, the vial assembly **580** is shown disposed within the vial assembly **580**, and in particular the vial body **589** is inserted within the vial chamber **558** with the cap **590** positioned proximate to the needle **559.** In this instance, the lateral edge **587** of the locking feature **586** encounters a first row of the annular array of projections **551** of the locking system **550.** Continued advancement of the vial assembly **580** into the sled assembly **540** causes the annular array of projections **551** positioned along the first row to flex outwardly in response to an application of force generated thereon by the lateral edge **587.** In other words, the lateral edge **587** of the locking feature **586** presses outwardly against the annular array of projections **551** in response to the vial assembly **580** being received within the vial chamber **558.**

As the annular array of projections **551** of the locking system **550** flex outwardly relative to the lateral edge **587** disposed therein, a continued translation of the vial assembly **580** into the vial chamber **558** causes the lateral edge **587** of the locking feature **586** to advance beyond a first row of the annular array of projections **551** such that the applied-force thereon from the lateral edge **587** is removed. In this instance, the annular array of projections **551** along the first row are permitted to flex inwardly and return to a default position with the lateral edge **587** positioned underneath the first row of projections **551.** In some embodiments, a feedback is generated (e.g., an audible click) by the annular array of projections **551** when the lateral edge **587** is extended therethrough to thereby indicate to an operator that the vial assembly **580** is engaged with the locking system **550.** Accordingly, with the first row of projections **551** positioned over the lateral edge **587** of the locking feature **586**, the locking system **550** effectively inhibits a withdrawal of the vial assembly **580** from the vial chamber **558** of the sled assembly **540** due to an impediment formed by the first row of projections **551.** In this instance, the needle **559** is positioned against and/or received through the cap **590** but is not in contact with the septum **592.**

Referring now to FIG. 26B, a continued translation of the vial assembly **580** into the vial chamber **558** of the sled assembly **540** provides for a subsequent engagement between the lateral edge **587** of the locking feature **586** and the locking system **550.** In particular, the lateral edge **587** encounters a second row of the annular array of projections **551** of the locking system **550.** Continued advancement of the vial assembly **580** into the sled assembly **540** causes the projections **551** positioned along the second row to flex outwardly in response to an application of force generated thereon by the lateral edge **587.** As the lateral edge **587** advances past the projections **551**, the lateral edge **587** presses outwardly against the projections **551** until the lateral edge **587** of the locking feature **586** advances beyond the second row of projections **551.**

In this instance, the applied-force from the lateral edge **587** is removed and the annular array of projections **551** along the second row are permitted to flex inwardly and return to a default position with the lateral edge **587** positioned underneath the second row of projections **551.** Accordingly, with the second row of projections **551** positioned over the lateral edge **587** of the locking feature **586**, the locking system **550** effectively inhibits a withdrawal of the vial assembly **580** from the vial chamber **558** of the sled assembly **540** due to an impediment formed by the second row of projections **551.** In this instance, the needle **559** is positioned against and received through the cap **590** and the septum **592.** More particularly, the needle **559** punctures the septum **592** of the vial assembly **580** such that the sled assembly **540** is in fluid communication with the vial body **589** of the vial assembly **580** through the needle **559.**

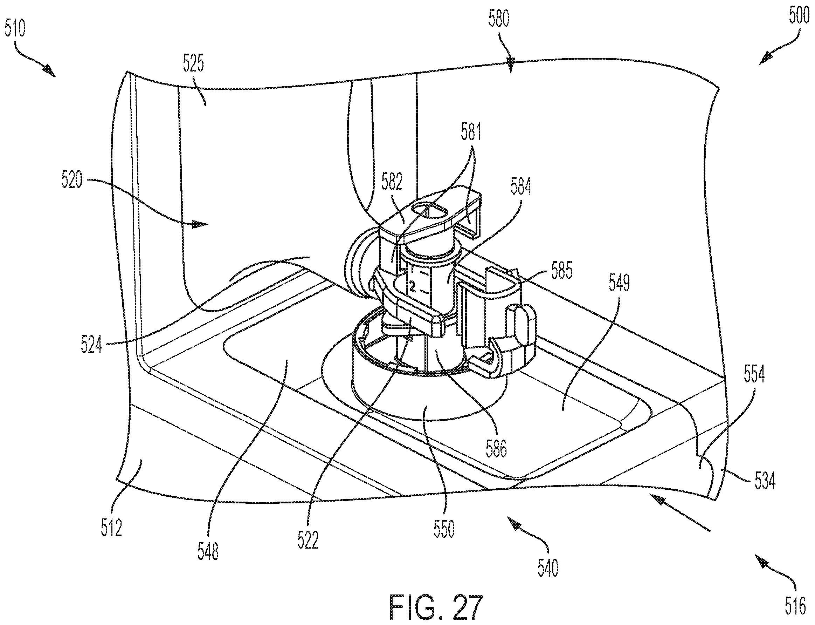

Referring now to FIG. 27 and at step **724**, with the vial assembly **580** securely coupled to the sled assembly **540**, the sled assembly **540** is coupled to the console assembly **510** by translating the proximal end **542** of the sled assembly **540** toward and into the distal end **516** of the console assembly **510.** In particular, the proximal end **542** of the sled assembly **540** is directed into the sled cavity **532** of the console assembly **510** by aligning the alignment ribs **554** of the sled assembly **540** with the alignment features **534** of the console assembly **510.** Once the distal end **544** and the proximal end **542** of the sled assembly **540** are fully seated within the sled cavity **532** of the console assembly **510**, the electrical contacts **574** of the removable battery pack **570** interact with corresponding electrical contacts **511** of the console assembly **510** *(See* FIG. 23). In this instance, power from the battery **572** is transmitted to the console assembly **510** via the electrical contacts **574**, thereby activating the console assembly **510** of the delivery device **500.** In this instance, the interface display **530** of the console assembly **510** is activated to display pertinent, real-time information relating to the delivery device **500** during a procedure.

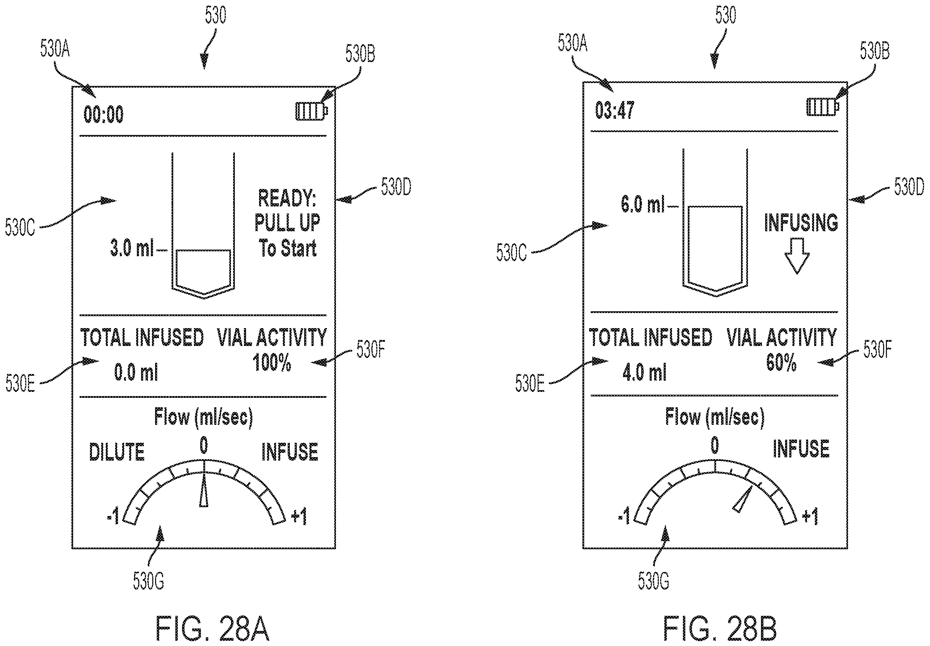

Referring to FIG. 28A, a schematic illustration of the interface display **530** is shown, where the interface display **530** of the console assembly **510** provides numerous data relating to the delivery device **500.** As merely an illustrative example, the interface display **530** of the present examples displays data relating to at least a total duration **530A** of a dose delivery; a lifespan **530B** of the battery **572**; a volume **530C** of fluid media stored in the vial assembly **580**; a current status **530D** of the delivery device **500**; a total volume **530E** of fluid media infused by the delivery device **500**; a radioactive percentage **530F** of the fluid media stored within the vial assembly **580**; and/or a volumetric infusion/dilution flow rate **530G** of fluid media being delivered and/or drawn by the delivery device **500.**

At step **724**, with the sled assembly **540** having been coupled to the console assembly 510, the interface display **530** indicates a commencement of a procedure with the delivery device **500** such that the data displayed thereon is indicative of such. As a use of the delivery device **500** progresses the data displayed along the interface display **530** may progressively update to reflect a current condition and characteristics of the delivery device **500.** It should be understood that the various information items **530A-530G** shown and described herein are merely for illustrative purposes such that additional and/or fewer data may be detected, monitored, and displayed by the delivery device **500** at the interface display **530.**

Referring back to FIG. 27, with the distal end **544** of the sled assembly **540** fully seated within the sled cavity **532** and the vial engagement mechanism **520** translated to a lower position at step **716**, the pair of lever arms **522** engage the safety tab **585** of the vial assembly **580** thereby decoupling the safety tab **585** from the plunger **584.** In other words, as the sled assembly **540** is translated into the sled cavity **532** in response to a force applied along the handle **552** at the proximal end **542**, a position of the lever arms **522** of the vial engagement mechanism **520** are aligned with and encounter the safety tab **585** of the vial assembly **580.** Accordingly, a continued translation of the sled assembly **540** into the sled cavity **532** provides for a disengagement of the safety tab **585** from the plunger **584** by the pair of lever arms **522.** In this instance, the plunger **584** of the vial assembly **580** is uninhibited from translating into and/or out of the internal chamber **588** of the vial body **589** in response to an actuation of the vial engagement mechanism **520** coupled thereto.

Additionally at step **724**, the safety shield **526** is coupled onto the base **512** of the console assembly **510** and over the vial containment region **518.** In this instance, with the safety shield **526** attached to the base **512** of the console assembly **510** over the vial containment region **518**, the safety shield **526** encloses the vial engagement mechanism **520**, the vial assembly **580**, and the sled assembly **540** within the vial containment region **518.** Accordingly, during a procedure with the delivery device **500**, the safety shield **526** maintains the one or more components of the delivery device **500** described herein enclosed within the vial containment region **518** to thereby shield an operator and/or patient from one or more fluid medias (e.g., radioembolizing microspheres) transferred between the console assembly **510**, the sled assembly **540**, and/or the vial assembly **580.**

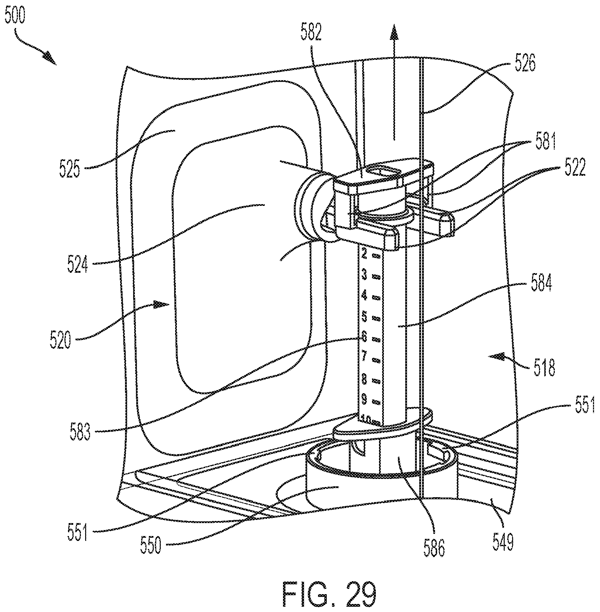

Referring now to FIG. 29 and at step **726**, the handle **528** of the console assembly **510** is actuated (e.g., translated relative downward) to thereby move (e.g., linearly translate) the vial engagement mechanism **520** within the vial containment region **518** distally away from the sled cavity **532** and the sled assembly **540** received therein. In this instance, with the pair of lever arms **522** of the vial engagement mechanism **520** positioned about the plunger **584** of the vial assembly **580**, translation of the neck **524** and the pair of lever arms **522** causes the pair of lever arms **522** to engage the engagement head **582**, and in particular a bottom end of the pair of arms **581.** With a removal of the safety tab **585**, the plunger **584** is operable to translate upward and out of the vial body **589** of the vial assembly **580** in response to a translation of the vial engagement mechanism **520.** Accordingly, the plunger **584** translates upward simultaneous with the translation of the vial engagement mechanism **520**, due to the pair of arms **581** of the engagement head **582** being pulled upwardly by the pair of lever arms **522**, in response to an actuation of the handle **528** at step **726.**

In this instance, the pair of lever arms **522** of the vial engagement mechanism **520** is not securely coupled to the pair of arms **581** of the engagement head **582.** Rather, the pair of lever arms **522** are merely positioned beneath the pair of arms **581** such that translation of the neck **524** of the vial engagement mechanism **520** causes the pair of lever arms **522** to abut against and pull the pair of arms **581** upward. It should be understood that the annular array of projections **551** of the locking system **550** inhibits a movement and/or an upward translation of the vial assembly **580**, and in particular the vial body **589**, from the vial chamber **558** of the sled assembly **540** as the vial engagement mechanism **520** pulls the plunger **584** of the vial assembly **580** relatively upward within the vial containment region **518.** Additionally, it should further be understood that the alignment features **534** of the console assembly **510** inhibit a movement and/or upward translation of the sled assembly **540** from the sled cavity **532** of the console assembly **510** as the vial engagement mechanism **520** pulls the vial assembly **580** stored within the sled assembly **540** relatively upward within the vial containment region **518.**

Still referring to FIG. 29, continued actuation of the handle **528** of the console assembly **510** provides for a continued translation of the vial engagement mechanism **520**, and the plunger **584** as a result, until the annular washer **596** encounters the locking feature **586** *(See* FIG. 20). In this instance, the annular washer **596** inhibits the plunger **584** from translating further relative to the vial body **589** despite a continued actuation of the handle **528** of the console assembly **510.** With the annular washer **596** of the vial assembly **580** abutting against the locking feature **586** and thereby inhibiting the plunger **584** from further translating out of the internal chamber **588** of the vial body **589** (and the aperture formed by the locking system **550**), continued actuation of the handle **528** causes the pair of arms **581** of the engagement head **582** to flex outwardly relative to the plunger **584** due to an upward force applied thereto by the pair of lever arms **522** in response to the vial engagement mechanism **520** translating upward and the plunger **584** being inhibited from moving further.

In other words, with the pair of lever arms **522** pressed against the pair of arms **581** of the engagement head **582**, continued translation of the neck **524** of the vial engagement mechanism **520** causes the pair of lever arms **522** to translate upward thereby applying a force against the pair of arms **581** of the engagement head **582.** With the engagement head **582** integrally formed with the plunger **584** and the plunger **584** inhibited from translating further relative to the vial body **589** due to an impediment formed between the annular washer **596** and the locking feature **586**, the pair of arms **581** of the engagement head **582** are flexibly deformed to expand outwardly to accommodate an upward translation of the pair of lever arms **522.** As a result, the pair of lever arms **522** of the vial engagement mechanism **520** are securely coupled to the pair of arms **581** of the engagement head **582** via a snap-fit engagement, thereby locking the vial engagement mechanism **520** to the vial assembly **580.**

Referring now to FIG. 30, as the vial engagement mechanism **520** and the plunger **584** are simultaneously translated within the vial containment region **518**, a negative pressure is generated within the internal chamber **588** of the vial body **589** due to a retraction of the stopper 594. In this instance, with the saline bag coupled to the sled assembly **540** via the contrast line 10B and the contrast port **556B**, saline from the saline bag is pulled into the internal chamber **588** of the vial body **589** through the proximal manifold **555B** and the needle **559.** Accordingly, with the vial body **589** being preloaded with a radioactive fluid media (e.g., radioembolizing microspheres), the saline is effectively mixed with the radioactive fluid media within the vial body **589** as the plunger **584** is retracted from the internal chamber **588** and the negative pressure is generated through the delivery device **500.**

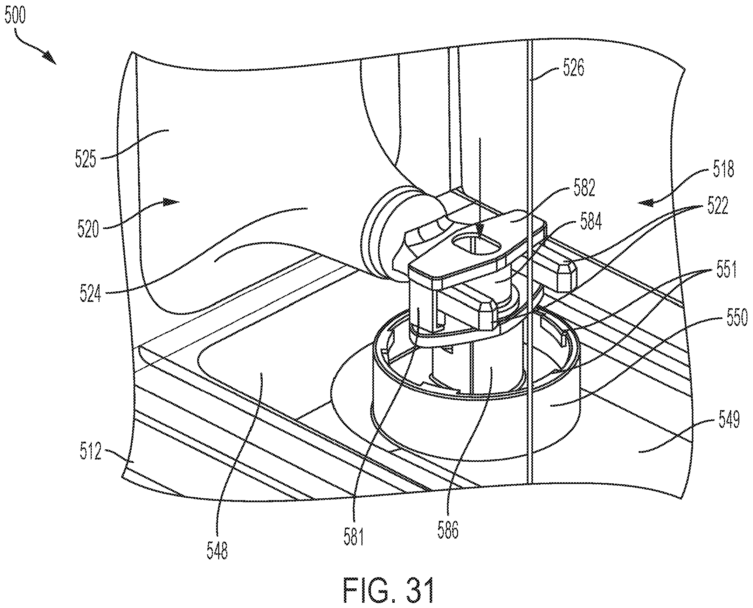

Referring now to FIG. 31 and at step **728**, actuation of the handle **528** in an opposite direction (e.g., translated and/or pivoted downward relative to the base **512**) provides for a simultaneous movement (e.g., linear translation) of the vial engagement mechanism **520.** In this instance, the neck **524** translates downward toward the sled cavity **532** thereby causing the plunger **584** to translate into the vial body **589** due to a secured engagement between the pair of arms **581** of the engagement head **582** and the pair of lever arms **522** of the vial engagement mechanism **520.** With the stopper **594** movably disposed within the vial body **589**, translation of the plunger **584** causes a simultaneous translation of the stopper **594** through the vial body **589** thereby generating a positive pressure therein. As a result, a dose of the saline and radioactive fluid media mixture stored within the internal chamber **588** is transferred outward of the vial body **589** through the needle **559** and into the proximal manifold **555B.** With the one-way check valve **553** configured to permit fluid communication from the proximal manifold **555B** to the distal manifold **555A**, the dose is delivered therethrough and into the dose delivery line **10A** via the dose delivery port **556A.**

Referring back to FIG. 30, the sled assembly **540** further includes one-way check valves **553A** in-line with the contrast line **10B** and the flushing line **10C.** In particular, the one-way check valves **553A** are configured to permit fluid communication from the contrast port **556B** and the flushing port **556C** into the manifolds **555A**, **555B**, and further configured to prevent fluid communication from the manifolds **555**A**, 555B** to the contrast port **556B** and the flushing port **556C.** Accordingly, it should be understood that the dose delivered from the vial body **589** to the manifold **555A**, **555B** is incapable of being directed into the contrast line **10B** or the flushing line **10C** due to the one-way check valves **553A** positioned therein. Thus, the dose is directed to the dose delivery port **556A** and received at the catheter fluidly coupled thereto by the dose delivery line **10A.** In other words, the one-way check valves **553A** prevent a backflow of fluid into the sled assembly **540** and/or the vial assembly **580** coupled thereto.

Referring now to FIG. 33 at step **730**, an operator determines whether delivery of additional doses from the delivery device **500** to the catheter is required during a procedure. In response to determining that additional doses for delivery are required at step **730**, step **726** and **728** are repeated as necessary to effectively delivery a required volume of the dosage. An operator may monitor the interface display **530** of the console assembly **510** to review the various information presented thereon to determine whether additional dose deliveries are necessary at step **730.** As described in greater detail above, the one or more sensors of the delivery device **500**, and in particular at least the linear displacement sensor **531** and the radiation sensor **533** are configured to detect and measure various characteristics of the delivery device **500** and/or the media stored therein for display at the interface display **530.**

Referring now to FIG. 28B, another schematic illustration of the interface display **530** is shown, where the interface display **530** of the console assembly **510** provides the various data relating to the delivery device **500** as described in greater detail above. In particular, at steps **726**, **728** and **730**, the interface display **530** continuously indicates a progressive status of the delivery device **500** during a procedure. As a use of the delivery device **500** progresses during steps **726**, **728** and **730**, the data displayed along the interface display **530** progressively updates to reflect a current condition and characteristic of the delivery device **500.**

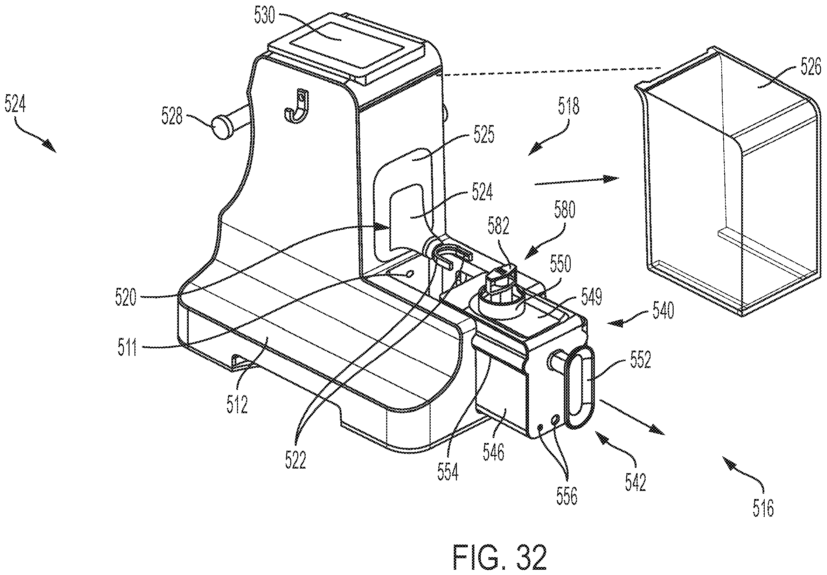

Referring to FIG. 32, in response to determining that additional doses for delivery are not required at step **730**, the safety shield **526** is decoupled from the base **512** of the console assembly **510** to thereby expose the vial containment region **518** encapsulated therein at step **732.** Additionally, the sled assembly **540** is decoupled from the sled cavity **532** of the console assembly **510** to thereby remove the sled assembly **540** from the vial containment region **518** at step **732.** Upon separating the distal end **544** of the sled assembly **540** from base **512** of the console assembly **510**, an engagement of the electrical contacts **574** of the removable battery pack **570** and the corresponding electrical contacts **511** of the console assembly **510** is terminated such that a power supply to the console assembly **510** is removed. Accordingly, the one or more components of the delivery device **500** requiring electrical power, such as, for example, the interface display **530**, cease to be operable. In this instance, the sled assembly **540** and the vial assembly **580** are collectively discarded due to the fixed assembly of the locking feature **586** and the locking system **550.** In other instances, the removable battery pack **570** is disengaged from the sled assembly **540** prior to discarding the sled assembly **540** and the vial assembly **580.** In this instance, the removable battery pack **570** containing the battery **572** is discarded separate from the sled assembly **540.**

### VI. Motorized Delivery Device with Sled Assembly

As briefly noted above, in some embodiments the delivery device **500** may include a motorized system in lieu of the mechanical assembly **529** shown and described herein. For example, the handle **528** may be communicatively coupled to the vial engagement mechanism **520** via an electrical linkage with at least one motor coupled therebetween. In this embodiment, actuation of the handle **528** to draw in a fluid media from the vial assembly **580** and to subsequently deliver the fluid media from the delivery device **500** is electrically-driven at a predetermined flow rate by computer readable and executable instructions executed by a processor. In other embodiments, the handle **528** is communicatively coupled to the vial engagement mechanism **520** via an electrical linkage with at least one motor coupled to each of the handle **528** and the vial engagement mechanism **520.** In this embodiment, actuation of the handle **528** to draw in the fluid media may be mechanically-driven as shown and described above, where the handle **528** is translated relatively downward by an operator to translate the vial engagement mechanism **520** linearly upward relative to the vial containment region **518.** It should be understood that the processor and memory storing the computer readable and executable instructions may be located at the delivery device **500**, a remote device, and/or both.

In either embodiment, a manual actuation of the handle **528** to infuse a dose of fluid media stored within the vial body **589** of the vial assembly **580** initiates a driving motor communicatively coupled to the handle **528**, where the driving motor is configured to generate a resistant force against the handle **528** proportionate and counter to the manual force applied thereto by an operator. In this instance, a haptic feedback is generated by the motor at the handle **528** in response to a physical manipulation of the handle **528** during a delivery of media from the delivery device **500.** A degree of resistive force generated by the motor at the handle **528** corresponds to a predetermined volumetric infusion flow rate preprogrammed in and/or determined by the computer readable and executable instructions executed by the processor. Accordingly, a manual manipulation of the handle **528** during an infusion process of the delivery device **500**, to a degree that alters a current infusion flow rate from the predetermined infusion flow rate, causes the motor to generate a resistance against the handle **528.**

It should be understood that the motor communicatively coupled to the handle **528** inhibits and does not prevent manual actuation of the handle **528**, such that a degree of resistive force and haptic feedback generated at the handle **528** corresponds to, and increases with, a variance of a current infusion flow rate from a predetermined infusion flow rate. In the present example, continued manual actuation of the handle **528** to a degree that increases a variance between a current infusion flow rate and a predetermined infusion flow rate causes the motor communicatively coupled to the handle **528** to progressively generate an increased resistive force thereto, thereby providing a greater haptic feedback for an operator indicative of the increased threshold. With another motor coupled to the vial engagement mechanism **520**, it should be understood that the driving motor coupled to the handle **528** is in communication with the motor coupled to the vial engagement mechanism **520** such that a manual actuation at the handle **528** is transmitted to the vial engagement mechanism **520.** In this instance, an input by an operator at the handle **528** that overcomes the resistive force applied thereto is proportionally applied a linear translation of the vial engagement mechanism **520.**

In other embodiments, the computer readable and executable instructions executed by the processor include a maximum variance threshold such that a manual actuation of the handle **528** by an operator of the delivery device **500** at a degree that exceeds the maximum variance threshold is prevented. The delivery device **500** may include one or more sensors coupled to the handle **528**, the plunger **584**, the vial engagement mechanism **520**, the manifold **555A**, **555B**, and/or other components of the delivery device **500** to detect and monitor various characteristics of the delivery device **500.** For example, the one or more sensors may be configured to measure a manual force applied by an operator to the handle **528**, a linear displacement of the vial engagement mechanism **520**, a current infusion flow rate of the delivery device **500**, a torque of the driving motor coupled to the handle **528** and/or the vial engagement mechanism **520**, and/or the like. By way of example, the one or more driving motors may comprise, but are not limited to, a linear stage actuator. Additionally, the one or more sensors may comprise, for example, a current sensor, a torque sensor, a pressure sensor, a flow sensor, and/or the like. Although not shown, it should be understood that in other embodiments the handle **528** of the delivery device **500** may be remotely located from the console assembly **510** such that a motor communicatively coupled to the handle **528** is similarly remote relative to the console assembly **510.**

In some embodiments, a manual actuation sensitivity of the handle **528** may be selectively programmed and adjusted prior to a use of the delivery device **500.** For example, the compute readable and executable instructions executed by the processor may include various settings for correlating a relative degree of movement at the handle **528** to a linear displacement of the vial engagement mechanism **520.** In this instance, a coarse and/or fine manipulation of the handle **528** may initiate varying torques at the driving motor communicatively coupled to the vial engagement mechanism **520** for translating the vial engagement mechanism **520** within the vial containment region **518.** An operator of the delivery device **500** may identify a predetermined infusion flow rate, a current infusion flow rate, and/or other data and characteristics pertaining a resistive force generated by the one or more driving motors along the interface display **530** of the console assembly **510.**

### VII. Dual-Component Plunger

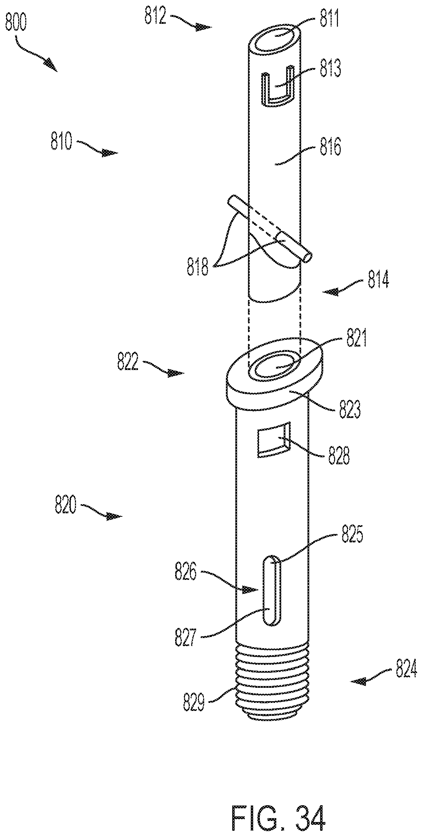

Referring now to FIG. 34, an alternative plunger assembly **800** is depicted. In the example shown and described herein, it should be understood that the plunger assembly **800** is configured and operable just like the plunger **584** described above except for the differences explicitly noted herein. Accordingly, the plunger assembly **800** of the present example may be readily incorporated into the vial assembly **580** described above. It should further be understood that the plunger assembly **800**, in many respects, functions substantially similar to the plunger **584** described above such that a version of the vial assembly **580** that is equipped with the plunger assembly **800** of the present example may be configured and operable similar to the vial assembly **580** described above with the plunger **584** except for the differences described below.

The plunger assembly **800** comprises an inner member **810** and an outer member **820**, with the outer member **820** sized and shaped to slidably receive the inner member **810** therethrough. In particular, the inner member **810** comprises a top end **812** and a bottom end **814** defining an elongated body **816** extending therebetween such that the ends **812**, **814** define a longitudinal length of the elongated body **816.** The top end **812** includes a top aperture **811** extending therethrough. It should be understood that the elongated body **816** defines a lumen extending through the inner member **810** from the top end **812** to the bottom end **814** such that the top aperture **811** is in communication with said lumen of the elongated body **816.** In the present example, the elongated body **816** of the inner member **810** is cylindrically-shaped similar to a shape of the vial body **589** in which the plunger assembly **800** is slidably received in.

Still referring to FIG. 34, the inner member **810** includes a pair of flexible latches **813** positioned along the elongated body **816** adjacent to the top end **812.** The pair of flexible latches **813** are resiliently biased to extend laterally outward from the elongated body **816.** As will be described in greater detail herein, an application of a laterally inward force onto the pair of flexible latches **813** (i.e. toward the elongated body **816**) causes the pair of flexible latches **813** to flexibly deform inwardly into the lumen defined by the elongated body **816.** The inner member **810** further includes a pair of pins **818** extending laterally outward from the elongated body **816** adjacent to the bottom end **814.** As will be described in greater detail herein, the pair of pins **818** are sized and shaped to be slidably received within a longitudinal slot **826** of the outer member **820.**

The outer member **820** of the plunger assembly **800** comprises a top end **822** and a bottom end **824** defining an elongated body extending therebetween such that the ends **822**, **824** define a longitudinal length of the elongated body. The top end **822** includes a top aperture **821** extending therethrough. It should be understood that the elongated body defines a lumen extending through the outer member **820** from the top end **822** to the bottom end **824** such that the top aperture **821** is in communication with said lumen of the outer member **820.** The elongated body of the outer member **820** is shaped substantially similar to the inner member **810** such that the outer member **820** is sized and shaped to slidably receive the inner member **810** through the lumen defined by the elongated body. Accordingly, the elongated body of the outer member **820** is cylindrically-shaped similar to a shape of the vial body **589** in which the plunger assembly **800** is slidably received in.

Still referring to FIG. 34, the outer member **820** includes an engagement head **823** extending about the elongated body adjacent to the top aperture **821.** In particular, the engagement head **823** extends about the elongated body at a lateral length such that the engagement head **823** includes a greater diameter than the elongated body. As will be described herein, a bottom surface of the engagement head **823** is sized such that the pair of lever arms **522** of the vial engagement mechanism **520** are received thereon in response to a vertical translation of the neck **524** and a corresponding linear displacement of the plunger assembly **800** relative to the vial body **589.** Accordingly, it should be understood that the engagement head **823** of the outer member **820** and the pair of flexible latches **813** of the inner member **810** collectively serve as an equivalent structural substitute for the pair of arms **581** of the engagement head **582** of the plunger **584.**

The outer member **820** further includes a pair of windows **828** disposed through the elongated body proximate to the top end **822** of the outer member **820.** The pair of windows **828** extend into the lumen defined by the elongated body and are sized and shaped in accordance with a size and shape of the pair of flexible latches **813.** As described in greater detail herein, the pair of windows **828** are configured to receive the pair of flexible latches **813** therethrough to securely fasten the inner member **810** to the outer member **820.** As briefly noted above, the outer member **820** includes a pair of longitudinal slots **826** extending through the elongated body adjacent to the bottom end **824.** In particular, the longitudinal slots **826** extend along opposing sides of the elongated body and are defined between an upper segment **825** and a lower segment **827.** The longitudinal slots **826** are sized and shaped to slidably receive at least one of the pair of pins **818** of the inner member **810** therethrough. Additionally, the outer member **820** includes a stopper **829** that is substantially similar to the stopper **594** described above such that the stopper **829** is configured and operable just like the stopper **594.**

Still referring to FIG. 34, in an exemplary mode of operation of the plunger assembly **800** with the vial assembly **580** described above, the inner member **810** is initially received with a lumen of the outer member **820** such that the top ends **812**, **822** are flush with one another and the pair of flexible latches **813** are disposed within the lumen of the outer member **820.** In particular, the pair of flexible latches **813** are positioned within the lumen of the outer member **820** between the top aperture **821** and the pair of windows **828.** In this instance, an inner surface of the elongated body of the outer member **820** applies a laterally inward force against the pair of flexible latches **813** such that the pair of flexible latches **813** are deformed inwardly into a lumen of the inner member **810.**

A resilient bias of the flexible latches **813** exerts an outward force against the laterally inward force generated by the elongated body such that a frictional interference is provided against the inner member **810** and the outer member **820** between the pair of flexible latches **813** and an inner surface of the elongated body. Accordingly, the inner member **810** is securely fixed within and relative to the outer member **820** prior to an actuation of the plunger assembly **800** in response to a linear translation of the vial engagement mechanism **520.**

Still referring to FIG. 34, with the pair of flexible latches **813** disposed within a lumen of the elongated body and positioned between the top aperture **821** and the pair of windows **828** in a default position, the pair of pins **818** of the inner member **810** is received within the longitudinal slot **826.** In particular, the pair of pins **818** are positioned along the upper segment **825** of the longitudinal slot **826** when the plunger assembly **800** is in a default position. With the plunger assembly **800** incorporated within the vial assembly **580** and the vial assembly **580** assembled with the sled assembly **540**, a coupling of the sled assembly **540** with the console assembly **510** provides for an engagement of the pair of lever arms **522** with a bottom surface of the engagement head **823.** Accordingly, an upward translation of the vial engagement mechanism **520** provides for an engagement with the bottom surface of the engagement head **823**, thereby translating the plunger assembly **800** vertically upward relative to the vial body **589** of the vial assembly **580.**

Still referring to FIG. 34, in the present example the vial body **589** and/or the locking feature **586** of the vial assembly **580** includes a retention feature that is sized and configured to engage the pair of pins **818** disposed within the vial body **589** upon a predetermined translation of the plunger assembly **800** relative to the vial body **589.** In other words, the retention feature is positioned within the vial body **589** and/or the locking feature **586** at a location such that the retention feature engages the pair of pins **818** thereon after the plunger assembly **800** is vertically translated a predetermined distance relative to the vial body **589.** It should be understood that a location of the retention feature, and the predetermined translation distance described above, is configured to correspond to a minimum threshold volume of fluid medium (e.g. saline) that is to be drawn into the internal chamber **588** in response to a linear displacement of the plunger assembly **800** therein.

Accordingly, locating the retention feature at the predetermined distance facilitates an extraction of the predetermined minimum volume threshold of fluid medium into the internal chamber **588** prior to a dose delivery by the delivery device. The predetermined minimum volume threshold may comprise various suitable quantities for creating a suitable mixture of the therapeutic particles and the fluid medium (e.g. saline) therein to ensure the resulting suspension fluid to be delivered is adequate for administration into a patient. For example, in some embodiments the predetermined minimum volume threshold may equal about **9** milliliters to **11** milliliters, and more specifically **10** milliliters.

Still referring to FIG. 34, once the plunger assembly **800** has translated the predetermined distance the pair of pins **818** arrive at, and engage, the retention feature thereby locking a vertical position of the pair of pins **818** thereat relative to the vial body **589.** Continued actuation of the vial engagement mechanism **520** provides for a continued translation of the pair of lever arms **522** and the outer member **820** due to an engagement of a bottom surface of the engagement head **823** with the pair of lever arms **522.** In this instance, the outer member **820** translates upward relative to the inner member **810**, a vertical position of which is fixedly secured due to an engagement of the pair of pins **818** and the retention feature, such that the pair of pins **818** translate along the longitudinal slot **826** from the upper segment **825** to the lower segment **827.** Additionally, the pair of flexible latches **813** of the inner member **810** translate within the lumen of the outer member **820** until arriving in alignment with the pair of windows **828.** In this instance, the pair of flexible latches **813** extend outwardly and through the pair of windows **828** due to a termination of the inward lateral force generated against the pair of flexible latches **813** by an inner surface of the outer member **820.** Accordingly, the pair of flexible latches **813** returns to a default configuration by extending laterally outward from a lumen of the elongated body **816** of the inner member **810** and through the pair of windows **828.**

It should be understood that the pair of pins **818** of the inner member **810** are positioned at the lower segment **827** of the longitudinal slot **826** as the pair of flexible latches **813** are aligned with and received in the pair of windows **828.** In this instance, the inner member **810** is fixedly secured to the outer member **820** such that a relative vertical position of the members **810**, **820** is fixed. It should further be understood that the pair of flexible latches **813** protrude outwardly from the pair of windows **828** at a predetermined length that effectively increases a lateral width of the outer member **820** at a location along the pair of windows **828.** In this instance, a downward translation of the neck **524** of the vial engagement mechanism **520** causes the pair of lever arms **522** to disengage from a bottom surface of the engagement head **823** and to engage the pair of flexible latches **813** positioned underneath such that the members **810**, **820** of the plunger assembly **800** are effectively translated downward into the internal chamber **588** to deliver a dose therefrom.

### VIII. Dual-Winged Plunger

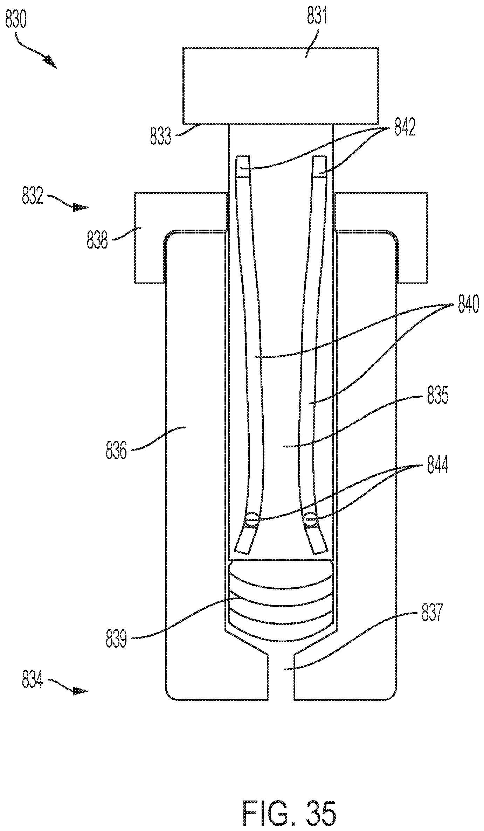

Referring now to FIGS. 35-36, an alternative vial assembly **830** is depicted. In the example shown and described herein, it should be understood that the vial assembly **830** is configured and operable just like the vial assembly **580** described above except for the differences explicitly noted herein. Accordingly, the vial assembly **830** of the present example may be readily incorporated into the sled assembly **540** described above. It should further be understood that the vial assembly **830**, in many respects, functions substantially similar to the vial assembly **580** described above such that a version of the sled assembly **540** that is equipped with the vial assembly **830** of the present example may be configured and operable similar to the sled assembly **540** described above with the vial assembly **580** received therein except for the differences described below.

Specifically referring to FIG. 35, the vial assembly **830** comprises an engagement head **831**, a locking feature **832**, a plunger **835**, a vial body **836** and a stopper **839.** The engagement head **831** and the stopper **839** define a longitudinal length of the plunger **835.** In other words, the engagement head **831** and the stopper **839** are positioned along opposing ends of the plunger **835.** The elongated head **831** of the present example includes a bottom surface **833** facing proximately toward the locking feature **832**, which includes a lateral edge **838** that extends about a top segment of the vial body **836.** The stopper **839** is coupled to a bottom segment of the plunger **835** and is configured and operable similar to the stopper **594** of the vial assembly **580** described above. The cap **834** of the vial assembly **830** includes an aperture **837** at a terminal end of the vial body **836** that is sized and shaped to receive the needle **559** of the sled assembly **540** when the vial assembly **830** is coupled thereto. It should be understood that in some embodiments the aperture **837** may comprise one or more features therein for receiving the needle **559**, such as, for example, an elastomer similar to the septum **592** of the vial assembly **580** described above.

The vial assembly **830** differs from the vial assembly **580** in that the plunger **835** includes a pair of flexible wings **840** coupled thereto. In particular, the pair of flexible wings **840** are movably coupled to an exterior surface of the plunger **835**, and extend along a longitudinal length of the plunger **835.** The pair of flexible wings **840** have a longitudinal length extending between a pivotable blade **842** and a rotatable coupler **844,** each of which are coupled to the exterior surface of the plunger **835.** In the present example, the pair of flexible wings **840** are shown in a default orientation with the pivotable blade **842** in a vertical configuration. With the pair of flexible wings **840** in a default orientation, a longitudinal length of the pair of flexible wings **840** are fully disposed within the vial body **836** of the vial assembly **830.** As will be described in greater detail herein, the pivotable blade **842** of the pair of flexible wings **840** is configured to pivot laterally outward away from the plunger **835** of the vial assembly **830** in response to a vertical translation of the plunger **835** out of the vial body **836.**

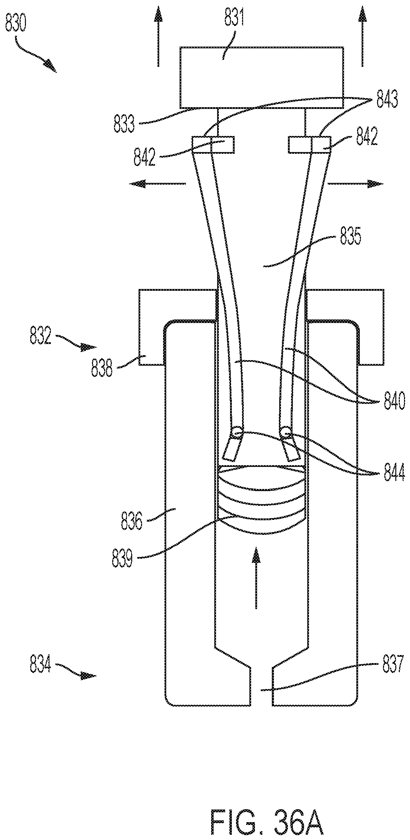

Referring now to FIG. 36A, in an exemplary mode of operation of the vial assembly **830**, the vial engagement mechanism **520** engages the engagement head **831**, and in particular the pair of lever arms **522** engage a bottom surface **833** of the engagement head **831.** In this instance, actuation of the handle **528** provides an upward translation of the neck **524** which thereby causes the pair of lever arms **522** to translate vertically upward. With the pair of lever arms **522** engaged against the bottom surface **833** of the engagement head **831**, the engagement head **831** and the plunger **835** are linearly displaced relative to the vial body **836** of the vial assembly **830.** With an upward translation of the plunger **835**, the pair of flexible wings **840** transition from a default orientation to a partially actuated position. In particular, the pair of flexible wings **840** rotate about the rotatable couplers **844** such that a longitudinal length of the pair of flexible wings **840** bow outward from the vial body **836.** In other words, the pair of flexible wings **840** are configured to flexibly deform such that a longitudinal length of the pair of flexible wings **840** are curved outward from the vial body **836.**

The pivotable blades **842** of the pair of flexible wings **840** pivot outwardly from the plunger **835** to thereby form an engagement surface **843** thereon. In other words, the pivotable blades **842** are configured to snap out and form the engagement surface **843** in response to a translation of the plunger **835** and a simultaneous rotation of the flexible wings **840** about the rotatable couplers **844.** It should be understood that a length of the engagement surface **843** formed by the pivotable blades **842** is configured to engage the pair of lever arms **522**, once the plunger **835** has translated a predetermined distance, with the predetermined distance corresponding to a minimum threshold volume of fluid medium (e.g. saline) that is to be drawn into the vial body **836** in response to a linear displacement of the plunger **835** therein.

Still referring to FIG. 36A, the plunger **835** is shown as translating a portion of the predetermined distance such that a length of the engagement surfaces **843** formed by the pivotable blades **842** of each of the flexible wings **840** is not operable to engage the pair of lever arms **522** during a downward translation of the neck **524** of the vial engagement mechanism **520.** Rather, the engagement surfaces **843** are partially formed in this instance such that an opposite translation of the vial engagement mechanism **520** will cause the pair of lever arms **522** to linearly translate by the pair of pivotable blades **842** and thus not interact with and/or engage a corresponding feature of the vial assembly **830.** In this instance, the plunger **835** is not pushed into the vial body **836**, thereby not administering a dose for delivery.

It should be understood that extending the pivotable blades **842** out further, in response to a continued upward translation of the plunger **835**, for engagement with the lever arms **522** facilitates an extraction of the predetermined minimum volume threshold of fluid medium into the vial body **836** prior to a dose delivery by the delivery device. The predetermined minimum volume threshold may comprise various suitable quantities for creating a suitable mixture of the therapeutic particles and the fluid medium (e.g. saline) therein to ensure the resulting suspension fluid to be delivered is adequate for administration into a patient. For example, in some embodiments the predetermined minimum volume threshold may equal about 9 milliliters to 11 milliliters, and more specifically 10 milliliters.

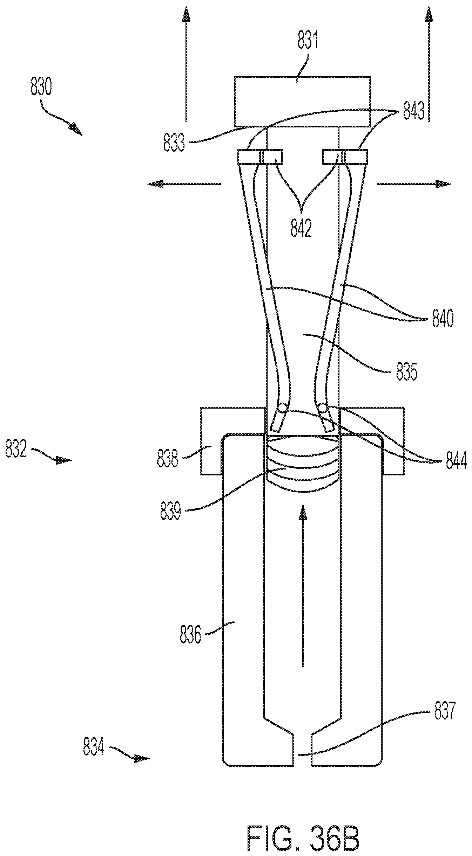

Referring now to FIG. 36B, once the plunger **835** has translated the predetermined distance the pair of pivotable blades **842** extend out from the plunger **835** at a greater length due to an increased deformation of the flexible wings **840.** Continued actuation of the vial engagement mechanism **520** provides for a continued translation of the pair of lever arms **522** and the plunger **835** due to an engagement of the bottom surface **833** of the engagement head **831** with the pair of lever arms **522.** In this instance, the plunger **835** translates upward relative to the vial body **836** such that the pair of flexible wings **840** bow out further from a longitudinal length of the plunger **835.** As a result, the pair of pivotable blades **842** extend laterally outward thereby forming the engagement surfaces **843** at a greater length. In this instance, the pair of pivotable blades **842** extend outwardly in a horizontal configuration.

It should further be understood that the pair of engagement surfaces **843** protrude outwardly from the plunger **835** at a predetermined length that effectively increases a lateral width of the plunger **835** at a location along the pair of pivotable blades **842.** In this instance, a downward translation of the neck **524** of the vial engagement mechanism **520** causes the pair of lever arms **522** to disengage from the bottom surface **833** of the engagement head **831** and to engage the engagement surface **843** of the pair of pivotable blades **842** positioned underneath such that the plunger **835** is effectively translated downward into the vial body **836** to deliver a dose therefrom.

### IX. Rotatable Plunger

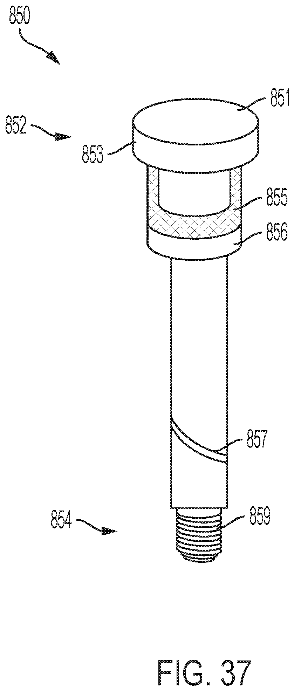

Referring now to FIGS. 37-38, an alternative plunger assembly **850** is depicted. In the example shown and described herein, it should be understood that the plunger assembly **850** is configured and operable just like the plunger **584** described above except for the differences explicitly noted herein. Accordingly, the plunger assembly **850** of the present example may be readily incorporated into the vial assembly **580** described above. It should further be understood that the plunger assembly **850**, in many respects, functions substantially similar to the plunger **584** described above such that a version of the vial assembly **580** that is equipped with the plunger assembly **850** of the present example may be configured and operable similar to the vial assembly **580** described above with the plunger **584** except for the differences described below.

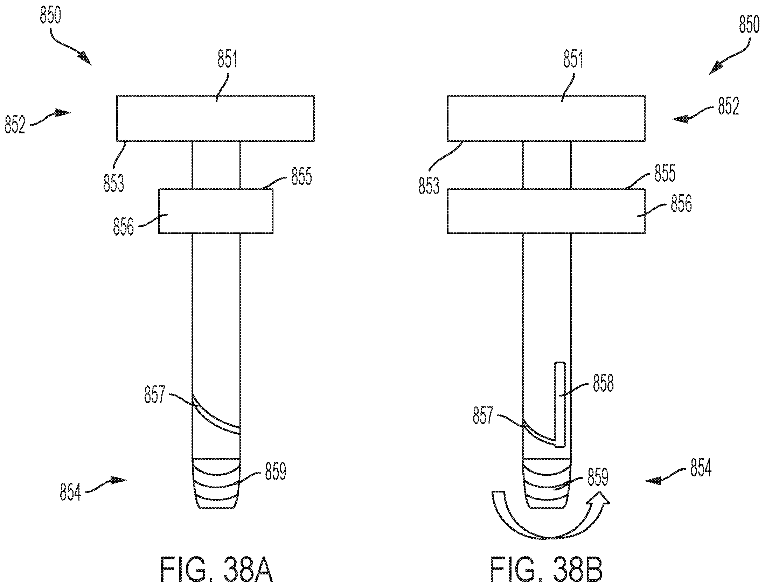

Referring specifically to FIG. 37, the plunger assembly **850** comprises a top end **852** and a bottom end **854** with a pair of engagement heads **851**, **856** positioned along the top end **852.** In particular, the plunger assembly **850** comprises an upper engagement head **851** and a lower engagement head **856**, with a bottom surface **853** of the upper engagement head **851** positioned relatively above an top surface **855** of the lower engagement head **856.** The plunger assembly **850** further includes a curved track **857** disposed along and extending about an exterior surface of the plunger assembly **850.** With the plunger assembly **850** of the present example comprising a cylindrically-shaped profile, the curved track **857** is formed thereon such that the curved track **857** extends about the cylindrical shape of the plunger assembly **850.** Although not shown, it should be understood that the curved track **857** is sized and shaped to slidably receive a pin from the vial body **589** and/or the locking feature **586** therein. In this instance, translation of the plunger assembly **850** with the pin received within the curved track **857** provides a translation of the plunger assembly **850** relative to the vial body **589** due a curved configuration of the curved track **857.** As described in greater detail herein, the plunger assembly **850** further includes a linear track **858** disposed along and extending on an exterior surface of the plunger assembly **850** (*see* FIG. 38B), where the linear track **858** is parallel to a longitudinal length of the plunger assembly **850.**

The plunger assembly **850** further includes a stopper **859** that is substantially similar to the stopper **594** of the plunger **584** described above. A size and shape of the upper engagement head **851** is distinct from a size and shape of the lower engagement head **856** such that the pair of engagement heads **851**, **856** have varying profiles relative to one another. In the present example, the upper engagement head **851** comprises a circularly-shaped profile and the lower engagement head **856** comprises an oval and/or oblong-shaped profile. It should be understood that the engagement heads **851**, **856** may comprise various other shapes and/or sizes than those shown and described herein without departing from a scope of the present disclosure. As will be described in greater detail herein, the shapes of the engagement heads **851**, **856** are configured to vary relative to one another to facilitate a delivery of a predetermined minimum threshold of fluid medium from the vial body **589.**

Referring now to FIG. 38A, the plunger assembly **850** is depicted in a first rotatable orientation relative to the vial body **589** of the vial assembly **580.** For example, in the first orientation a width of the upper engagement head **851** is greater than a width of the lower engagement head **856** due to the relatively varying profiles of the engagement heads **851**, **856**, respectively. With the upper engagement head **851** comprising a circular shape in the present example, it should be understood that the upper engagement head **851** comprises a similar profile in the first orientation as in a plurality of other orientations, including, for example, a second rotatable orientation shown in FIG. 38B. In contrast, with the lower engagement head **856** comprising an oval and/or oblong shape, the lower engagement head **856** comprises varying profiles in a plurality of orientations. For example, in the first orientation a width of the lower engagement head **856** is less than a width of the lower engagement head **856** in a second orientation shown in FIG. 38B.

In an exemplary mode of operation of the plunger assembly **850**, the vial engagement mechanism **520** is coupled to the plunger assembly **850** by receiving the pair of lever arms **522** between the upper engagement head **851** and the lower engagement head **856.** In particular, the pair of lever arms **522** of the vial engagement mechanism **520** are slidably positioned between the pair of engagement head **851**, **856** such that a vertical translation of the neck **524** of the vial engagement mechanism **520** causes an engagement of the bottom surface **853** of the upper engagement head **851** by the pair of lever arms **522** positioned underneath thereof. As briefly noted above, with the plunger assembly **850** received within the vial body **589** of the vial assembly **580**, a pin extending from the vial body **589** and/or the locking feature **586** is slidably received within the curved track **857** of the plunger assembly **850.**

Still referring to FIG. 38A, translation of the vial engagement mechanism **520**, with the pair of lever arms **522** engaged against the bottom surface **853** of the upper engagement head **851**, provides an upward translation of the plunger assembly **850** relative to the vial body **589.** With a fixed pin of the vial body **589** slidably coupled to the plunger assembly **850** within the curved track **857**, translation of the vial engagement mechanism **520** further provides a rotation of the plunger assembly **850** in a direction corresponding to a travel path of the fixed pin within the curved track **857.** It should be understood that in an initial default position, the fixed pin of the vial assembly **580** is received along a top portion of the curved track **857.** With the curved track **857** of the plunger assembly **850** extending relatively downward from the top portion toward the bottom end **854** and wrapping around the plunger assembly **850**, the curved track **857** is configured to facilitate a rotation of the plunger assembly **850** along with a simultaneous upward translation relative to the vial body **589.**

In this instance, the fixed pin travels through the curved track **857** from the top portion and toward a bottom portion of the curved track **857** adjacent to the bottom end **854.** With the curved track **857** extending about a cylindrical-shape of the plunger assembly **850**, the plunger assembly **850** is directed in a rotatable direction (e.g., counterclockwise, clockwise, etc.) from a first orientation to a second orientation (*See* FIG. 38B). It should be understood that a configuration and length of the curved track **857** corresponds to a predetermined translation distance that the plunger assembly **850** undergoes relative to the vial body **589.** The predetermined translation distance further corresponds to a minimum threshold volume of fluid medium (e.g. saline) that is to be drawn into the internal chamber **588** in response to a linear displacement of the plunger assembly **850** therein.

Accordingly, translating the fixed pin from a top portion of the curved track **857** to a bottom portion facilitates an extraction of the predetermined minimum volume threshold of fluid medium into the internal chamber **588** prior to a dose delivery by the delivery device as the plunger assembly **850** translates upward. The predetermined minimum volume threshold may comprise various suitable quantities for creating a suitable mixture of the therapeutic particles and the fluid medium (e.g. saline) therein (e.g. 10 milliliters) to ensure the resulting suspension fluid to be delivered is adequate for administration into a patient.

Referring now to FIG. 38B, once the fixed pin has slidably moved to a terminal end of the curved track **857** and the plunger assembly **850** has translated upward relative to the vial body **589** by the predetermined distance, the fixed pin is slidably received within the linear track **858** of the plunger assembly **850.** The linear track **858** is in connection with the curved track **857** and extends parallel to a longitudinal length of the plunger assembly **850.** In addition to a relocation of the fixed pin within the linear track **858**, moving the fixed pin through the curved track **857** provides for a rotation of the lower engagement head **856** to the second orientation due to a simultaneous rotation of the plunger assembly **850.** In this instance, due to the shape of the lower engagement head **856**, the lower engagement head **856** provides a larger lateral width positioned beneath the pair of lever arms **522.** Accordingly, actuation of the vial engagement mechanism **520** in a downward direction causes a disengagement of the pair of lever arms **522** with the bottom surface **853** of the upper engagement head **851** and a subsequent engagement with the top surface **855** of the lower engagement head **856.**

It should be understood that actuation of the vial engagement mechanism **520** in a downward direction prior rotating the lower engagement head **856** to the second orientation will not provide a corresponding downward translation of the plunger assembly **850.** In particular, a lateral width formed beneath the pair of lever arms **522** by the top surface **855** of the lower engagement head **856** is less than a width of the pair of lever arms **522** such that downward translation of the vial engagement mechanism **520** causes the pair of lever arms **522** to pass by the lower engagement head **856.**

Still referring to FIG. 38B, with the plunger assembly **850** rotated to the second orientation, continued actuation of the vial engagement mechanism **520** provides for a translation of the pair of lever arms **522** and the plunger assembly **850** due to an engagement of the top surface **855** of the lower engagement head **856** with the pair of lever arms **522.** In this instance, the fixed pin of the vial body **589** translates downward through the linear track **858**, a vertical position of which is fixedly to the vial body **589** such that the plunger assembly **850** moves relative to the vial body **589** to deliver a dose therefrom. It should be understood that the plunger assembly **850** maintains a fixed orientation relative to the vial body **589** when the fixed pin of the vial body **589** translates downward through the linear track **858.**

In other embodiments, the curved track **857** and the linear track **858** may be formed within the vial body **589** and/or the locking feature **586** of the vial assembly **580** such that the plunger assembly **850** includes the fixed pin extending laterally outward therefrom. In this instance, the plunger assembly **850** translates and rotates in a substantially similar manner as that described and shown herein as the fixed pin of the plunger assembly **850** travels along a travel path formed by the curved track of the vial assembly **580** prior to reaching a connection with the linear track of the vial assembly **580.** In this embodiment, a length and geometry of the curved track and/or the linear track of the vial assembly **580** may be substantially similar to the configuration of the tracks **857**, **858** shown and described herein.

### IIX. Suspension Chamber Vial Assembly

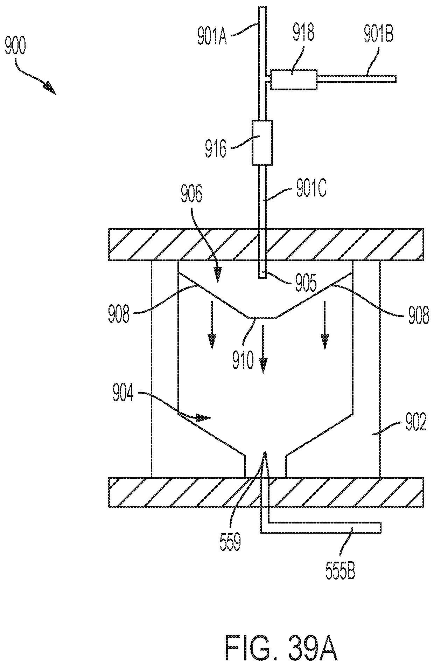

Referring now to FIGS. 39A-39B, an alternative vial assembly **900** is depicted. In the example shown and described herein, it should be understood that the vial assembly **900** is configured and operable just like the vial assembly **580** described above except for the differences explicitly noted herein. Accordingly, the vial assembly **900** of the present example may be readily incorporated into the sled assembly **540** described above. It should further be understood that the vial assembly **900**, in many respects, functions substantially similar to the vial assembly **580** described above such that a version of the sled assembly **540** that is equipped with the vial assembly **900** of the present example may be configured and operable similar to the sled assembly **540** described above with the vial assembly **580** received therein except for the differences described below.

Although not shown, it should be understood that the vial assembly **900** may include a locking feature disposed along a top end of the vial assembly **900** that is substantially similar to the locking feature **586** of the vial assembly **580** shown and described above. Accordingly, the vial assembly **900** of the present example is configured to be received in, and securely couple with, the sled assembly **540** via an interlocking engagement between the locking feature of the vial assembly **900** and the locking system **550** of the sled assembly **540.**

Specifically referring to FIG. 39A, the vial assembly **900** comprises a vial body **902** defining an inner chamber **904** with a pair of stoppers **908** and a floating septum **910** positioned therein. In particular, the pair of stoppers **908** and the floating septum **910** are disposed within the vial body **902** and are translatable with the inner chamber **904** in response to the vial assembly **900** receiving one or more fluid mediums therein. The pair of stoppers **908** are integrally formed with the floating septum **910**, and more specifically extend laterally outward therefrom at opposing ends of the floating septum **910.** The pair of pair of stoppers **908** are movably coupled to edges of the vial body **902** such that the pair of stoppers **908** are translatable thereon. With the floating septum **910** secured to the pair of stoppers **908**, translation of the pair of stoppers **908** within the vial body **902** provides for a simultaneous translation of the floating septum **910** in the inner chamber **904.**

It should be understood that the pair of pair of stoppers **908** are configured and operable similar to the stopper **594** of the vial assembly **580** shown and described above. Accordingly, the pair of stoppers **908** are configured to form a liquid-seal against the vial body **902** and are formed of various polymers with a predetermined viscoelasticity. For example, in some embodiments the stoppers **908** are formed of an elastomer, silicone, rubber, urethane, plastic, polyethylene, polypropylene, and/or the like. In this instance, the stoppers **908** are operable to inhibit a fluid media stored within the vial body **902** from extending (i.e., leaking) past the stoppers **908** and out of the vial body **902.** Further, the floating septum **910** is configured and operable similar to the septum **592** of the vial assembly **580** shown and described above. The septum **910** forms a seal against a terminal end of the vial body **902.** The septum **910** may be formed of various materials, including, for example, an elastomer, silicon, bromobutyl elastomer, rubber, urethanes, and/or the like. The septum **910** is configured to provide an airtight seal for the vial body **902** to thereby inhibit a release of a fluid media stored therein (e.g., radioembolizing beads). As will be described in greater detail herein, the septum **910** of the vial assembly **900** is configured to be punctured by the needle **559** of the sled assembly **540** when the vial assembly **900** is received within the vial chamber **558**, thereby establishing fluid communication between the vial body **902** and the sled assembly **540.**

Still referring to FIG. 39A, in an exemplary mode of operation of the vial assembly **900** with the sled assembly **540**, the vial body **902** of the vial assembly **900** is slidably received within the vial chamber **558** of the sled assembly **540** and a locking feature (not shown) of the vial assembly **900** securely fastens the vial body **902** therein in response to engaging the locking system **550** of the sled assembly **540.** As briefly noted above, it should be understood that a locking feature of the vial assembly **900** may be configured and operable substantially similar to the locking feature **586** of the vial assembly **580** shown and described above.

A delivery line **901A** is fluidly coupled to an external device, such as, for example, a syringe. Another delivery line **901**B is fluidly coupled to the delivery line **901**A via a once-way check valve **918** and to another external device, such as, for example, a bag containing a fluid medium therein (e.g. saline). It should be understood that the one-way check valve **918** is configured to permit fluid communication from the delivery line **901**B to the delivery line **901**A and simultaneously inhibit fluid communication from the delivery line **901**A to the delivery line **901**B. In this instance, the syringe is actuated to withdraw a fluid medium from the bag via the connection between the pair of delivery lines **901**A, **901**B and through the one-way check valve **918.** With the syringe filled with the fluid medium therein, subsequent actuation of the syringe provides for a delivery of fluid medium to the vial assembly **900** via a delivery line **901**C fluidly coupled to the syringe via a one-way check valve **916.** Similar to the valve **918** described above, the one-way check valve **916** is configured to permit fluid communication from the delivery line **901**A to the delivery line **901**C and simultaneously inhibit fluid communication from the delivery line **901**C to the delivery line **901**A.

Still referring to FIG. 39A, it should be understood that the pair of stoppers **908** and the floating septum **910** are positioned along an upper region of the inner chamber **904** of the vial body **902** in a default position prior to the syringe delivering fluid medium thereto via the delivery line **901**C**.** In this instance, the inner chamber **904** of the vial body **902** includes therapeutic particles preloaded therein such a volume of the therapeutic particles is determinative of a relative position of the pair of stoppers **908** and the floating septum **910** within the vial body **902.** As the syringe is actuated and the fluid medium stored therein is delivered through the delivery lines **901**A, **901**C, the fluid medium is received within the inner chamber **904** of the vial body **902** via an inlet port **905** disposed within the inner chamber **904.** In particular, the inlet port **905** is positioned relatively above a location of the pair of stoppers **908** and the floating septum **910** such that the inlet port **905** is separated from fluid communication with the needle **559** of the sled assembly **540** by the stoppers **908** and the floating septum **910** located therebetween.

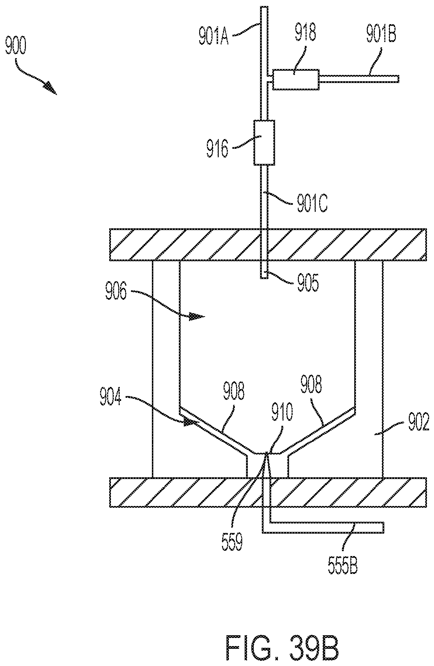

Referring now to FIG. 39B, as the fluid medium is received within the inner chamber **904** of the vial body **902** and mixed with the therapeutic particles preloaded in the vial body **902**, a volume of fluid in the inner chamber **904** is increased. In this instance, a pressure within the vial body **902** is increased and a force generated against the pair of stoppers **908** and the floating septum **910** causes the stoppers **908** and the floating septum **910** to translate within the vial body **902.** In particular, the stoppers **908** and the floating septum **910** are linearly displaced away from the inlet port **905** such that the pair of stoppers **908** and the floating septum **910** translate toward the needle **559** as the fluid volume in the inner chamber **904** increases. Upon the vial body **902** receiving a predetermined volume of fluid therein, the floating septum **910** translates a corresponding linear distance within the inner chamber **904** to thereby encounter the needle **559.** In this instance, the needle **559** punctures the floating septum **910** and the proximal manifold **555B** of the sled assembly **540** establishes fluid communication with the fluid stored within the vial body **902** through the needle **559.**

It is noted that the terms "substantially" and "about" may be utilized herein to represent the inherent degree of uncertainty that may be attributed to any quantitative comparison, value, measurement, or other representation. These terms are also utilized herein to represent the degree by which a quantitative representation may vary from a stated reference without resulting in a change in the basic function of the subject matter at issue.

For the purposes of describing and defining the present invention it is noted that the term "substantially" is used herein to represent the inherent degree of uncertainty that may be attributed to any quantitative comparison, value, measurement, or other representation. The term "substantially" is used herein also to represent the degree by which a quantitative representation may vary from a stated reference without resulting in a change in the basic function of the subject matter at issue. As such, it is used to represent the inherent degree of uncertainty that may be attributed to any quantitative comparison, value, measurement, or other representation, referring to an arrangement of elements or features that, while in theory would be expected to exhibit exact correspondence or behavior, may in practice embody something slightly less than exact.

While particular embodiments have been illustrated and described herein, it should be understood that various other changes and modifications may be made without departing from the scope of the claimed subject matter. Moreover, although various aspects of the claimed subject matter have been described herein, such aspects need not be utilized in combination. It is therefore intended that the appended claims cover all such changes and modifications that are within the scope of the claimed subject matter.

## Claims

1. A delivery assembly, comprising:
a console (510) including a vial containment region (518); a vial assembly (250,580);
a vial engagement mechanism (520) extending from the console (510) within the vial containment region (518), wherein the engagement mechanism (520) is configured to engage the vial assembly (250); and
a sled assembly (540) removably couplable to the console (510) at the vial containment region (518); wherein the sled assembly is configured to partially receive the vial assembly; **characterized in that**: the delivery assembly further comprises:
a safety shield (526) removably couplable to the console (510) over the vial containment region (518) such that the vial engagement mechanism (520) and the sled assembly (540) are encapsulated within the safety shield (526) when the safety shield (526) is coupled thereto; and **in that**:
the sled assembly (540), the vial assembly (250), and the safety shield (526) are configured to inhibit radioactive emissions from within the vial containment region (518).

2. The delivery assembly of claim 1, wherein the vial assembly comprises:
a vial body (589) formed of a material that inhibits radioactive emissions from within the vial body;
a locking feature (586) coupled to the vial body and configured to securely engage the vial assembly (250) to the sled assembly (540) when the vial body is received therein; and
a plunger (584; 835) slidably translatable relative to the locking feature (586) and through the vial body in response to the vial engagement mechanism (520) engaging the vial assembly (250),
wherein, preferably, the plunger (584) includes an engagement head (528A) having a pair of resilient arms (581A) that are sized and shaped to interlock with a pair of lever arms (522) of the vial engagement mechanism (520), or
wherein, preferably, the vial assembly (250) comprises a septum (255) disposed within a distal end of the vial body that is configured to seal the vial body.

3. The delivery assembly of any preceding claim, wherein the console (510) comprises an interface display (136) and one or more of a dosimeter, a linear encoder, an optical sensor, a linear displacement sensor, a flow sensor, an ultrasonic sensor, a magnetic encoder, a laser distance sensor, an inductance sensor, a radial encoder, a volumetric sensor, a radiation sensor, and a mechanical transducer, and wherein, preferably, the interface display (136) is communicatively coupled to one or more of the dosimeter, linear encoder, optical sensor, linear displacement sensor, flow sensor, ultrasonic sensor, magnetic encoder, laser distance sensor, inductance sensor, radial encoder, volumetric sensor, radiation sensor, and mechanical transducers.

4. The delivery assembly of any preceding claim, wherein the vial engagement mechanism (520) is disposed within the vial containment region (518) and proximate to the sled assembly (540) when the sled assembly (540) is coupled to the console (510), and further, preferably, comprising a delivery handle (528) coupled to the vial engagement mechanism (520) and configured to move the vial engagement mechanism (520) within the vial containment region (518), and wherein, preferably, the delivery handle (528) is configured to move the vial assembly (250) relative to the sled assembly (540) in response to actuating the delivery handle (528).

5. The delivery assembly of any preceding claim, wherein the sled assembly (540) comprises a locking system (550) and a vial chamber (558), wherein the locking system (550) forms an aperture sized and shaped to receive the vial assembly (250) therethrough and into the vial chamber(558) , wherein, preferably, the locking system (550) comprises annular projections extending laterally into the aperture, wherein the annular projections are sized and shaped to engage a locking feature of the vial assembly (250) when the vial assembly (250) is received through the aperture and into the vial chamber (558), or wherein, preferably, the vial chamber (558) is encapsulated in a protective shield (253) that is formed of a material that inhibits radioactive emissions from within the vial chamber (558).

6. The delivery assembly of claim 5, wherein the sled assembly (540) comprises a needle (559) extending within the vial chamber (558), the needle (559) being configured to puncture a septum of the vial assembly when the vial assembly is received within the vial chamber through the aperture, wherein, preferably, the sled assembly further comprises a manifold (555) fluidically coupled to the needle (559) such that the manifold (555) is in fluidic communication with the vial chamber (558) via the needle (559), and wherein, preferably, the sled assembly (540) comprises one or more ports that are in fluid communication with the vial chamber (558) through the manifold (555), wherein one or more delivery lines are operable to couple to the manifold (555) via the one or more ports.

7. The delivery assembly of any preceding claim, wherein the sled assembly (540), the vial assembly (250), and the safety shield (526) are formed of materials that inhibit radioactive emissions from within the vial containment region (518).

8. The delivery assembly of claim 2, wherein the plunger (584) includes an engagement head (582) having a bottom surface that is sized and shaped to interlock with a pair of lever arms (581) of the vial engagement mechanism, wherein, preferably, the vial assembly further includes a pair of flexible wings (840) rotatably coupled to the plunger (835) such that the pair of flexible wings (840) are configured to flexibly deform in response to translation of the plunger (835) outwardly from the vial body, and wherein, preferably, the pair of flexible wings (840) are configured to form an engagement surface that is sized and shaped to interlock with the pair of lever arms (581) in response to a deformation of the pair of flexible wings (840) as the plunger (835) translates outwardly from the vial body.

9. The delivery assembly of claim 2, wherein the plunger (835) includes an upper engagement head (851) and a lower engagement head (856) positioned relatively beneath the upper engagement head (851) along a longitudinal length of the plunger (835), wherein, preferably, the upper engagement head (851) includes a bottom surface (853) that is sized and shaped to interlock with a pair of lever arms (522) of the vial engagement mechanism (520) in response to an upward translation of the vial engagement mechanism (520), and wherein, preferably, the lower engagement head (856) includes a top surface (855) that is sized and shaped to interlock with the pair of lever arms (522) of the vial engagement mechanism (520) in response to a downward translation of the vial engagement mechanism (520).

10. The delivery assembly of claim 9, wherein the plunger (835) further includes at least a curved track (857) and a linear track (858) disposed along an exterior surface of the plunger (835), wherein the curved track (857) and the linear track (858) are sized and shaped to slidably receive a fixed pin of the vial body therein, wherein, preferably, the curved track (857) extends around the exterior surface of the plunger (835) and the linear track (858) extends along the exterior surface and parallel to a longitudinal length of the plunger (835).

11. The delivery assembly of claim 10, wherein the plunger (835) is configured to rotate within and translate outwardly from the vial body in response to the pair of lever arms (522) interlocking with the bottom surface of the upper engagement head (851) when the vial engagement mechanism (520) translates upward due to the fixed pin being received within the curved track (857), or wherein the plunger (835) is configured to translate into the vial body at a fixed orientation relative thereto in response to the pair of lever arms (522) interlocking with the top surface of the lower engagement head (856) when the vial engagement mechanism (520) translates downward due to the fixed pin being received within the linear track (858).

12. The delivery assembly of any preceding claim, wherein the vial assembly (250) comprises an inner chamber (904) and a floating septum (910) movably disposed therein, wherein, preferably, the floating septum (910) is configured to translate within the vial body in response to the inner chamber (904) receiving fluid medium therein, and wherein, preferably, the floating septum (910) engages a needle of the sled assembly (540) positioned at a bottom end of the inner chamber (904) as the floating septum (910) translates within the vial body and the fluid medium within the inner chamber (904) increases.

13. The delivery device of any preceding claim, wherein the vial engagement mechanism (520) is coupled to an electrical system such that translation of the vial engagement mechanism (520) within the vial containment region (518) is electrically-driven.

14. The delivery device of any preceding claim, wherein the sled assembly (540) includes a battery that is configured to supply power to the sled assembly(540) and the console (510) in response to the sled assembly (540) coupling with the console (510) at the vial containment region (518), wherein, preferably, the sled assembly (540) includes a battery status indicator communicatively coupled to the battery and configured to display data indicative of a charge of the battery.

15. The delivery device of any preceding claim, wherein the sled assembly (540) is configured to maintain fluid leaks of therapeutic particles therein such that the sled assembly is operable to inhibit radioactive emissions and fluid leaks from the therapeutic particles stored therein.

## Patentansprüche

1. Abgabeanordnung, umfassend:
eine Konsole (510), die einen Fläschchenaufnahmebereich (518) einschließt; eine Fläschchenanordnung (250, 580);
einen Fläschchen-Eingriffsmechanismus (520), der sich von der Konsole (510) innerhalb des Fläschchenaufnahmebereichs (518) erstreckt, wobei der Eingriffsmechanismus (520) konfiguriert ist, um mit der Fläschchenanordnung (250) in Eingriff zu kommen; und
eine Schlittenanordnung (540), die mit der Konsole (510) an dem Fläschchenaufnahmebereich (518) lösbar koppelbar ist; wobei die Schlittenanordnung konfiguriert ist, um die Fläschchenanordnung teilweise aufzunehmen; **dadurch gekennzeichnet, dass**: die Abgabeanordnung weiter Folgendes umfasst:
eine Sicherheitsabschirmung (526), die abnehmbar an die Konsole (510) über dem Fläschchenaufnahmebereich (518) koppelbar ist, so dass der Fläschchen-Eingriffsmechanismus (520) und die Schlittenanordnung (540) innerhalb der Sicherheitsabschirmung (526) eingekapselt sind, wenn die Sicherheitsabschirmung (526) daran gekoppelt ist; und dadurch, dass:
die Schlittenanordnung (540), die Fläschchenanordnung (250) und die Sicherheitsabschirmung (526) konfiguriert sind zum Unterbinden radioaktiver Emissionen aus dem Inneren des Fläschchenaufnahmebereichs (518).

2. Abgabeanordnung nach Anspruch 1, wobei die Fläschchenanordnung Folgendes umfasst:
einen Fläschchenkörper (589), der aus einem Material gebildet ist, das radioaktive Emissionen aus dem Inneren des Fläschchenkörpers unterbindet;
ein Verriegelungsmerkmal (586), das mit dem Fläschchenkörper gekoppelt und so konfiguriert ist, dass es die Fläschchenanordnung (250) sicher mit der Schlittenanordnung (540) in Eingriff bringt, wenn der Fläschchenkörper darin aufgenommen ist; und
einen Kolben (584; 835), der relativ zu dem Verriegelungsmerkmal (586) und durch den Fläschchenkörper hindurch als Reaktion darauf, dass der Fläschchen-Eingriffsmechanismus (520) mit der Fläschchenanordnung (250) in Eingriff kommt, gleitend verschiebbar ist,
wobei der Kolben (584) vorzugsweise einen Eingriffskopf (528A) mit einem Paar elastischer Arme (581A) einschließt, die so bemessen und geformt sind, dass sie mit einem Paar von Hebelarmen (522) des Fläschchen-Eingriffsmechanismus (520) ineinandergreifen, oder wobei die Fläschchenanordnung (250) vorzugsweise ein Septum (255) umfasst, das innerhalb eines distalen Endes des Fläschchenkörpers angeordnet und konfiguriert ist, um den Fläschchenkörper abzudichten.

3. Abgabeanordnung nach einem der vorstehenden Ansprüche, wobei die Konsole (510) eine Schnittstellenanzeige (136) und eines oder mehrere von einem Dosimeter, einem linearen Kodierer, einem optischen Sensor, einem linearen Verschiebungssensor, einem Durchflusssensor, einem Ultraschallsensor, einem magnetischen Kodierer, einem Laserabstandssensor, einem Induktivitätssensor, einem radialen Kodierer, einem volumetrischen Sensor, einem Strahlungssensor, und einem mechanischen Wandler umfasst, und wobei die Schnittstellenanzeige (136) vorzugsweise kommunikativ mit einem oder mehreren von dem Dosimeter, dem linearen Kodierer, dem optischen Sensor, dem linearen Verschiebungssensor, dem Durchflusssensor, dem Ultraschallsensor, dem magnetischen Kodierer, dem Laserabstandssensor, dem Induktivitätssensor, dem radialen Kodierer, dem volumetrischen Sensor, dem Strahlungssensor und den mechanischen Wandlern gekoppelt ist.

4. Abgabeanordnung nach einem der vorstehenden Ansprüche, wobei der Fläschchen-Eingriffsmechanismus (520)
innerhalb des Fläschchenaufnahmebereichs (518) und in der Nähe der Schlittenanordnung (540) angeordnet ist, wenn die Schlittenanordnung (540) mit der Konsole (510) gekoppelt ist, und weiter vorzugsweise einen Abgabegriff (528) umfasst, der mit dem Fläschchen-Eingriffsmechanismus (520) gekoppelt und so konfiguriert ist, dass er den Fläschchen-Eingriffsmechanismus (520) innerhalb des Fläschchenaufnahmebereichs (518) bewegt, und wobei der Abgabegriff (528) vorzugsweise so konfiguriert ist, dass er die Fläschchenanordnung (250) als Reaktion auf eine Betätigung des Abgabegriffs (528) relativ zu der Schlittenanordnung (540) bewegt.

5. Abgabeanordnung nach einem der vorstehenden Ansprüche, wobei die Schlittenvorrichtung (540)
ein Verriegelungssystem (550) und eine Fläschchenkammer (558) umfasst, wobei das Verriegelungssystem (550) eine Öffnung bildet, die so bemessen und geformt ist, dass sie die Fläschchenanordnung (250) durch sie hindurch und in die Fläschchenkammer (558) hinein aufnimmt, wobei das Verriegelungssystem (550) vorzugsweise ringförmige Vorsprünge umfasst, die sich seitlich in die Öffnung hinein erstrecken, wobei die ringförmigen Vorsprünge so bemessen und geformt sind, dass sie in ein Verriegelungsmerkmal der Fläschchenanordnung (250) eingreifen, wenn die Fläschchenanordnung (250) durch die Öffnung und in die Fläschchenkammer (558) aufgenommen wird, oder wobei die Fläschchenkammer (558) vorzugsweise in eine Schutzabschirmung (253) eingekapselt ist, die aus einem Material gebildet ist, das radioaktive Emissionen aus dem Inneren der Fläschchenkammer (558) unterbindet.

6. Abgabeanordnung nach Anspruch 5, wobei die Schlittenanordnung (540) eine Nadel (559) umfasst, die sich innerhalb der Fläschchenanordnung (558) erstreckt, wobei die Nadel (559) so konfiguriert ist, dass sie ein Septum der Fläschchenanordnung durchsticht, wenn die Fläschchenanordnung in der Fläschchenkammer durch die Öffnung aufgenommen wird, wobei die Schlittenanordnung vorzugsweise weiter einen Verteiler (555) umfasst, der fluidisch mit der Nadel (559) gekoppelt ist, so dass der Verteiler (555) über die Nadel (559) in Fluidverbindung mit der Fläschchenkammer (558) steht, und wobei die Schlittenanordnung (540) vorzugsweise einen oder mehrere Anschlüsse umfasst, die durch den Verteiler (555) in Fluidverbindung mit der Fläschchenkammer (558) stehen, wobei eine oder mehrere Zuführungsleitungen so betrieben werden können, dass sie über den einen oder die mehreren Anschlüsse mit dem Verteiler (555) gekoppelt werden.

7. Abgabeanordnung nach einem der vorstehenden Ansprüche, wobei die Schlittenanordnung (540), die Fläschchenanordnung (250) und die Sicherheitsabschirmung (526) aus Materialien gebildet sind, die radioaktive Emissionen aus dem Inneren des Fläschchenaufnahmebereichs (518) unterbinden.

8. Abgabeanordnung nach Anspruch 2, wobei der Kolben (584) einen Eingriffskopf (582) mit einer unteren Fläche einschließt, die so bemessen und geformt ist, dass sie mit einem Paar von Hebelarmen (581) des Fläschchen-Eingriffsmechanismus ineinandergreift, wobei die Fläschchenanordnung weiter vorzugsweise ein Paar flexibler Flügel (840) einschließt, die drehbar mit dem Kolben (835) gekoppelt sind, so dass das Paar flexibler Flügel (840) so konfiguriert ist, dass es sich als Reaktion auf eine Verschiebung des Kolbens (835) nach außen aus dem Fläschchenkörper flexibel verformt, und wobei vorzugsweise das Paar flexibler Flügel (840) so konfiguriert ist, dass es eine Eingriffsfläche bildet, die so bemessen und geformt ist, dass sie mit dem Paar von Hebelarmen (581) als Reaktion auf eine Verformung des Paars flexibler Flügel (840) ineinandergreift, wenn sich der Kolben (835) von dem Fläschchenkörper nach außen verschiebt.

9. Abgabeanordnung nach Anspruch 2, wobei der Kolben (835) einen oberen Eingriffskopf (851) und einen unteren Eingriffskopf (856) einschließt, der relativ unterhalb des oberen Eingriffskopfes (851) entlang einer Längslänge des Kolbens (835) positioniert ist, wobei vorzugsweise der obere Eingriffskopf (851) eine untere Fläche (853) einschließt, die so bemessen und geformt ist, dass sie mit einem Paar von Hebelarmen (522) des Fläschchen-Eingriffsmechanismus (520) als Reaktion auf eine Aufwärtsverschiebung des Fläschchen-Eingriffsmechanismus (520) ineinandergreift, und wobei vorzugsweise der untere Eingriffskopf (856) eine obere Fläche (855) einschließt, die so bemessen und geformt ist, dass sie mit dem Paar von Hebelarmen (522) des Fläschchen-Eingriffsmechanismus (520) als Reaktion auf eine Abwärtsverschiebung des Fläschchen-Eingriffsmechanismus (520) ineinandergreift.

10. Abgabeanordnung nach Anspruch 9, wobei der Kolben (835) weiter mindestens eine gekrümmte Spur (857) und eine lineare Spur (858) einschließt, die entlang einer Außenfläche des Kolbens (835) angeordnet sind, wobei die gekrümmte Spur (857) und die lineare Spur (858) so bemessen und geformt sind, dass sie einen festen Stift des Fläschchenkörpers gleitend darin aufnehmen, wobei sich vorzugsweise die gekrümmte Spur (857) um die Außenfläche des Kolbens (835) herum erstreckt und die lineare Spur (858) sich entlang der Außenfläche und parallel zu einer Längslänge des Kolbens (835) erstreckt.

11. Abgabeanordnung nach Anspruch 10, wobei der Kolben (835) so konfiguriert ist, dass er sich als Reaktion auf ein Ineinandergreifen des Paares von Hebelarmen (522) mit der unteren Fläche des oberen Eingriffskopfes (851) innerhalb des Fläschchenkörpers dreht und sich von diesem nach außen verschiebt, wenn sich der Fläschchen-Eingriffsmechanismus (520) aufgrund dessen, dass der feste Stift innerhalb der gekrümmten Spur (857) aufgenommen wird, nach oben verschiebt, oder wobei der Kolben (835) so konfiguriert ist, dass er sich in den Fläschchenkörper in einer festen Ausrichtung relativ dazu als Reaktion auf ein Ineinandergreifen des Paars von Hebelarmen (522) mit der oberen Fläche des unteren Eingriffskopfes (856) verschiebt, wenn sich der Fläschchen-Eingriffsmechanismus (520) aufgrund dessen, dass der feste Stift in der linearen Spur (858) aufgenommen wird, nach unten verschiebt.

12. Abgabeanordnung nach einem der vorstehenden Ansprüche, wobei die Fläschchenanordnung (250)
eine innere Kammer (904) und ein darin beweglich angeordnetes schwimmendes Septum (910) umfasst, wobei das schwimmende Septum (910) vorzugsweise so konfiguriert ist, dass es sich innerhalb des Fläschchenkörpers als Reaktion darauf verschiebt, dass die innere Kammer (904) ein fluides Medium darin aufnimmt, und wobei vorzugsweise das schwimmende Septum (910) mit einer Nadel der Schlittenanordnung (540) in Eingriff kommt, die an einem unteren Ende der inneren Kammer (904) positioniert ist, wenn sich das schwimmende Septum (910) innerhalb des Fläschchenkörpers verschiebt und das fluide Medium innerhalb der inneren Kammer (904) zunimmt.

13. Abgabevorrichtung nach einem der vorstehenden Ansprüche, wobei der Fläschchen-Eingriffsmechanismus (520) mit einem elektrischen System gekoppelt ist, so dass die Verschiebung des Fläschchen-Eingriffsmechanismus (520) innerhalb des Fläschchenaufnahmebereichs (518) elektrisch angetrieben ist.

14. Abgabevorrichtung nach einem der vorstehenden Ansprüche, wobei die Schlittenanordnung (540) eine Batterie einschließt, die so konfiguriert ist, dass sie die Schlittenanordnung (540) und die Konsole (510) als Reaktion darauf mit Strom versorgt, dass die Schlittenanordnung (540) mit der Konsole (510) in dem Fläschchenaufnahmebereich (518) gekoppelt wird, wobei die Schlittenanordnung (540) vorzugsweise eine Batteriestatusanzeige einschließt, die kommunikativ mit der Batterie gekoppelt und so konfiguriert ist, dass sie Daten anzeigt, die einen Ladezustand der Batterie anzeigen.

15. Abgabevorrichtung nach einem der vorstehenden Ansprüche, wobei die Schlittenanordnung (540) so konfiguriert ist, dass sie Flüssigkeitslecks von therapeutischen Partikeln darin aufrechterhält, so dass die Schlittenanordnung in der Lage ist, radioaktive Emissionen und Flüssigkeitslecks aus den darin gelagerten therapeutischen Partikeln zu unterbinden.

## Revendications

1. Ensemble de distribution, comprenant :
une console (510) incluant une région (518) de confinement de flacon ;
un ensemble flacon (250, 580) ;
un mécanisme (520) de mise en prise de flacon s'étendant depuis la console (510) au sein de la région (518) de confinement de flacon, dans lequel le mécanisme de mise en prise (520) est configuré pour venir en prise avec l'ensemble flacon (250) ; et
un ensemble traîneau (540) pouvant être couplé de manière amovible à la console (510) au niveau de la région (518) de confinement de flacon ; dans lequel l'ensemble traîneau est configuré pour recevoir partiellement l'ensemble flacon ; **caractérisé en ce que** : l'ensemble de distribution comprend en outre :
un blindage de sécurité (526) pouvant être couplé de manière amovible à la console (510) au-dessus de la région (518) de confinement de flacon de telle sorte que le mécanisme (520) de mise en prise de flacon et l'ensemble traîneau (540) sont encapsulés au sein du blindage de sécurité (526) lorsque le blindage de sécurité (526) est couplé à ceuxci ; et **en ce que** :
l'ensemble traîneau (540), l'ensemble flacon (250), et le blindage de sécurité (526) sont configurés pour empêcher des émissions radioactives depuis l'intérieur de la région (518) de confinement de flacon.

2. Ensemble de distribution selon la revendication 1, dans lequel l'ensemble flacon comprend :
un corps (589) de flacon formé d'un matériau qui empêche des émissions radioactives depuis l'intérieur du corps de flacon ;
un élément de verrouillage (586) couplé au corps de flacon et configuré pour mettre en prise de manière sécurisée l'ensemble flacon (250) sur l'ensemble traîneau (540) lorsque le corps de flacon est reçu dans celui-ci ; et
un piston-plongeur (584 ; 835) pouvant translater de manière coulissante par rapport à l'élément de verrouillage (586) et à travers le corps de flacon en réponse à la mise en prise du mécanisme de mise en prise (520) avec l'ensemble flacon (250),
dans lequel, de préférence, le piston-plongeur (584) inclut une tête de mise en prise (528A) présentant une paire de bras élastiques (581A) qui sont dimensionnés et façonnés pour se verrouiller avec une paire de bras de levier (522) du mécanisme de mise en prise (520), ou
dans lequel, de préférence, l'ensemble flacon (250) comprend un septum (255) disposé dans une extrémité distale du corps de flacon qui est configuré pour sceller le corps de flacon.

3. Ensemble de distribution selon une quelconque revendication précédente, dans lequel la console (510) comprend un écran d'interface (136) et un ou plusieurs parmi un dosimètre, un codeur linéaire, un capteur optique, un capteur de déplacement linéaire, un capteur de débit, un capteur à ultrasons, un codeur magnétique, un capteur de distance laser, un capteur à inductance, un codeur radial, un capteur volumétrique, un capteur de rayonnement, et un transducteur mécanique, et dans lequel, de préférence, l'écran d'interface (136) est couplé de manière communicative à un ou plusieurs parmi le dosimètre, le codeur linéaire, le capteur optique, le capteur de déplacement linéaire, le capteur de débit, le capteur à ultrasons, le codeur magnétique, le capteur de distance laser, le capteur à inductance, le codeur radial, le capteur volumétrique, le capteur de rayonnement et des transducteurs mécaniques.

4. Ensemble de distribution selon une quelconque revendication précédente, dans lequel le mécanisme (520) de mise en prise de flacon est disposé au sein de la région (518) de confinement de flacon et à proximité de l'ensemble traîneau (540) lorsque l'ensemble traîneau (540) est couplé à la console (510), et en outre, de préférence, comprenant une poignée de distribution (528) couplée au mécanisme (520) de mise en prise de flacon et configurée pour déplacer le mécanisme (520) de mise en prise de flacon au sein de la région (518) de confinement de flacon, et dans lequel, de préférence, la poignée de distribution (528) est configurée pour déplacer l'ensemble flacon (250) par rapport à l'ensemble traîneau (540) en réponse à un actionnement de la poignée de distribution (528).

5. Ensemble de distribution selon une quelconque revendication précédente, dans lequel l'ensemble traîneau (540) comprend un système de verrouillage (550) et une chambre (558) à flacon, dans lequel le système de verrouillage (550) forme une ouverture dimensionnée et façonnée pour recevoir l'ensemble flacon (250) à travers celle-ci et dans la chambre (558) à flacon, dans lequel, de préférence, le système de verrouillage (550) comprend des saillies annulaires s'étendant latéralement dans l'ouverture, dans lequel les saillies annulaires sont dimensionnées et façonnées pour mettre en prise un élément de verrouillage de l'ensemble flacon (250) lorsque l'ensemble flacon (250) est reçu à travers l'ouverture et dans la chambre (558) à flacon, ou dans lequel, de préférence, la chambre (558) à flacon est encapsulée dans un blindage protecteur (253) qui est formé d'un matériau qui empêche des émissions radioactives depuis l'intérieur de la chambre (558) à flacon.

6. Ensemble de distribution selon la revendication 5, dans lequel l'ensemble traîneau (540) comprend une aiguille (559) s'étendant au sein de la chambre (558) à flacon, l'aiguille (559) étant configurée pour percer un septum de l'ensemble flacon lorsque l'ensemble flacon est reçu au sein de la chambre à flacon à travers l'ouverture, dans lequel, de préférence, l'ensemble traîneau comprend en outre une tubulure (555) couplée fluidiquement à l'aiguille (559) de telle sorte que la tubulure (555) est en communication fluidique avec la chambre (558) à flacon via l'aiguille (559), et dans lequel, de préférence, l'ensemble traîneau (540) comprend un ou plusieurs orifices qui sont en communication fluidique avec la chambre (558) à flacon par le biais de la tubulure (555), dans lequel une ou plusieurs conduites de distribution servent à être couplées à la tubulure (555) via les un ou plusieurs orifices.

7. Ensemble de distribution selon une quelconque revendication précédente, dans lequel l'ensemble traîneau (540), l'ensemble flacon (250), et le blindage de sécurité (526) sont formés de matériaux qui empêchent des émissions radioactives depuis l'intérieur de la région (518) de confinement de flacon.

8. Ensemble de distribution selon la revendication 2, dans lequel le piston-plongeur (584) inclut une tête de mise en prise (582) présentant une surface de fond qui est dimensionnée et façonnée pour se verrouiller avec une paire de bras de levier (581) du mécanisme de mise en prise de flacon, dans lequel, de préférence, l'ensemble flacon inclut en outre une paire d'ailes flexibles (840) couplées de manière rotative au piston-plongeur (835) de telle sorte que la paire d'ailes flexibles (840) sont configurées pour se déformer de manière flexible en réponse à une translation du piston-plongeur (835) vers l'extérieur depuis le corps de flacon, et dans lequel, de préférence, la paire d'ailes flexibles (840) sont configurées pour former une surface de mise en prise qui est dimensionnée et façonnée pour se verrouiller avec la paire de bras de levier (581) en réponse à une déformation de la paire d'ailes flexibles (840) quand le piston-plongeur (835) se translate vers l'extérieur depuis le corps de flacon.

9. Ensemble de distribution selon la revendication 2, dans lequel le piston-plongeur (835) inclut une tête supérieure de mise en prise (851) et une tête inférieure de mise en prise (856) positionnée relativement sous la tête supérieure de mise en prise (851) le long d'une longueur longitudinale du piston-plongeur (835), dans lequel, de préférence, la tête supérieure de mise en prise (851) inclut une surface de fond (853) qui est dimensionnée et façonnée pour se verrouiller avec une paire de bras de levier (522) du mécanisme (520) de mise en prise de flacon en réponse à une translation vers le haut du mécanisme (520) de mise en prise de flacon, et dans lequel, de préférence, la tête inférieure de mise en prise (856) inclut une surface supérieure (855) qui est dimensionnée et façonnée pour se verrouiller avec la paire de bras de levier (522) du mécanisme (520) de mise en prise de flacon en réponse à une translation vers le bas du mécanisme (520) de mise en prise de flacon.

10. Ensemble de distribution selon la revendication 9, dans lequel le piston-plongeur (835) inclut en outre au moins une voie incurvée (857) et une voie linéaire (858) disposées le long d'une surface extérieure du piston-plongeur (835), dans lequel la voie incurvée (857) et la voie linéaire (858) sont dimensionnées et façonnées pour recevoir de manière coulissante une broche fixe du corps de flacon dans celles-ci, dans lequel, de préférence, la voie incurvée (857) s'étend autour de la surface extérieure du piston-plongeur (835) et la voie linéaire (858) s'étend le long de la surface extérieure et parallèlement à une longueur longitudinale du piston-plongeur (835).

11. Ensemble de distribution selon la revendication 10, dans lequel le piston-plongeur (835) est configuré pour tourner dans et se translater vers l'extérieur depuis le corps de flacon en réponse à un verrouillage de la paire de bras de levier (522) avec la surface de fond de la tête supérieure de mise en prise (851) lorsque le mécanisme (520) de mise en prise de flacon se translate vers le haut en raison d'une réception de la broche fixe au sein de la voie incurvée (857), ou dans lequel le piston-plongeur (835) est configuré pour se translater dans le corps de flacon à une orientation fixe par rapport à celui-ci en réponse à un verrouillage de la paire de bras de levier (522) avec la surface supérieure de la tête inférieure de mise en prise (856) lorsque le mécanisme (520) de mise en prise de flacon se translate vers le bas en raison d'une réception de la broche fixe au sein de la voie linéaire (858).

12. Ensemble de distribution selon une quelconque revendication précédente, dans lequel l'ensemble flacon (250) comprend une chambre intérieure (904) et un septum flottant (910) disposé de manière mobile dans celle-ci, dans lequel, de préférence, le septum flottant (910) est configuré pour se translater au sein du corps de flacon en réponse à une réception d'un milieu fluide dans la chambre intérieure (904), et dans lequel, de préférence, le septum flottant (910) met en prise une aiguille de l'ensemble traîneau (540) positionnée au niveau d'une extrémité de fond de la chambre intérieure (904) quand le septum flottant (910) se translate au sein du corps de flacon et que le milieu fluide au sein de la chambre intérieure (904) augmente.

13. Dispositif de distribution selon une quelconque revendication précédente, dans lequel le mécanisme (520) de mise en prise de flacon est couplé à un système électrique de telle sorte qu'une translation du mécanisme (520) de mise en prise de flacon au sein de la région (518) de confinement de flacon est entraînée électriquement.

14. Dispositif de distribution selon une quelconque revendication précédente, dans lequel l'ensemble traîneau (540) inclut une batterie qui est configurée pour alimenter électriquement l'ensemble traîneau (540) et la console (510) en réponse à un couplage de l'ensemble traîneau (540) avec la console (510) au niveau de la région (518) de confinement de flacon, dans lequel, de préférence, l'ensemble traîneau (540) inclut un indicateur d'état de batterie couplé de manière communicative à la batterie et configuré pour afficher des données indicatives d'une charge de la batterie.

15. Dispositif de distribution selon une quelconque revendication précédente, dans lequel l'ensemble traîneau (540) est configuré pour maintenir des fuites de fluide de particules thérapeutiques dans celui-ci de telle sorte que l'ensemble traîneau sert à empêcher des émissions radioactives et des fuites de fluide depuis les particules thérapeutiques stockées dans celui-ci.
